# EUROPEAN PATENT SPECIFICATION

(11) **EP 2 515 112 B1**
(45) Date of publication and mention of the grant of the patent: **12.08.2015**
(21) Application number: 12164401.7
(22) Date of filing: 17.04.2012
(51) Int. Cl.: G01N 33/543, G01N 27/327

(54) **Method for electrochemically detecting analyte**
Verfahren zum elektrochemischen Erkennen eines Analyts
Procédé de détection électrochimique d'analytes

(30) Priority: 22.04.2011 JP 2011096341; 22.04.2011 JP 2011096319; 29.03.2012 JP 2012075914
(43) Date of publication of application: 24.10.2012
(73) Proprietor: SYSMEX CORPORATION, Kobe-shi, Hyogo 651-0073 (JP)
(72) Inventor: Suzuki, Seigo, Hyogo, 651-0073 (JP); Kirimura, Hiroya, Hyogo, 651-0073 (JP); Seike, Masayoshi, Hyogo, 651-0073 (JP); Iwanaga, Shigeki, Hyogo, 651-0073 (JP); Hori, Nobuyasu, Hyogo, 651-0073 (JP); Uraoka, Yukiharu, Nara, 630-0192 (JP); Bin, Zheng, Nara, 630-0192 (JP)
(74) Representative: Hoffmann Eitle

(56) References cited:
- EP-A1- 2 458 383
- EP-A2- 0 125 139
- WO-A1-99/32662
- FR-A1- 2 816 058
- US-A1- 2002 121 314
- US-A1- 2010 112 578

## Description

### FIELD OF THE INVENTION

The present invention relates to a method for electrochemically detecting an analyte. More particularly, it relates to a method for electrochemically detecting an analyte, which is useful for detecting and quantifying analytes such as nucleic acids and proteins as well as clinically examining and diagnosing diseases using these methods.

### BACKGROUND

Clinical examination and diagnosis of diseases are performed by detecting genes and proteins related to the diseases which are contained in biological samples by detection methods such as a gene detection method and an immunological detection method. As a method for performing a clinical examination and diagnosis, a photochemical detection method using a photocurrent generated by exciting a photochemically active labeling substance with light to detect analytes such as nucleic acids or proteins is suggested. Here, in the clinical examination and diagnosis, it is necessary to detect a very small amount of analytes included in a specimen. Accordingly, there is a need to improve detection sensitivity of analytes.

For example, U.S. Patent Publication No. 2011/193187 discloses a method for specifically detecting an analyte by a photocurrent comprising: using an electrode in which an antibody, which is a trapping substance for specifically recognizing a protein as an analyte, is immobilized on the surface; and a labeled antibody in which an antibody, which is a binding substance, is labeled with an electrochemically active labeling substance. In the method described in U.S. Patent Publication No. 2011/193187, the analyte is brought into contact with the antibody on the electrode, and the analyte is trapped on the electrode by the antibody. Thereafter, the analyte trapped on the electrode is brought into contact with the labeled antibody to form a complex. Then, the analyte is detected by measuring the photocurrent based on the labeling substance in the labeled antibody.

However, as described in U.S. Patent Publication No. 2011/193187, when the binding substance is directly labeled with the labeling substance not via a support, there is a limit on the number of the labeling substance which can be bound to the binding substance. Thus, in the method described in U.S. Patent Publication No. 2011/193187, the number of the labeling substance per analyte cannot be increased beyond a limit, which results in difficulty in achieving high sensitivity.

In the method described in U.S. Patent Publication No. 2011/193187, since the complex formed on the electrode is bulky, a distance between the labeling substance included in the complex and the electrode becomes longer physically. Thus, the transportation of electrons between the labeling substance and the electrode is hardly performed. For example, an IgG antibody has a size of about 10 nm. In the method described in U.S. Patent Publication No. 2011/193187, when the IgG antibody is used as an antibody constituting a trapping substance and a labeled antibody, the labeling substance in the complex to be formed on the electrode is present in a position very distant from the electrode surface where the transportation of electrons may be efficiently occurred in detecting the analyte. Consequently, in the method described in U.S. Patent Publication No. 2011/193187, the specific volume of the complex formed in detecting the analyte is a large restriction in terms of achieving high sensitivity.

WO 99/32662 discloses methods and apparatus for improved luminescence assays using microparticles cross-reference to related application.

US 2002/121314 relates to the use asymmetric monolayer forming species and electroconduit forming species to detect target analytes.

EP 0125139 relates to assay techniques and especially to the detection measuring and monitoring of complex, usually biologically-arising, molecules or part-molecular structures often in complicated admixture with other similar molecules.

US 2010/112578 relates to a test chip, a detection apparatus and a method for detecting an analyte.

FR 816058 relates to a method and a device for determining the concentration of dioxin in a sample.

### SUMMARY OF THE INVENTION

The scope of the present invention is defined solely

by the appended claims, and is not affected to any degree by the statements within this summary.

The present invention has been achieved in view of the above circumstances. Its object is to provide a method for electrochemically detecting an analyte which can detect the analyte with high detection sensitivity.

The present inventors have found that the detection sensitivity can-be significantly improved by binding a labeling substance which is used to detect an analyte to a binding substance via a support composed of polypeptide in the method for electrochemically detecting an analyte, and the above-described problems can be solved. Thus, the present invention has been completed.

Further, the present inventors have found that the detection sensitivity can be significantly improved by using a label binding substance in which a labeling substance is immobilized on an antibody via a modulator which generates an interaction with a working electrode site except a site where an electrolytic solution and a trapping substance are bound in detecting the analyte. Thus, the present invention has been completed.
(1) The present invention relates to
   a method for electrochemically detecting an analyte in an electrolytic solution comprising:
   bringing a sample containing an analyte into contact with a working electrode on which trapping substance (281) for trapping the analyte is immobilized to allow the analyte to be trapped on the working electrode by the trapping substance;
   forming a complex containing the analyte (S) trapped on the working electrode in the trapping process and a label binding substance (290, 390), wherein the label binding substance (290, 390) is formed of a binding substance (291), a modulator (292), and a labeling substance (293, 393), wherein the labeling substance (293, 393) is retained via the modulator (292) on a binding substance (291) which binds to the analyte (S) on the working electrode, wherein the modulator generates an interaction with an electrolytic solution and a working
   electrode site except a site where the trapping substance are bound, and
   electrochemically detecting the labeling substance present on the working electrode obtained in the complex formation process, wherein
   the labeling substance is retained via the modulator on the binding substance, wherein
   the electrolytic solution contains an aprotic solvent, and the surface of the working electrode and the modulator exhibit hydrophilicity, or
   the electrolytic solution contains a protic solvent, and the surface of the working electrode and the modulator exhibit hydrophobicity
(2) The method according to (1), wherein the modulator is DNA.
(3) The method according to (1) or (2), wherein in the process of forming a complex, the analyte trapped on the working electrode in the trapping process is brought into contact with the label binding substance to form the complex on the working electrode.
(4) A method for electrochemically detecting an analyte in an electrolytic solution comprising:
   bringing a sample containing an analyte into contact with a working electrode on which trapping substance (281) for trapping the analyte is immobilized to allow the analyte to be trapped on the working electrode by the trapping substance;
   forming a complex, on the working electrode, by bringing a conjugate containing a first binding substance which binds to the analyte into contact with the analyte trapped on the working electrode in the trapping process and bringing the conjugate bound to the analyte into contact with a labeled form in which the labelingsubstance is bound, via the modulator, to a second binding substance, which binds to the conjugatein the complex formation process; and
   electrochemically detecting the labeling substance present on the working electrode obtained in the complex formation process, wherein
   the labeling substance is retained via the modulator on the binding substance, wherein
   the electrolytic solution contains an aprotic solvent, and the surface of the working electrode and the modulator exhibit hydrophilicity, or
   the electrolytic solution contains a protic solvent, and the surface of the working electrode and the modulator exhibit hydrophobicity.
(5) The method according to any one (1) to (4), wherein the working electrode is washed to remove the label binding substance which is not bound to the analyte after the process of forming a complex.
(6) The method according to any one of (1) to (5), wherein the labeling substance is an electrochemically or photochemically active substance.
(7) The method according to any one of (1) to (6), wherein the label binding substance is a multivalent-labeled binding substance in which a plurality of labeling substances are attached to the binding substance via modulators.
(8) The method according to any one of (1) to (7), wherein the analyte is quantified based on the detection results obtained in the detecting process.
(9) The method according to any one of (1) to (8), wherein the analyte is a plurality of analytes.

According to the method for electrochemically detecting an analyte of the present invention, the analyte can be detected with high detection sensitivity.

### BRIEF DESCRIPTION OF THE DRAWINGS

Fig. 1 is a perspective view showing a detector which is used for a method for electrochemically detecting an analyte according to first and second embodiments of the present disclosure;
Fig. 2 is a block diagram showing the configuration of the detector shown in Fig. 1;
Fig. 3 is a perspective view showing a detection chip which is used for the method for electrochemically detecting an analyte according to first and second embodiments of the present disclosure;
Fig. 4A is a cross sectional view in an AA line of the detection chip shown in Fig. 3;
Fig. 4B is a perspective view of the upper substrate of the detection chip shown in Fig. 3 as viewed from the lower surface;
Fig. 4C is a perspective view of the lower substrate of the detection chip shown in Fig. 3 as viewed from the upper surface;
Fig. 5 is a cross sectional explanatory view showing an example of a portion including electrodes in the detection chip to be used in the method for electrochemically detecting an analyte according to the first embodiment of the present disclosure;
Fig. 6 is a process explanatory view showing the procedure of the method for photoelectrochemically detecting an analyte according to the first embodiment of the present disclosure;
Fig. 7 is an outline explanatory view showing detection processes in a conventional method for electrochemically detecting an analyte;
Fig. 8 is a process explanatory view showing another example of the procedure of the method for photoelectrochemically detecting an analyte according to the first embodiment of the present disclosure;
Fig. 9 is a process explanatory view showing an example of the procedure of the oxidation reduction current/electrochemiluminescence detection method for an analyte according to the first embodiment of the present disclosure;
Fig. 10 is an outline explanatory view showing DNAs used in Test example 1-1 (reference);
Fig. 11 is an outline explanatory view showing the operating procedure of (Example 1-1 (reference)) the method for electrochemically detecting an analyte in Test example 1-1;
Fig. 12 is an outline explanatory view showing the operating procedure of (Comparative example 1-1 (reference)) the method for electrochemically detecting an analyte in Test example 1-1;
Fig. 13 is a graph showing examined results of a relationship between the kind of the detection method and photocurrent in Test example 1-1;
Fig. 14 is an outline explanatory view showing DNAs used in Test example 1-2 (reference);
Fig. 15 is an outline explanatory view showing the operating procedure of (Example 1-2 (reference)) the method for electrochemically detecting an analyte in Test example 1-2;
Fig. 16 is an outline explanatory view showing the operating procedure of (Comparative example 1-2 (reference)) the method for electrochemically detecting an analyte in Test example 1-2;
Fig. 17 is a graph showing examined results of a relationship between the kind of the detection method and photocurrent in Test example 1-2;
Fig. 18 is an outline explanatory view showing DNAs used in Test example 1-3 (reference);
Fig. 19 is an outline explanatory view showing the operating procedure of (Example 1-3(reference)) the method for electrochemically detecting an analyte in Test example 1-3;
Fig. 20 is an outline explanatory view showing the operating procedure of (Comparative example 1-3(reference)) the method for electrochemically detecting an analyte in Test example 1-3;
Fig. 21 is a graph showing examined results of a relationship between the kind of the detection method and photocurrent in Test example 1-3;
Fig. 22 is a cross sectional explanatory view showing an example of a portion including electrodes in the detection chip to be used in the method for electrochemically detecting an analyte according to the second embodiment of the present invention;
Fig. 23 is a process explanatory view showing an example of the procedure of the method for photoelectrochemically detecting an analyte according to the second embodiment of the present invention;
Fig. 24 is an outline explanatory view showing detection processes in a conventional method for electrochemically detecting an analyte;
Fig. 25 is a process explanatory view showing another example of the procedure of the method for photoelectrochemically detecting an analyte according to the second embodiment of the present invention;
Fig. 26 is a process explanatory view showing an example of the procedure of the oxidation reduction current/electrochemiluminescence detection method for an analyte according to the second embodiment of the present invention;
Fig. 27 is an outline explanatory view showing a detection process (27A) when an analyte is detected using a label binding substance obtained in Example 2-1 (Test No. 1) and a detection process (27B) when an analyte is detected using a labeled antibody obtained in Comparative example 2-1 (Test No. 3) in Test example 2-1;
Fig. 28 is a graph showing examined results of a relationship between the kind of the detection method and photocurrent in Test example 2-1;
Fig. 29 is a process explanatory view showing a part of the procedures of the method for electrochemically detecting an analyte of Test No. 5 in Example 2-2;
Fig. 30 is a process explanatory view showing a part of the procedures of the method for electrochemically detecting an analyte of Test No. 7 in Comparative example 2-2;
Fig. 31 is an outline explanatory view showing detection processes (31A) and (31B) in the method for electrochemically detecting an analyte using Test No. 5 in Example 2-2 and Test No. 7 in Comparative example 2-2;
Fig. 32 is a graph showing examined results of a relationship between the kind of the detection method and photocurrent in Test example 2-2;
Fig. 33 is an outline explanatory view of a biotinylated-DNA/Alexa Fluor 750-labeled DNA complex obtained in Preparation example 2-5;
Fig. 34 is a process explanatory view showing a part of the procedures of the method for electrochemically detecting an analyte of Test No. 9 in Example 2-3;
Fig. 35 is a graph showing examined results of a relationship between the kind of the detection method and photocurrent in Example 2-3;
Fig. 36 is a graph showing examined results of a relationship between the concentration of the analyte (mouse IgG) and photocurrent in Example 2-4;
Fig. 37 is a graph showing examined results of a relationship between the concentration of the analyte (human IL-6) and photocurrent in the Example 2-5;
Fig. 38 is a graph showing examined results of a relationship between the kind of the detection subject and photocurrent in Example 2-6.

### DETAILED DESCRIPTION OF THE PREFERRED EMBODIMENTS

The preferred embodiments of the present disclosure will be described hereinafter with reference to the drawings.

### [Configuration of detector]

An example of the detector to be used in the method for electrochemically detecting an analyte according to the first and second embodiments of the present disclosure will be explained with reference to the accompanying drawings.

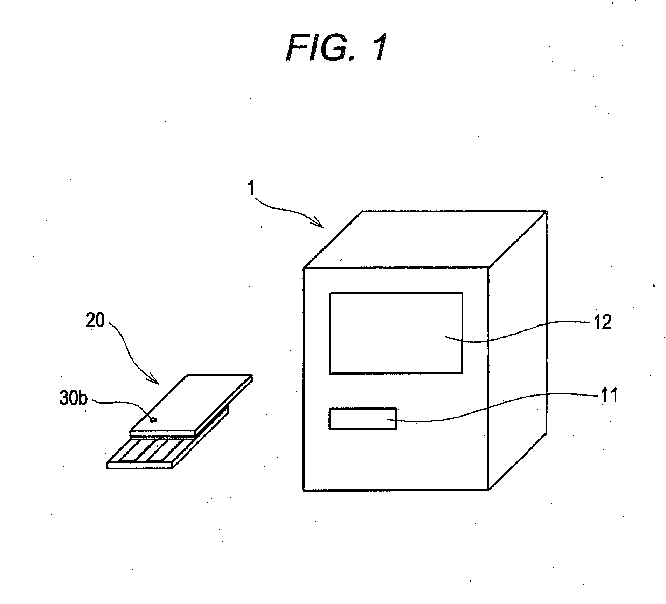

Referring to Fig. 1, a detector 1 is used for the electrochemical detection method which uses a photochemically active substance as a labeling substance.

The detector 1 includes a chip insertion unit 11 into which a detection chip 20 is inserted and a display 12 which displays the detection results.

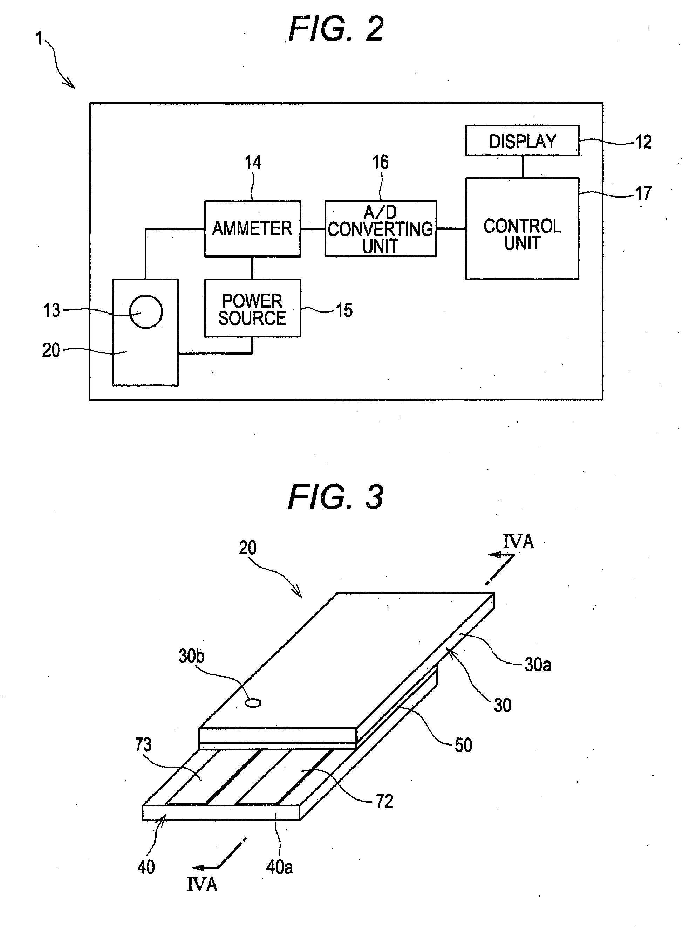

Referring to Fig. 2, the detector 1 includes a light source 13, an ammeter 14, a power source 15, an A/D converting unit 16, a control unit 17, and a display 12.

The light source 13 irradiates a labeling substance present on the working electrode of the detection chip 20 with light to excite the labeling substance. The light source 13 may be a light source which generates excitation light. Examples of the light source include fluorescent lamps, black light, bactericidal lamps, incandescent lamps, low-pressure mercury lamps, high-pressure mercury lamps, xenon lamps, mercury-xenon lamps, halogen lamps, metal halide lamps, light emitting diodes (white LED, blue LED, green LED, and red LED), lasers (carbon dioxide gas lasers, dye lasers, semiconductor lasers), and sunlight. Among the light sources, fluorescent lamps, incandescent lamps, xenon lamps, halogen lamps, metal halide lamps, LEDs, lasers or sunlight is preferred. Particularly, lasers are preferred. The light source may be configured such that only light in a specified wavelength region is emitted by a spectrometer or a bandpass filter, if necessary.

The ammeter 14 measures an electric current which flows through the detection chip 20 due to electrons released from the excited labeling substance.

The power source 15 applies a predetermined potential to an electrode formed in the detection chip 20.

The A/D converting unit 16 digitally converts the photocurrent values measured by the ammeter 14.

The control unit 17 is configured to include a CPU (Central Processing Unit), a ROM (Read Only Memory), and a RAM (Random Access Memory). The control unit 17 controls the operation of the display 12, the light source 13, the ammeter 14, and the power source 15. The control unit 17 estimates the amount of the labeling substance from the photocurrent value which has been digitally converted by the A/D converting unit 16 based on a calibration curve indicating a relationship between a photocurrent value created in advance and the amount of the labeling substance and calculates the amount of the analyte.

The display 12 displays information such as the amount of the analyte which has been estimated by the control unit 17.

When the labeling substance is detected according to the oxidation reduction current/electrochemiluminescence detection method to be described later, the detector may not include the light source 13 (not shown).

When the labeling substance is detected by electrochemical luminescence, the detector may further include a sensor for detecting light generated from the labeling substance.

### [Configuration of detection chip]

Next, the configuration of the detection chip 20 which is used for the method for electrochemically detecting an analyte according to the first and second embodiments of the present disclosure will be described.

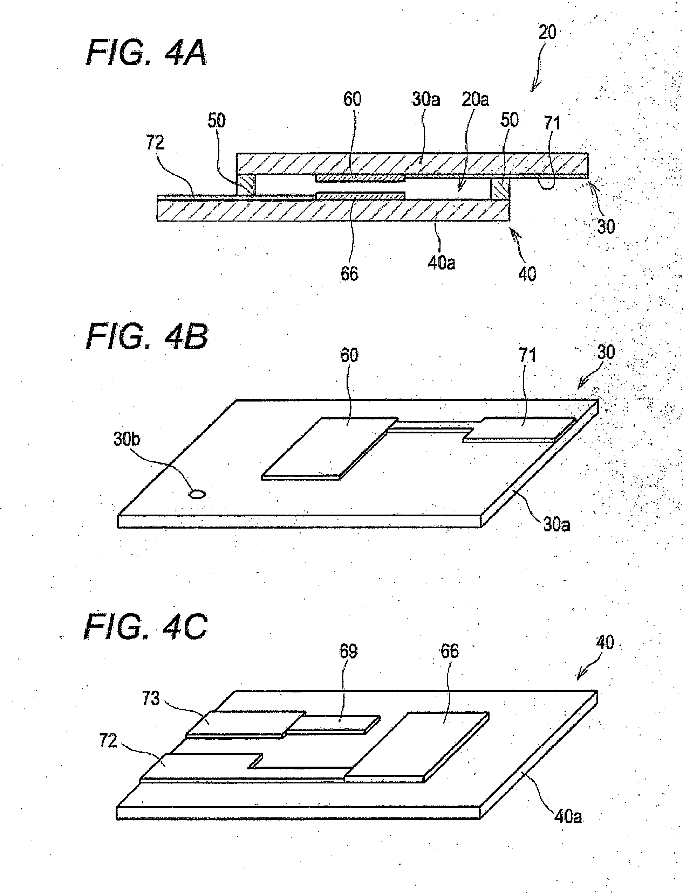

Referring to Fig. 3 and Figs. 4A to 4C, the detection chip 20 includes an upper substrate 30, a lower substrate 40 formed on the lower side of the upper substrate 30, and a spacing member 50 sandwiched between the upper substrate 30 and the lower substrate 40. In the detection chip 20, the upper substrate 30 and the lower substrate 40 are overlappedly arranged at one side portion. The spacing member 50 is intervened in a portion where the upper substrate 30 and the lower substrate 40 are overlapped.

The upper substrate 30 includes a substrate body 30a and a working electrode 60 as shown in Fig. 4B. A sample inlet 30b for injecting a sample containing an analyte into the inside is formed in the substrate body 30a. The working electrode 60 and an electrode lead 71 connected to the working electrode 60 are formed on the surface of the substrate body 30a. In the upper substrate 30, the working electrode 60 is disposed at one side portion [the left side of Fig. 4B] of the substrate body 30a. The electrode lead 71 is extended from the working electrode 60 to the other side portion of the substrate body 30a [the right side of Fig. 4B]. The sample inlet 30b is formed at an inner side than a portion where the spacing member 50 is interposed in the substrate body 30a.

The substrate body 30a is formed into a rectangular shape. The shape of the substrate body 30a is not particularly limited and it may be polygonal, discoid or the like. The shape of the substrate body 30a is preferably rectangular from the viewpoint of the production and easy handling of the substrate.

The material for forming the substrate body 30a is not particularly limited and examples thereof include glass; plastics such as polyethylene terephthalate and polyimide resin; and inorganic materials such as metal. Among them, glass is preferred from the viewpoint of ensuring light transmission properties, sufficient heat resistance, durability, and smoothness and reducing costs required for the materials. The thickness of the substrate body 30a is preferably from 0.01 to 1 mm, more preferably from 0.1 to 0.7 mm, still more preferably about 0.5 mm from the viewpoint of ensuring sufficient durability. The size of the substrate body 30a is not particularly limited, and it is usually about 20 mm x 20 mm and it varies depending on the number of items on the premise of detection of various types of analytes (many items).

The lower substrate 40 includes a substrate body 40a, a counter electrode 66, and a reference electrode 69 as shown in Fig. 4C. The substrate body 40a is formed into a rectangular shape with almost the same size as the substrate body 30a of the upper substrate 30. It does not need that the substrate body 40a has the same size as the substrate body 30a.

The material for forming the substrate body 40a is not particularly limited. Examples thereof include glass, plastics such as polyethylene terephthalate and polyimide resin; and inorganic materials such as metal. Among them, the glass is preferred from the viewpoint of ensuring heat resistance, durability, and smoothness and reducing the cost required for the materials. The thickness and size of the substrate body 40 are the same as those of the substrate body 30a of the upper substrate 30.

The counter electrode 66, an electrode lead 72 connected to the counter electrode 66, the reference electrode 69, and an electrode lead 73 connected to the reference electrode 69 are formed on the surface of the substrate body 40a. In the lower substrate 40, the counter electrode 66 is disposed at one side portion of the substrate body 40a [the right side of Fig. 4C]. The reference electrode 69 is disposed at a position opposed to the counter electrode 66 on the substrate body 40a. The electrode lead 72 of the counter electrode 66 and the electrode lead 73 of the reference electrode 69 are extended from one side portion of the substrate body 40a [the right side of Fig. 4C] to the other side portion [the left side of Fig. 4C]. The electrode leads 72 and 73 are disposed at the other side portion of the substrate body 40a [the left side of Fig. 4C] so as to be parallel to each other. The electrode lead 72 and 73 are protruded from the portion where the upper substrate 30 and the lower substrate 40 are overlapped and exposed to the outside [see Figs. 3 and 4A]. The substrate body 30a and the substrate body 40a are desirably substrate bodies formed of a material having permeability when light is emitted so as to transmit the substrate body. In this case, of the substrate body 30a and the substrate body 40a, the substrate body to be irradiated with light may be formed from the material having permeability.

Subsequently, the working electrode 60, the counter electrode 66, and the reference electrode 69 will be explained in detail.

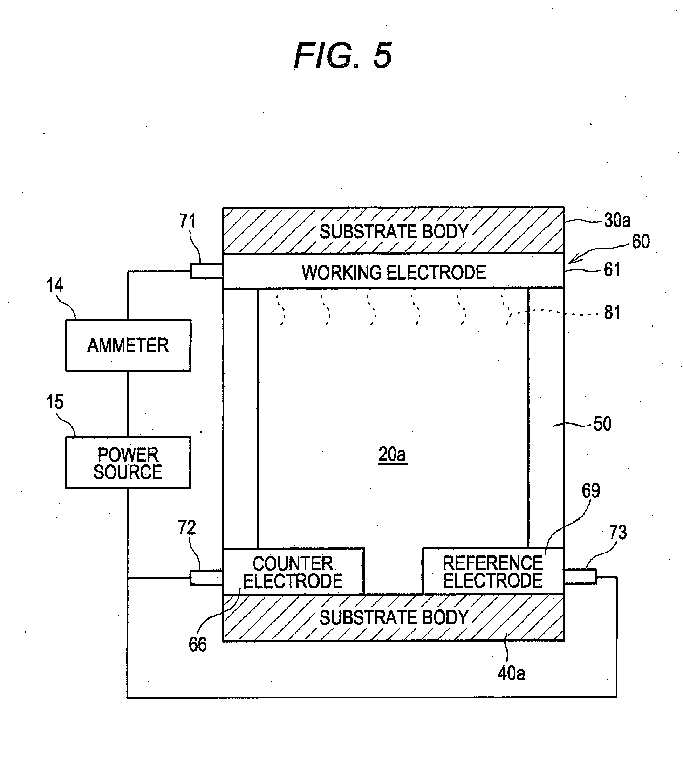

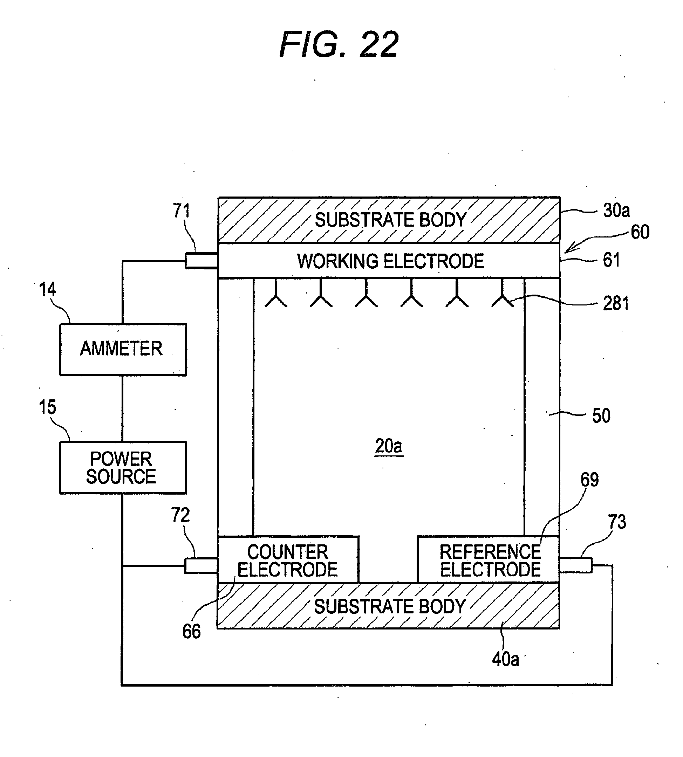

Referring to Figs. 5 and 22, the working electrode 60 is formed into a nearly rectangular shape. The working electrode 60 is configured to include a working electrode body 61 formed on the substrate body 30a and trapping substances 81 or 281 immobilized on the working electrode body 61 as shown in Figs. 5 and 22. The electrode lead 71 is connected to the working electrode body 61.

In the detection chip which is used for the photoelectrochemical detection method to be described later, the working electrode body 61 is formed of a semiconductor which receives electrons from the analyte generated by irradiation with excitation light. The semiconductor functions as a conductive body and an electron acceptor. The semiconductor may be a substance which may have an energy level capable of injecting electrons from the analyte excited by light. Here, the term "energy level capable of injecting electrons from the analyte excited by light" means a conduction band. That is, the semiconductor may have an energy level lower than an energy level of lowest unoccupied molecular orbital of the labeling substance (LUMO) to be described later. The semiconductor is not particularly limited. Examples thereof include element semiconductors such as silicon and germanium; oxide semiconductors containing oxides of titanium, tin, zinc, iron, tungsten, zirconium, hafnium, strontium, indium, cerium, yttrium, lanthanum, vanadium, and niobium, tantalum; perovskite-type semiconductors such as strontium titanate, calcium titanate, sodium titanate, vanadium titanate, and potassium niobate; sulfide semiconductors containing sulfides of cadmium, zinc, lead, silver, antimony, and bismuth; semiconductors containing nitrides of gallium and titanium; semiconductors composed of selenides of cadmium and lead (e.g. cadmium selenide); semiconductors containing telluride of cadmium; semiconductors composed of phosphorus compounds of zinc, gallium, indium, and cadmium; and semiconductors containing compounds such as gallium arsenide, copper-indium selenide, and copper-indium sulfide; and compound semiconductors of carbon or organic semiconductors. The semiconductors may be either intrinsic semiconductors or extrinsic semiconductors. Among the above semiconductors, the oxide semiconductors are preferred. Among the intrinsic semiconductors of the oxide semiconductors, titanium oxide, zinc oxide, tin oxide, niobium oxide, indium oxide, tungsten oxide, tantalum oxide, and strontium titanate are preferred. Among the extrinsic semiconductors of the oxide semiconductors, indium oxide (ITO) which includes tin as a dopant and tin oxide (FTO) which includes fluorine as a dopant are preferred. The thickness of the working electrode is usually from 0.1 to 1 µm, preferably from 0.1 to 200 nm, more preferably from 0.1 to 10 nm.

In the present disclosure the working electrode body 61 in the detection chip to be used for the photoelectrochemical detection method may be formed of a semiconductor layer and a conductive layer. In this case, the electrode lead 71 of the working electrode body 61 is connected to the conductive layer.

A semiconductor for forming the semiconductor layer is the same as the above-described semiconductor. In this case, the thickness of the semiconductor layer is preferably from 0.1 to 100 nm, more preferably from 0.1 to 10 nm.

The conductive layer is formed of a conductive material. Examples of the conductive material include metals such as gold, silver, copper, carbon, platinum, palladium, chromium, aluminium, and nickel or an alloy containing at least one of those metals; indium oxide-based materials such as indium oxide and indium oxide (ITO) which includes tin as a dopant; tin oxide-based materials such as tin oxide, tin oxide (ATO) which includes antimony as a dopant, and tin oxide (FTO) which includes fluorine as a dopant; titanium-based materials such as titanium, titanium oxide, and titanium nitride; and carbon-based materials such as graphite, glassy carbon, pyrolytic graphite, carbon paste, and carbon fiber. The thickness of the conductive layer is preferably from 1 to 1000 nm, more preferably from 1 to 200 nm, still more preferably from 1 to 100 nm. The thickness of the conductive layer is desirably a film thickness capable of ensuring the conductivity and making the photocurrent generated from the electrode (back ground current) a minimum photocurrent. The conductive base material may be a composite base material in which a conductive material layer composed of a material having conductivity is formed on the surface of a nonconductive base material composed of nonconductive substances such as glass and plastics. The shape of the conductive material layer may be filmy or spot-like. Examples of the material for forming the conductive material layer include indium oxide (ITO) which includes tin as a dopant, tin oxide (ATO) which includes antimony as a dopant, and tin oxide (FTO) which includes fluorine as a dopant. The conductive layer is formed, for example, by a film formation method according to the type of the material for forming the conductive layer.

On the other hand, in the detection chip which is used for the oxidation reduction current/electrochemiluminescence detection method to be described later, the working electrode body 61 is composed of a conductive material.

The conductive material is the same as that used for the conductive layer of the working electrode body 61 in the detection chip to be used for the photoelectrochemical detection method.

The conductive material may be a composite base material in which a conductive material layer composed of a material having conductivity is formed on the surface of a nonconductive base material composed of nonconductive substances such as glass and plastics. The shape of the conductive material layer may be filmy or spot-like.

In this case, the thickness of the working electrode body 61 is preferably from 1 to 1000 nm, more preferably from 10 to 200 nm.

The trapping substances 81 or 281 are immobilized on the surface of the working electrode body 61 [see Figs. 5 and 22]. A trapping substance 81 or 281 is a substance which traps the analyte. Accordingly, the analyte is allowed to be present near the working electrode body 61. The trapping substance 81 or 281 can be appropriately selected depending on the type of the analyte. Examples of the trapping substance 81 or 281 include nucleic acids, proteins, peptides, sugar chains, antibodies, and nanostructures with specific recognition ability.

The counter electrode 66 is formed on the substrate body 40a as shown in Figs. 5 and 22. The counter electrode 66 is composed of a thin film of a conductive material. Examples of the conductive material include metals such as gold, silver, copper, carbon, platinum, palladium, chromium, aluminum, and nickel or an alloy containing at least one of those metals; conductive ceramics such as ITO and indium oxide; metal oxides such as ATO and FTO; and titanium compounds such as titanium, titanium oxide, and titanium nitride. The thickness of the thin film composed of a conductive material is preferably from 1 to 1000 nm, more preferably from 10 to 200 nm.

The reference electrode 69 is formed on the substrate body 40a as shown in Figs. 5 and 22. The reference electrode 69 is composed of a thin film of a conductive material. Examples of the conductive material include metals such as gold, silver, copper, carbon, platinum, palladium, chromium, aluminum, and nickel or an alloy containing at least one of those metals; conductive ceramics such as ITO and indium oxide; metal oxides such as ATO and FTO; and titanium compounds such as titanium, titanium oxide, and titanium nitride. The thickness of the thin film composed of a conductive material is preferably from 1 to 1000 nm, more preferably from 10 to 200 nm. Although the reference electrode 69 is formed in the present embodiment, it is not necessary to form the reference electrode 69 in the present disclosure. Depending on the type and film thickness of the electrode to be used for the counter electrode 66, when a small current (e.g. 1 µA or less) to be less affected by the voltage drop influences is measured, the counter electrode 66 may serve as the reference electrode 69. On the other hand, when measuring a large current, it is preferable to form the reference electrode 69 from the viewpoint of suppressing voltage drop influences and stabilizing a voltage to be applied to the working electrode 60.

Subsequently, the spacing member 50 will be explained. The spacing member 50 is formed into a rectangular-circular shape and is composed of silicone rubber which is an insulating material. The spacing member 50 is arranged so as to surround the working electrode 60, the counter electrode 66, and the reference electrode 69 [see Figs. 4A, 5, and 22]. A space corresponding to the thickness of the spacing member 50 is formed between the upper substrate 30 and the lower substrate 40. Thus, a space 20a for housing a sample and an electrolytic solution is formed among the electrodes (the working electrode 60, the counter electrode 66, and the reference electrode 69) [see Figs. 4A, 5, and 22]. The thickness of the spacing member 50 is usually from 0.2 to 300 µm. In the present disclosure, in place of silicone rubber, a double-sided plastic tape such as a polyester film can also be used as the material for forming the spacing member 50.

In the present disclosure, the working electrode 60, the counter electrode 66, and the reference electrode 69 may be arranged in a frame of the spacing member 50 so as not to bring the electrodes into contact with other electrodes. Therefore, the working electrode 60, the counter electrode 66, and the reference electrode 69 may be formed on the same substrate body. In the present disclosure, the counter electrode 66 and the reference electrode 69 may not be a film-like electrode formed on the substrate body. In this case, at least one of the counter electrode 66 and the reference electrodes 69 may be formed on the member body of the spacing member 50. The electrodes other than the electrode formed on the member body of the spacing member 50 may be formed on either the upper substrate 30 or the lower substrate 40.

### - First embodiment -

### [Method for electrochemically detecting analyte]

The method for electrochemically detecting an analyte of the present disclosure according to the first embodiment is a method for electrochemically detecting an analyte comprising:
(1) bringing a sample containing an analyte into contact with a working electrode on which a trapping substance for trapping the analyte is immobilized to allow the analyte to be trapped by the trapping substance on the working electrode;
(2) forming a complex containing the analyte trapped by the trapping substance on the working electrode obtained by the process (1) and a label binding substance in which a labeling substance and a binding substance trapping the analyte are at least retained by a support composed of polypeptide; and
(3) electrochemically detecting the labeling substance present on the working electrode obtained by the process (2).

A major characteristic of the method for electrochemically detecting an analyte according to the first embodiment of the present disclosure is that polypeptide is used as a support of the labeling substance.

In the method for electrochemically detecting an analyte according to the first embodiment of the present disclosure, for example, a label binding substance in which a binding substance to be bound to the analyte S is linked to many labeling substances via a polypeptide support composed of polypeptide having a nano size and low specific gravity is used. Thus, when the polypeptide support is used, it is possible to link many labeling substances while maintaining the avidities of the binding substances, unlike the case where the labeling substance is directly linked to the binding substance not via the polypeptide support. Therefore, according to the method for electrochemically detecting an analyte according to the first embodiment of the present disclosure, it is possible to significantly improve the detection sensitivity. The polypeptide has the structure and sequence determined for each species. Additionally, the number of a bonding site to which the labeling substance can be bound (an amino group or sulfhydryl group as a side chain of an amino acid) is determined. Therefore, according to the method for electrochemically detecting an analyte according to the first embodiment of the present disclosure, it is possible to detect and quantify an analyte with high reproducibility as compared with the case where a support other than polypeptide, such as a metal nanoparticle is used. Further, the polypeptide can be synthesized in vitro. An amino acid residue to which the labeling substance can be bound (amino acid residue having an amino group or a thiol group) can be introduced into a desired site by genetic engineering. The number of the labeling substance in the label binding substance can be increased with sufficient controllability.

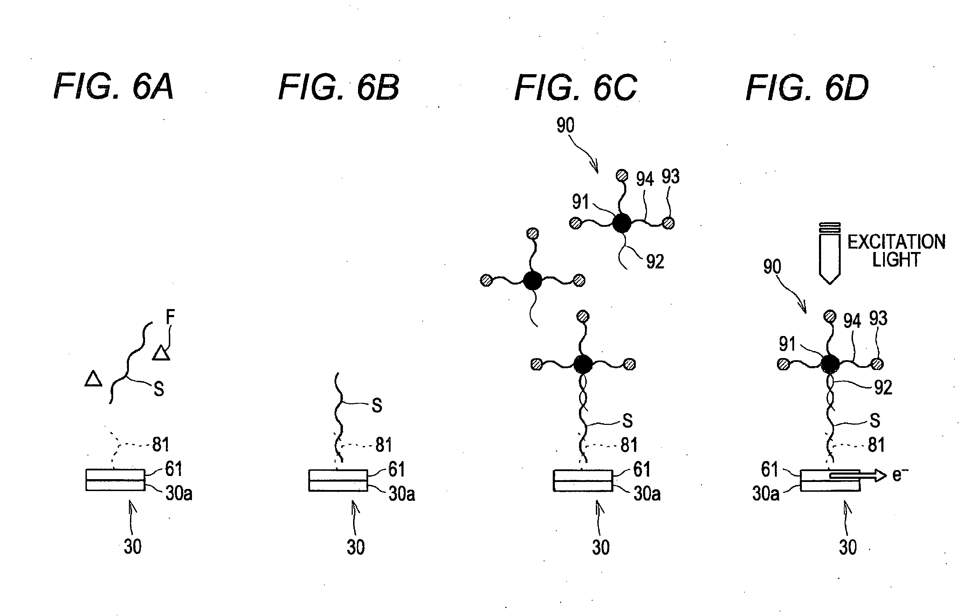

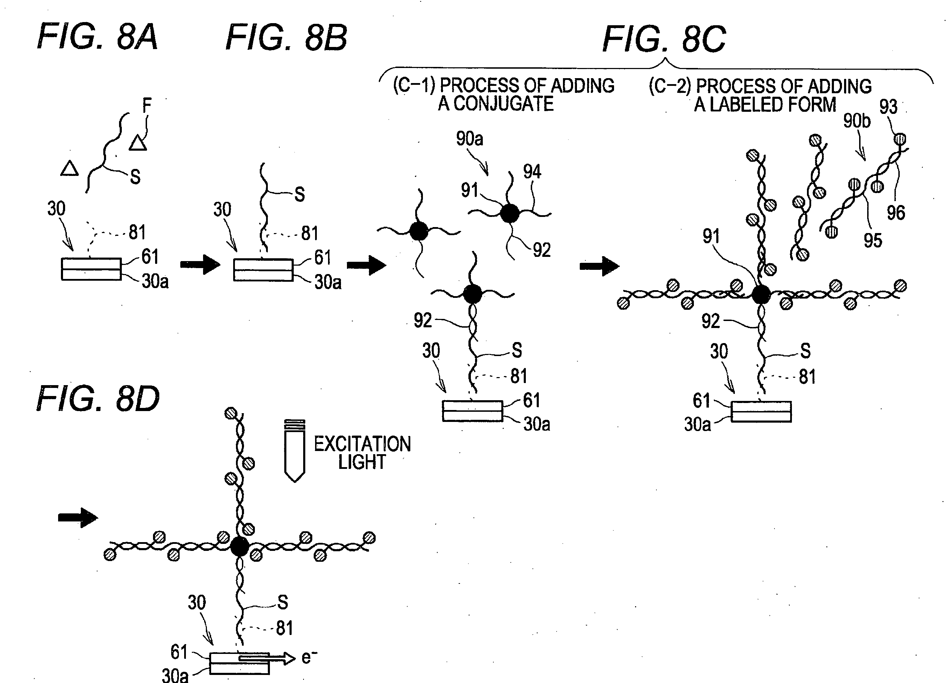

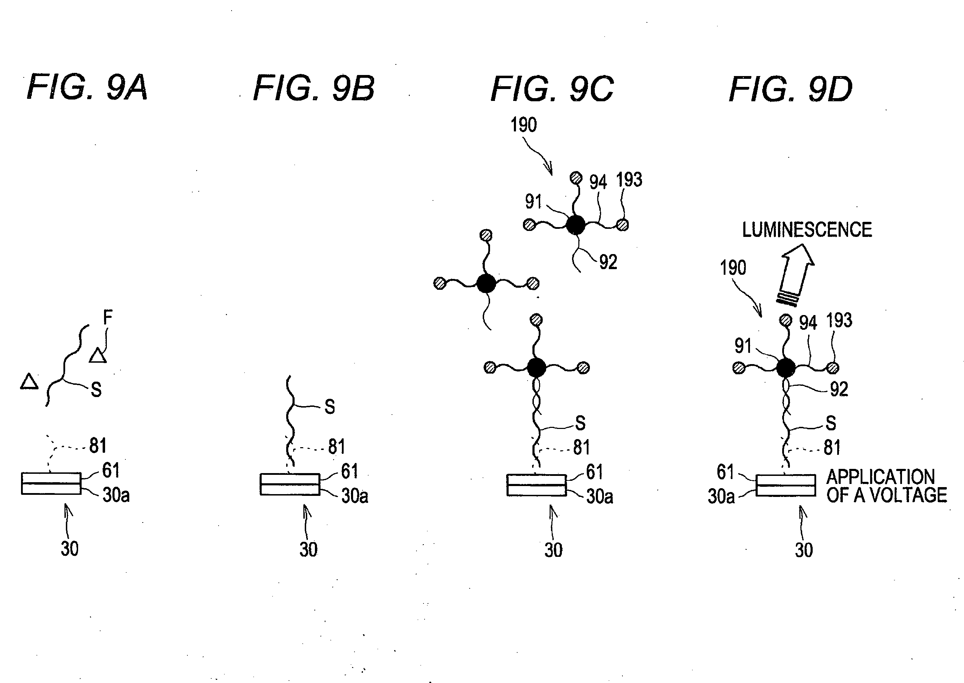

In the method according to the first embodiment of the present disclosure, a photochemically or electrochemically active substance is used as the labeling substance. The photochemically active substance is detected using electrons released by excitation of the substance by light. On the other hand, the electrochemically active substance is detected using an oxidation reduction current and/or electrochemical luminescence based on the substance. Therefore, the method according to the first embodiment of the present disclosure can be divided broadly into the photoelectrochemical detection method (see Figs. 6 and 8) and the oxidation reduction current/electrochemiluminescence detection method (see Fig. 9) depending on the type of detection technique of the labeling substance.

### 1.Photoelectrochemical detection method

First, the photoelectrochemical detection method will be explained. In the photoelectrochemical detection method, the detector illustrated in Fig. 1 and the detection chip illustrated in Fig. 3 can be used, however, they are not limited thereto.

Hereinafter, the method will be explained taking an example of the case of using the detector illustrated in Fig. 1 and the detection chip illustrated in Fig. 3.

Referring to Fig. 6, in the photoelectrochemical detection method, a user injects a sample containing the analyte S through the sample inlet 30b of the detection chip 20 [see the process of supplying a sample of Fig. 6A]. Thus, the analyte in the sample is trapped by the trapping substance 81 on the working electrode body 61 of the upper substrate 30 constituting the detection chip 20 [see the process of trapping an analyte of Fig. 6B]. In this case, substances (contaminants F) other than the analyte S in the sample are not trapped by the trapping substance 81.

The trapping substance 81 can be suitably selected depending on the type of the analyte S. For example, when the analyte S is a nucleic acid, a nucleic acid probe hybridizing to the nucleic acid, an antibody to the nucleic acid, a protein binding to the nucleic acid or the like can be used as the trapping substance 81. When the analyte S is a protein or peptide, an antibody to the protein or peptide can be used as the trapping substance 81.

The process of trapping an analyte by the trapping substance 81 can be performed for example, under conditions where the trapping substance 81 is bound to the analyte. The conditions where the trapping substance 81 is bound to the analyte can be suitably selected depending on the type of the analyte. For example, when the analyte is a nucleic acid and the trapping substance 81 is a nucleic acid probe to be hybridized with the nucleic acid, the process of trapping an analyte can be performed in the presence of a hybridization buffer. When the analyte is a nucleic acid, protein or peptide and the trapping substance 81 is an antibody to nucleic acid, an antibody to protein or an antibody to peptide, the process of trapping an analyte can be performed in a solution suitable for performing an antigen-antibody reaction, such as phosphate buffered saline, a HEPES buffer, a PIPES buffer or a Tris buffer. When the analyte is a ligand and the trapping substance 81 is a receptor to ligand, or when the analyte is a receptor and the trapping substance 81 is a ligand to receptor, the process of trapping an analyte can be performed in a solution suitable for binding the ligand to the receptor.

Then, the user injects the label binding substance 90 into the detection chip 20 from the sample inlet 30b to allow the label binding substance 90 to be bound to the analyte S trapped on the working electrode body 61 [see the labeling process of Fig. 6C]. In the labeling process, a complex containing the trapping substance 81, the analyte S, and the label binding substance 90 is formed on the working electrode body 61.

The label binding substance 90 is formed of a polypeptide support 91, a first binding substance 92 to be bound to the analyte S, a labeling substance 93, and a first linker 94. In the label binding substance 90, the first binding substance 92 to be bound to the analyte S and the first linker 94 are directly immobilized on the surface of the polypeptide support 91. The labeling substance 93 is immobilized on the support 91 via the first linker 94.

The polypeptide support 91 is composed of polypeptide. The diameter of the polypeptide support 91 can be suitably set depending on the type of the analyte and the labeling substance and it is usually from 3 to 100 nm.

The polypeptide may be any of a naturally occurring purified polypeptide, a recombinant polypeptide synthesized in vitro, a genetically modified artificial polypeptide, and a chemically synthesized peptide.

The molecular weight of the polypeptide is preferably from 1000 to 1000000 Da, more preferably from 10000 to 700000 Da, still more preferably from 50000 to 500000 Da.

The shape of the polypeptide may be any shape capable of being formed by the polypeptide. Examples of the shape of the polypeptide include spherical, linear, and string shapes. However, the present disclosure is not limited only thereto.

The polypeptide may contain an amino acid residue which is easily bound to the labeling substance. Examples of the amino acid residue include amino acid residues having a primary amino group at the side chain (e.g. a lysine residue, an asparagine residue, and a glutamine residue) and amino acid residues having a sulfhydryl group (e.g. a cysteine residue). Among them, a polypeptide containing many lysine residues, namely, a strongly basic protein is effective in order to bind many labeling substances. Specific examples of the polypeptide include albumin (e.g. bovine serum albumin), polylysine, histone H1, myelin basic protein (MBP), and albumen lysozyme. However, the present disclosure is not limited only thereto.

The polypeptide constituting the polypeptide support 91 may be a polypeptide composed of a multimer in which a plurality of subunits are associated. In this case, the multimer may be a homo-multimer in which each subunit is mutually the same or may be a hetero-multimer in which each subunit is mutually different. Further, the multimer may be a multimer in which each subunit is mutually homologous. Examples of the polypeptide composed of such a multimer include a polypeptide having a homo multimer structure, such as ferritin, streptoavidin or Listeria Dps; and a polypeptide produced by using the outer shells of particles of viruses such as HSV (simple herpes virus), Rotavirus, Reovirus, poliovirus, Ross river virus and poliovirus. However, the present disclosure is not limited only thereto.

Among the polypeptides, ferritin and albumin are preferred from the viewpoint of the easy bulk preparation at low cost. Preferably, ferritin is modified so that a bonding site to which the labeling substance can be bound is located at the outside of a ferritin molecule. As the ferritin modified in such a manner, for example, a variant ferritin in which the 86th serine residue located at the outside of a horse ferritin molecule is modified to a cysteine residue to which the labeling substance by modified with maleimide can be bound (cysteine residue having high reactivity with a maleimide group) is listed.

The first binding substance 92 may be a substance which binds to a position or site in the analyte S, which is different from that of the trapping substance 81. The first binding substance 92 is suitably selected depending on the type of the analyte S. For example, when the analyte S is a nucleic acid, a nucleic acid probe hybridizing to the nucleic acid, an antibody to the nucleic acid, a protein binding to the nucleic acid or the like can be used as the first binding substance 92. When the analyte S is a protein or peptide, an antibody to the protein or peptide can be used as the first binding substance 92.

The labeling substance 93 is a substance which becomes in an excited state when irradiated with light and releases electrons. As the labeling substance 93, at least one selected from the group consisting of a metal complex, an organic phosphor, a quantum dot, and an inorganic phosphor can be used.

Specific examples of the labeling substance include metal phthalocyanine dyes, a ruthenium complex, an osmium complex, an iron complex, a zinc complex, 9-phenylxanthene-based dyes, cyanine-based dyes, metallocyanine dyes, xanthene-based dyes, triphenylmethane-based dyes, acridine-based dyes, oxazine-based dyes coumarin-based dyes, merocyanine-based dyes, rhodacyanine-based dyes, polymethine-based dyes, porphyrin-based dyes, phthalocyanine-based dyes, rhodamine-based dyes, xanthene-based dyes, chlorophyl-based dyes, eosine-based dyes, mercurochrome-based dyes, indigo-based dyes, BODIPY-based dyes, CALFluor-based dyes, Oregon green-based dyes, Rhodol green, Texas red, Cascade blue, nucleic acids (DNA and RNA), cadmium selenide, cadmium telluride, Ln₂O₃:Re, Ln₂O₂S:Re, ZnO, CaWO₄, MO · xAl₂O₃:Eu, Zn₂SiO₄:Mn, LaPO₄:Ce, Tb, Cy3, Cy3.5, Cy5, Cy5.5, Cy7, Cy7.5, and Cy9 (all products are manufactured by Amersham Biosciences K.K.); Alexa Fluor 355, Alexa Fluor 405, Alexa Fluor 430, Alexa Fluor 488, Alexa Fluor 532, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, Alexa Fluor 750 and Alexa Fluor 790 (all products are manufactured by Molecular Probes, Inc.); DY-610, DY-615, DY-630, DY-631, DY-633, DY-635, DY-636, EVOblue10, EVOblue30, DY-647, DY-650, DY-651, DY-800, DYQ-660, and DYQ-661 (all products are manufactured by Dyomics); Atto425, Atto465, Atto488, Atto495, Atto520, Atto532, Atto550, Atto565, Atto590, Atto594, Atto610, Atto611X, Atto620, Atto633, Atto635, Atto637, Atto647, Atto655, Atto680, Atto700, Atto725 and Atto740 (all products are manufactured by Atto-TEC GmbH); and VivoTagS680, VivoTag680, and VivoTagS750 (all products are manufactured by VisEn Medical). Ln represents La, Gd, Lu, or Y, Re represents a lanthanide element, M represents an alkali earth metal element, and x represents a number of 0.5 to 1.5. Concerning other examples of the labeling substance, refer to, for example, U.S. Patent Publication No. 2009/294305 and U.S. Patent No. 5893999.

Examples of the first linker 94 include a carbon chain, a polyethylene glycol (PEG) chain, and nucleic acid. Since a suitable range of the length of the linker varies depending on the type of labeling substances or functional groups, it is preferable that the range is suitably set according to the type of labeling substances or functional groups.

The label binding substance 90 may not have the first linker 94 as long as it can directly bind the labeling substance 93 to the polypeptide support 91.

Examples of the method for binding the labeling substance 93 to the polypeptide support 91 in the label binding substance 90 include a method for binding the labeling substance 93 to the polypeptide support 91 by a covalent bond and a method for binding the labeling substance 93 to the polypeptide support 91 by a non-covalent bond.

The method for binding the labeling substance 93 to the polypeptide support 91 by a covalent bond may be a method capable of covalently binding the labeling substance 93 to a polypeptide and it is not particularly limited.

In the polypeptide support 91, a site to which the labeling substance 93 is covalently bound is not particularly limited. From the viewpoint that the binding of the polypeptide support 91 to the labeling substance 93 is easy, an amino group (NH₂) in a polypeptide and a sulfhydryl (SH) group are preferred. Examples of a reaction group capable of binding to an amino group (NH₂) in a polypeptide include a succinimido group (NHS), an isothiocyano group (ITC), a chlorosulfonyl group, a chloroacyl group, an oxyethylene group, a chloroalkyl group, an aldehyde group, and a carboxyl group. Among them, NHS and ITC are preferred because when a target labeling substance is covalently bound via an amino group of polypeptide, a reaction in an aqueous system is essential, and the conditions capable of using a reaction compound are limited such that the pH of the reaction solution is in a neutral to weak alkaline region and the reaction is progressed at a reaction temperature of ice-cooling to about 37°C for a short time. Therefore, a labeling substance having NHS and/or ITC can be used as the labeling substance 93.

Examples of the reaction group capable of binding to a sulfhydryl (SH) group in polypeptide include a maleimide group and a bromoacetamide group. The sulfhydryl (SH) group normally forms a disulfide (S-S) bond in a polypeptide. Thus, when the sulfhydryl (SH) group is used as a site for binding to the labeling substance, the disulfide structure in the polypeptide is reduced to be used as a sulfhydryl group (SH). In the reduction of the disulfide bond, dithiothreitol (DTT), β-mercaptoethanol (β-ME), and mercaptoethylamine (MEA) can be used. Therefore, when the labeling substance has a functional group having high reactivity with an amino group or a thiol group (e.g. a succinimido group and a maleimide group), the labeling substance can be directly bound to the amino and thiol groups of polypeptide by mixing the polypeptide with the labeling substance. Examples of the labeling substance include Alexa Fluor750 modified with succinimide ester and Alexa Fluor790 (manufactured by Invitrogen).

When the labeling substance 93 has an amino group, a thiol group, an aldehyde group, and a carboxyl group, the labeling substance 93 can be easily bound to the polypeptide support 91 by binding the labeling substance 93 to the polypeptide support 91, for example, via a chemical cross-linker; forming a dithiol bond between the labeling substance 93 and the polypeptide support 91; and performing a general chemical reaction.

The cross-linker generally has a linear structure and is formed of a spacer having a succinimido group which reacts with amino and thiol groups as well as a maleimide group at the both ends. The use of the cross-linker as the first linker 94 allows the polypeptide support 91 to be linked to the labeling substance 93. For example, when the labeling substance 93 has a thiol group, a cross-linker having a succinimido group at one end and having a maleimide group at the other end can be used in binding the labeling substance 93 to the amino group of polypeptide. In this case, the amino group in polypeptide is reacted with the succinimide group in the cross-linker, and the maleimide group of the cross-linker is exposed to the surface of polypeptide. The binding can be performed by reacting the maleimide group with the thiol group in the labeling substance. Here, the length of the spacer of the cross-linker is not particularly limited. Examples of the spacer include PEG chains and nucleic acids. Examples of the specific example of the cross-linker include N-[α-maleimide acetoacetoxy]succinimide ester (AMAS), N-[β-maleimide propyloxy] succinimide ester (BMPS), maleimide butyryloxy succinimide ester (GMBS), m-maleimide benzoyl-N-hydroxysuccinimide ester (MBS), succinimidyl trans-4-(N-maleimidyl methyl)-cyclohexane-1-carboxylate (SMCC), N-[ε-maleimide caproyl oxy] succinimide ester (EMCS), succinimidyl-4-(p-maleimide phenyl)butyrate (SMPB), succinimidyl-6-[(β-maleimide propionamide)hexanoate] (SMPH), succinimidyl-4-[N-maleimidemethyl]cyclohexane-1-carboxy-[6-amide caproate] (LC-SMCC), and NHS-PEGn-Maleimid. The cross-linker may be glutaraldehyde in which functional groups at both ends have reactivity with an amino group, a cross-linker which has two functional groups (an amine-reactive NHS ester group and a light-reactive diazirine group) at the end or the like.

When the labeling substance 93 has a thiol group, the binding is possible by reacting the thiol group of the labeling substance 93 with the thiol group of polypeptide to form a dithiol bond. When the labeling substance 93 has a carboxyl group, the labeling substance 93 can be bound to the amino group of polypeptide by activating using NHS. When the labeling substance 93 has an aldehyde group, a stable bond can be formed by forming a Schiff base with the amino group of polypeptide and reducing it.

As the method for binding the polypeptide support 91 to the labeling substance 93 by a non-covalent bond, a method for binding the labeling substance 93 to polypeptide by a non-covalent and a method for binding the labeling substance 93 via a substance bound to polypeptide by a covalent bond by a non-covalent bond are contemplated.

Examples of the method for binding the labeling substance 93 to polypeptide by a non-covalent bond include a method for utilizing binding of streptoavidin to a labeling substance labeled with biotin and the like. Examples of the method for binding the labeling substance via a substance bound to polypeptide by a covalent bond by a non-covalent bond include a method comprising covalently-binding DNA having an amino group at the end to polypeptide in the above manner and non-covalently binding complementary DNA to which the labeling substance is bound to the DNA by hybridization and the like.

As described above, when the polypeptide support 91 is used as a support of the labeling substance 93, a labeling substance-binding substance in which the sum of labeling substances is accurately controlled can be easily produced as compared with the case where a support composed of an inorganic material is used.

The method for binding the first binding substance 92 to the polypeptide support 91 is performed by the same method as the method for binding the labeling substance 93 to the polypeptide support 91.

Subsequently, the detection process is performed [see the detection process of Fig. 6D].

In the detection process, the user first injects an electrolytic solution through the sample inlet 30b of the detection chip 20. Thereafter, the user inserts the detection chip 20 into the chip insertion unit 11 of the detector 1 shown in Fig. 1. Then, the user gives an instruction to start measuring to the detector 1. Here, the electrode leads 71, 72, and 73 of the detection chip 20 inserted into the detector 1 are connected to the ammeter 14 and the power source 15. Then, an arbitrary potential based on the reference electrode 69 is applied to the working electrode 60 by the power source 15 of the detector 1. As the potential to be applied to the electrode, a potential in which the current value (stationary current, dark current) is low when the analyte is not irradiated with excitation light and the photocurrent generated from the analyte becomes a maximum photocurrent is preferred. The potential may be applied to the counter electrode or the working electrode.

Thereafter, the light source 13 of the detector 1 emits excitation light to the labeling substance 93 on the working electrode 60. Thus, the labeling substance 93 is excited to generate electrons. The generated electrons move to the working electrode 60. As a result, current flows between the working electrode 60 and the counter electrode 66. Then, the current flowing between the working electrode 60 and the counter electrode 66 is measured by the ammeter 14 of the detector 1. The current value measured by the ammeter 14 correlates with the number of the labeling substance 93. Therefore, the analyte S can be quantified based on the measured current value. The excitation light may be only light in a specified wavelength region, which is obtained using a spectrometer or a bandpass filter, if necessary.

Thereafter, a current value digitally converted by the A/D converting unit 16 is input into the control unit 17. Then, the control unit 17 estimates the amount of the analyte in the sample from the digitally converted current value based on a calibration curve indicating a relationship between a current value created in advance and the amount of the analyte. The control unit 17 creates a detection result screen for displaying the information on the estimated amount of the analyte on the display 12. Thereafter, the detection result screen created by the control unit 17 is sent to the display 12 so as to be displayed on the display 12.

As the electrolytic solution, a solution containing an electrolyte composed of salts which may supply electrons to the labeling substance 93 in an oxidized state, an aprotic polar solvent, a protonic polar solvent, or a mixture of the aprotic polar solvent and the protonic polar solvent can be used. The electrolytic solution may further contain other components, if desired. The electrolytic solution may be in gel or solid form.

Examples of the electrolyte include iodide, bromide, a metal complex, thiosulfate, sulfite, and a mixture thereof. Specific examples of the electrolyte include metal iodides such as lithium iodide, sodium iodide, potassium iodide, cesium iodide, calcium iodide; iodine salts of quaternary ammonium compounds such as tetraalkylammonium iodide, pyridinium iodide, and imidazolium iodide; metal bromides such as lithium bromide, sodium bromide, potassium bromide, cesium bromide, and calcium bromide; bromine salts of quaternary ammonium compounds such as tetraalkylammonium bromide and pyridinium bromide; metal complexes such as ferrocyanic acid salt and ferricinium ion; thiosulfate salts such as sodium thiosulfate, ammonium thiosulfate, potassium thiosulfate, and calcium thiosulfate; sulfites such as sodium sulfite, potassium sulfite, ammonium sulfite, iron sulfite, sodium bisulfite, and calcium sulfite; and mixtures thereof. Among them, tetrapropylammonium iodide and calcium iodide are preferred.

The electrolyte concentration of the electrolytic solution is preferably from 0.001 to 15 M.

Water, a polar solvent containing a buffer component and a main component of water, or the like may be used as the protonic polar solvent.

Examples of the aprotic polar solvent include nitriles such as acetonitrile (CH₃CN); carbonates such as propylene carbonate and ethylene carbonate; heterocyclic compounds such as 1,3-dimethylimidazolinone, 3-methyloxazolinone and dialkylimidazolium salt; dimethylformamide, dimethyl sulfoxide, and sulfolane. Among the aprotic polar solvents, acetonitrile is preferred. The protonic polar solvent and the aprotic polar solvent can be used alone or mixed for use. As a mixture of the protonic polar solvent and the aprotic polar solvent, a mixture of water and acetonitrile is preferred.

When the labeling substance 93 is irradiated with light, a light source which can emit light in a wavelength capable of photoexciting the labeling substance 93 can be used. The light source can be suitably selected depending on the type of the labeling substance 93. Examples of the light source include fluorescent lamps, black light, bactericidal lamps, incandescent lamps, low-pressure mercury lamps, high-pressure mercury lamps, xenon lamps, mercury-xenon lamps, halogen lamps, metal halide lamps, light emitting diodes (white LED, blue LED, green LED, and red LED), lasers (carbon dioxide lasers, dye lasers, semiconductor lasers), and sunlight. Among the light sources, fluorescent lamps, incandescent lamps, xenon lamps, halogen lamps, metal halide lamps, light emitting diodes, and sunlight are preferred. In the detection process, the labeling substance 93 may be irradiated with only light in a specified wavelength region, which is obtained using a spectrometer or a bandpass filter, if necessary.

In the measurement of a photocurrent derived from the labeling substance 93, for example, a measurement device which includes an ammeter, a potentiostat, a recorder, and a computer can be used.

In the detection process, the amount of the analyte can be examined by quantifying the photocurrent.

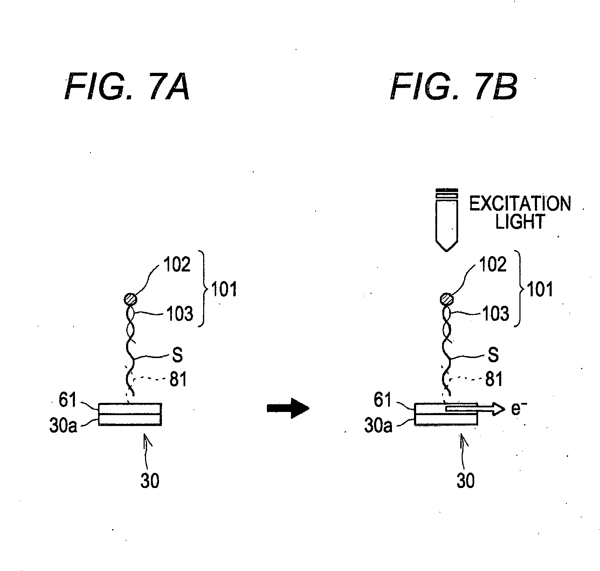

As described above, in the method for electrochemically detecting an analyte according to the first embodiment of the present disclosure, the polypeptide support 91 is used as the support of the labeling substance. Therefore, according to the method for electrochemically detecting an analyte according to the present embodiment, the photocurrent based on the labeling substance per an analyte S can be increased. On the other hand, in the conventional method for electrochemically detecting an analyte, the polypeptide support 91 is not used. As shown in Fig. 7, when detecting the analyte S, a label binding substance 101 in which a labeling substance 102 directly bound to a binding substance 103 which is bound to the analyte S is generally used. Thus, in the conventional method for electrochemically detecting an analyte, the photocurrent based on the labeling substance per an analyte S is small.

In the method for electrochemically detecting an analyte according to the present embodiment, from the viewpoint of suppressing the generation of noises due to contaminants, the user may discharge a remaining liquid containing contaminants from the sample inlet 30b of the detection chip 20 after the process of trapping an analyte and wash an inside of the detection chip 20. In the washing of the inside of the detection chip 20, organic solvents such as a buffer (particularly a buffer containing a surfactant); purified water (particularly purified water containing a surfactant); and ethanol can be used.

In the method for electrochemically detecting an analyte according to the present embodiment, from the viewpoint of removing the label binding substance 90 which is not bound to the analyte S and improving the detection accuracy, the process of washing the inside of the detection chip 20 to remove free label binding substance 90 may be further performed after the labeling process. For example, ethanol and purified water can be used for the washing.

In the present disclosure, the operation may be performed so as to form a label binding substance in the labeling process as shown in Fig. 8C in place of labeling the analyte S using the label binding substance to which the labeling substance is bound in advance in the labeling process. In the method for electrochemically detecting an analyte shown in Fig. 8, the process of supplying a sample (Fig. 8A), the process of trapping an analyte (Fig. 8B), and the detection process (Fig. 8D) are the same as the process of supplying a sample (Fig. 6A), the process of trapping an analyte (Fig. 6B), and the detection process (Fig. 6D) in the above method shown in Fig. 6. On the other hand, in the method for electrochemically detecting an analyte shown in Fig. 8, a conjugate 90a retaining the first binding substance 92 and the first linker 94 is bound to the analyte S via the polypeptide support 91 in the labeling process (Fig. 8C) [the process of adding a conjugate (C-1) of Fig. 8C]. Thereafter, the conjugate 90a is bound to a labeled form 90b [the process of adding a labeled form (C-2) of Fig. 8C]. The labeled form 90b is formed of the labeling substance 93, a second linker 96 for retaining the labeling substance 93, and a second binding substance 95 which binds to the second linker 96. In the labeled form 90b, a plurality of complexes containing the labeling substance 93 and the second linker 96 are linked to the second binding substance 95.

### 2.Oxidation reduction current/electrochemiluminescence detection method

Subsequently, the oxidation reduction current/electrochemiluminescence detection method will be explained.

Referring to Fig. 9, the oxidation reduction current/electrochemiluminescence detection method according to the present embodiment is largely different from the photoelectrochemical detection method in that a labeling substance which generates oxidation reduction current when a voltage is applied or a labeling substance which emits light when a voltage is applied is used as the labeling substance 193 in the labeling process [see the labeling process of Fig. 9C], and a voltage is applied to the working electrode 60 and the light generated from the labeling substance 193 is detected in the detection process [see the detection process of Fig. 9D]. Therefore, the process of supplying a sample [see the process of supplying a sample of Fig. 9A] and the process of trapping an analyte [see the process of trapping an analyte of Fig. 9B] are the same as those in the photoelectrochemical detection method. The detector 1 which is used in the method for electrochemically detecting an analyte according to the present embodiment does not include the light source 13 and includes a sensor for detecting light generated from the labeling substance. In the detection chip 20 to be used in the method for electrochemically detecting an analyte according to the present embodiment, the working electrode 60 is composed of a conductive material.

In the labeling process, the user injects the label binding substance 190 into the detection chip 20 from the sample inlet 30b to allow the label binding substance 190 to be bound to the analyte S trapped on the working electrode body 61 [see the labeling process of Fig. 9C]. In the labeling process, a complex containing the trapping substance 81, the analyte S, and the label binding substance 190 is formed on the working electrode body 61.

The label binding substance 190 is formed of a polypeptide support 91, a first binding substance 92 to be bound to the analyte S, a labeling substance 193, and a first linker 94. In the label binding substance 190, the first binding substance 92 to be bound to the analyte S and the first linker 94 are directly immobilized on the surface of the polypeptide support 91. The labeling substance 193 is immobilized on the polypeptide support 91 via the first linker 94.

The labeling substance 193 is a labeling substance which emits light when a voltage is applied.

Examples of the labeling substance which emits light when a voltage is applied include luminol, lucigenin, pyrene, diphenylanthracene, and rubrene.

The luminescence of the labeling substance can be enhanced, for example, by using luciferin derivatives such as firefly luciferin and dehydro luciferin, enhancers such as phenols such as phenylphenol and chlorophenol or naphthols.

In the oxidation reduction current/electrochemiluminescence detection method for an analyte according to the present embodiment, as the labeling substance 193, a labeling substance which generates oxidation reduction current when a voltage is applied may be used in place of the labeling substance which emits light when a voltage is applied.

Examples of the labeling substance which generates oxidation reduction current when a voltage is applied include metal complexes containing metal which causes an electrically reversible oxidation-reduction reaction as a central metal. Examples of the metal complexes include tris(phenanthroline) zinc complex, tris(phenanthroline) ruthenium complex, tris(phenanthroline) cobalt complex, di(phenanthroline) zinc complex, di(phenanthroline) ruthenium complex, di(phenanthroline) cobalt complex, bipyridine platinum complex, terpyridine platinum complex, phenanthroline platinum complex, tris(bipyridyl) zinc complex, tris(bipyridyl) ruthenium complex, tris(bipyridyl) cobalt complex, di(bipyridyl) zinc complex, di(bipyridyl) ruthenium complex, and di(bipyridyl) cobalt complex.

In the oxidation reduction current/electrochemiluminescence detection method for an analyte according to the present embodiment, the polypeptide support 91, the first binding substance 92, and the first linker 94 are the same as those in the photoelectrochemical detection method.

Subsequently, the detection process is performed [see the detection process in Fig. 9D].

In the detection process, the user first injects an electrolytic solution through the sample inlet 30b of the detection chip 20. Thereafter, the user inserts the detection chip 20 into the chip insertion unit 11 of the detector 1 shown in Fig. 1. Then, the user gives an instruction to start measuring to the detector 1. Here, the electrode leads 71, 72, and 73 of the detection chip 20 inserted into the detector 1 are connected to the ammeter 14 and the power source 15. Then, a voltage is applied to the working electrode 60 by the power source 15 of the detector 1. Thus, the labeling substance 193 is excited to generate light. In the measurement of light based on the labeling substance 193, a photon counter is used. In this case, the light can be indirectly detected by using an optical fiber electrode obtained by forming a transparent electrode at the distal end of an optical fiber in place of the electrode (see U.S. Patent No. 5776672 and U.S. Patent No. 5972692).

Thereafter, a light value digitally converted by the A/D converting unit 16 is input into the control unit 17. Then, the control unit 17 estimates the amount of the analyte in the sample from the digitally converted current value based on a calibration curve indicating a relationship between a light value created in advance and the amount of the analyte. The control unit 17 creates a detection result screen for displaying the information on the estimated amount of the analyte on the display 12. Thereafter, the detection result screen created by the control unit 17 is sent to the display 12 so as to be displayed on the display 12.

In the oxidation reduction current/electrochemiluminescence detection method for an analyte according to the present embodiment, from the viewpoint of suppressing the generation of noises due to contaminants, the user may discharge a remaining liquid containing contaminants from the sample inlet 30b of the detection chip 20 after the process of trapping an analyte and wash an inside of the detection chip 20. In the washing of the inside of the detection chip 20, organic solvents such as a buffer (particularly a buffer containing a surfactant); purified water (particularly purified water containing a surfactant); and ethanol can be used.

In the oxidation reduction current/electrochemiluminescence detection method for an analyte according to the present embodiment, from the viewpoint of removing free label binding substance 190 which is not bound to the analyte S and improving the detection accuracy, the process of washing the inside of the detection chip 20 to remove the label binding substance 190 may be further performed after the labeling process. For example, ethanol and purified water can be used for the washing.

In the oxidation reduction current/electrochemiluminescence detection method for an analyte according to the present embodiment, the operation may be performed so as to form a label binding substance in the labeling process as shown in Fig. 8 in place of labeling the analyte S using the label binding substance 190 to which the labeling substance 193 is bound in advance in the labeling process.

In Fig. 9D, taking the case where the light is measured as an example, the process is illustrated. When the labeling substance 193 is the labeling substance which generates oxidation reduction current when a voltage is applied, the labeling substance 193 is excited to generate electrons. The generated electrons move to the working electrode 60. As a result, current flows between the working electrode 60 and the counter electrode 66. Then, the current flowing between the working electrode 60 and the counter electrode 66 is measured by the ammeter 14 of the detector 1. The current value measured by the ammeter 14 correlates with the number of the labeling substance. Therefore, the analyte can be quantified based on the measured current value.

### [First example]

Hereinafter, the present disclosure will be described in detail with reference to Examples, however, the present disclosure is not limited thereto.

### (Preparation example 1-1 (reference))

1% by volume of 3-aminopropyltriethoxysilane (APTES), i.e., a silane coupling agent, was added to toluene to prepare a solution A.

### (Preparation example 1-2 (reference))

Acetonitrile and ethylene carbonate were mixed at a volume ratio of 2:3 to prepare an aprotic polar solvent. As an electrolyte salt, tetrapropylammonium iodide was dissolved in the aprotic polar solvent at a concentration of 0.6 M. As an electrolyte, iodine was dissolved in the obtained solution at a concentration of 0.06 M to prepare an electrolytic solution.

### (Preparation example 1-3 (reference))

A counter electrode of a 200-nm thick platinum thin film (conductive layer) was formed on the substrate body of silicon dioxide (SiO₂) by the spattering method to obtain a counter electrode substrate. The counter electrode lead for connecting to the ammeter was connected to the counter electrode. Thus, the counter electrode substrate was obtained.

### (Test example 1-1 (reference))

### (1-1) Production of DNA binding ferritin

In order to bind DNA to be used as a binding substance or linker to the outside of ferritin (a support composed of polypeptide), the 86th serine residue located outside was modified to a cysteine residue having high reactivity with a maleimide group by the gene-recombination technology using a kit for mutagenesis of a horse ferritin subunit [trade name: QuikChange Site-Directed Mutagenesis kit, manufactured by Stratagene] to obtain a recombinant ferritin subunit.

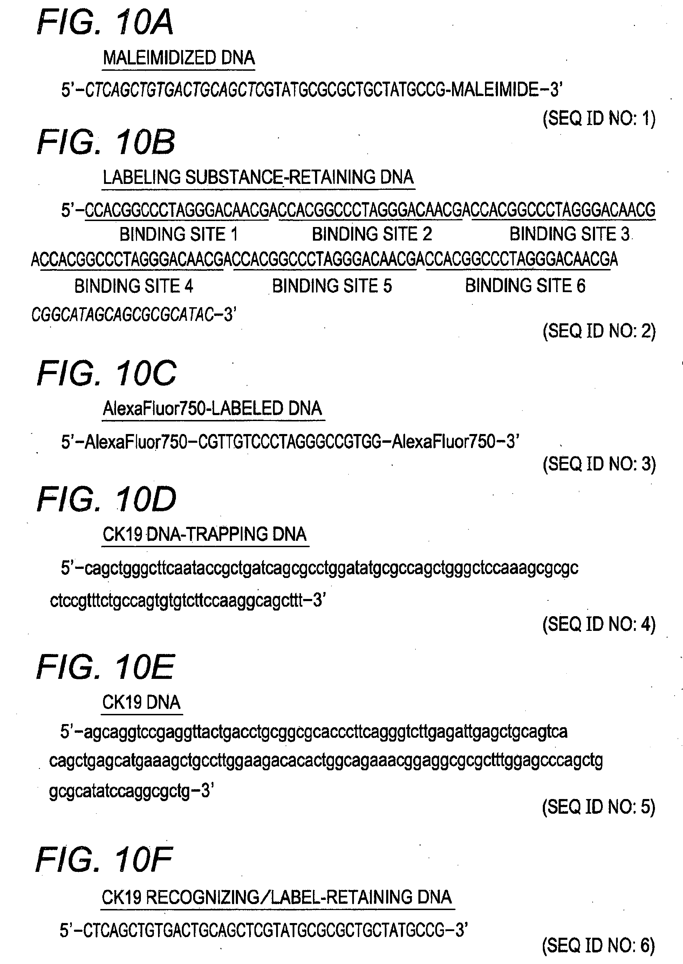

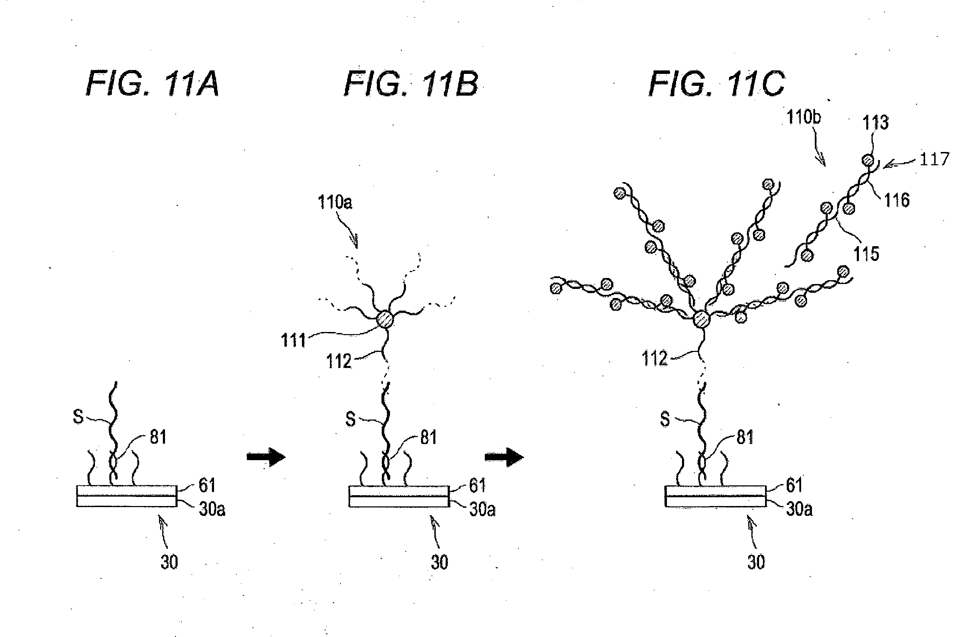

Then, the recombinant ferritin subunit was self-associated to obtain a spherical shell-shaped ferritin. The spherical shell-shaped ferritin thus obtained [see 111 in Fig. 11B] was bound to maleimidized DNA [manufactured by Japan Bio Services Co.,LTD., SEQ ID NO: 1] in 1 mM ethylenediaminetetraacetate-containing 20 mM phosphoric acid buffer at 50°C. The maleimidized DNA is DNA of 41 bases in which the 3' terminal is modified with maleimide. The maleimidized DNA has a sequence [the italicized sequence in Fig. 10A] complementary to CK19 DNA (SEQ ID NO: 5) which is the analyte S being as a target and a sequence to which a labeling substance-retaining DNA to be described later is bound [the sequence indicated by boldface in Fig. 10A].

The obtained product was subjected to gel filtration chromatography to purify the DNA-binding ferritin. The DNA-binding ferritin (conjugate) [see 110a in Fig. 11B] was assumed to have a structure in which about 12 DNAs were bound to the spherical shell-shaped ferritin based on SDS-PAGE. In Fig. 11, in order to make the description easy, as for the DNA-binding ferritin 110a (conjugate), a part of DNA bound to the spherical shell-shaped ferritin is omitted.

### (1-2) Production of label binding substance

100 nM of a labeling substance-retaining DNA [manufactured by Hokkaido System Science Co., Ltd., SEQ ID NO: 2, see 115 in Fig. 10B and Fig. 11C] and 1 µM of AlexaFluor750-labeled DNA [manufactured by Japan Bio Services Co.,LTD., SEQ ID NO: 3, see 117 in Fig. 11C] were incubated in 1 x MES hybridization solution (manufactured by Affymetrix) at 45°C for 1 hour to obtain a labeled form [see 110b in of Fig. 11C].

A labeling substance retaining DNA 115 has six sequences which hybridize with an AlexaFluor750-labeled DNA 117 [the underlined sequence in Fig. 10B]. Further, The labeling substance retaining DNA 115 has a sequence which hybridizes with maleimidized DNA in the DNA-binding ferritin [the italicized sequence in Fig. 10B] at the 3' terminal. The AlexaFluor750-labeled DNA 117 has a sequence which hybridizes with the labeling substance-retaining DNA 115 [see Fig. 10C]. Both ends of DNA (linker) [see 116 in Fig. 11C] included in the AlexaFluor750-labeled DNA 117 were labeled with AlexaFluor750 (labeling substance) [see 113 in Fig. 11C].

### (1-3) Production of working electrode substrate

A working electrode composed of a thin film (about 200 nm in thickness) of tin-doped indium oxide was formed on the surface of the substrate body 30a composed of silicon dioxide (SiO₂) by the spattering method. The thin film serves both as the conductive layer and the electron accepting layer. Subsequently, a working electrode lead for connecting to the ammeter was connected to the working electrode.

Then, the surface of the working electrode was brought into contact with the solution A obtained in Preparation example 1-1 to provide an amino group on the surface of the working electrode.

A DNA solution containing 10 µM of CK19 DNA-trapping DNA (the trapping substance 81) [SEQ ID NO: 4, see Fig. 10D] and a crosslinking reagent [trade name: microarray crosslinking reagent D, manufactured by GE Healthcare] were mixed at a volume ratio of 1:9. The obtained mixture was dropped onto the working electrode. Thereafter, the working electrode was irradiated with ultraviolet rays (160 mJ) to immobilize CK19 DNA-trapping DNA on the working electrode. Thus, a working electrode substrate was obtained.

### (1-4) Trapping of analyte

Silicone rubber (0.1 mm in thickness) was placed around the working electrode of the working electrode substrate so that a partition was formed. Thereafter, a hybridization buffer [trade name: Perfect Hyb, manufactured by TOYOBO CO., LTD] and 1 nM CK19 DNA [150 bases, SEQ ID NO:5, Manufactured by Hokkaido System Science Co., Ltd.] as the analyte S were placed in the space surrounded by the working electrode substrate and the silicone rubber. The working electrode substrate was incubated at 58°C for 2 hours. Thus, the analyte S was trapped by the trapping substance [see 81 in Fig. 11A] on the working electrode body [see 61 in Fig. 11A] [see Fig. 11A].

The working electrode 60 was washed with 6 x SSPE containing 0.1% by mass of polyoxyethylene sorbitan mono laurate (Tween-20) [composition of 1 x SSPE: 0.01 M phosphate buffer (pH 7.4), 0.149 M sodium chloride, 0.001 M EDTA]. Thereafter, 1 x MES hybridization solution (manufactured by Affymetrix) and 50 nM DNA binding ferritin (conjugate) were placed in the above space. The working electrode substrate (the upper substrate 30) was incubated at 45°C for 1 hour. Thus, the analyte S and the DNA-binding ferritin 110a (conjugate) were trapped on the working electrode body 61 [see Fig. 11B] by hybridizing the analyte S trapped by the trapping substance 81 on the working electrode body 61 with DNA (binding substance) included in the DNA-binding ferritin 110a (conjugate) [see 112 in Fig. 11B]. Here, the analyte S is hybridized with a dashed line portion [the italicized sequence in Fig. 10A] of a DNA 112 (binding substance) included in the DNA-binding ferritin 110a (conjugate).

Then, the working electrode 60 was washed with 6 x SSPE containing 0.1% by mass of Tween-20. Thereafter, 1 x MES hybridization solution (manufactured by Affymetrix) and a labeled form 110b were placed in the above space. The working electrode substrate (the upper substrate 30) was incubated at 45°C for 1 hour. Thus, a complex containing the trapping substance 81, the analyte S, the DNA binding ferritin 110a (conjugate), and the labeled form 110b was formed on the working electrode body 61 of the working electrode substrate (the upper substrate 30) [see Fig. 11C] (Example 1-1 (reference)).

### (2) Control experiment

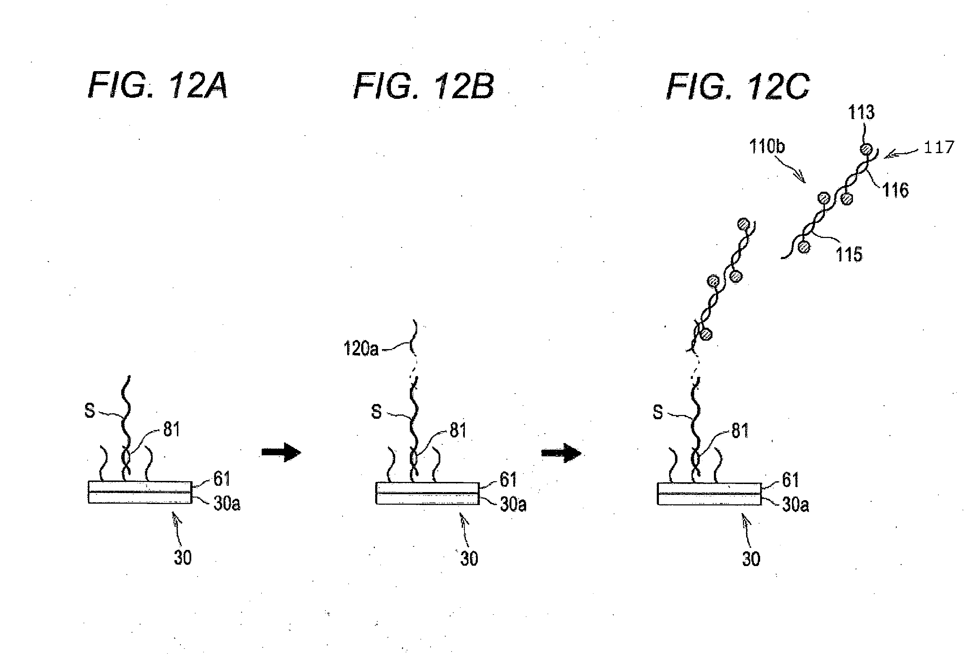

The same operation as Example 1-1 was performed except that 1 µM CK19 recognizing/label-retaining DNA [SEQ ID NO: 6, see Fig. 10F and 120a in Fig. 12] was used in place of the DNA binding ferritin in Example 1-1. Thus, a complex containing the trapping substance 81, the analyte S, a CK19 recognizing/label-retaining DNA 120a, and the labeled form 110b was formed on the working electrode body 61 of the working electrode substrate (the upper substrate 30) [see Fig. 12C] (Comparative example 1-1(reference)).

### (3) Measurement of photocurrent

Silicone rubber was placed around the working electrode substrates of Example 1-1 and Comparative example 1-1 so that a 0.2-mm-thick side wall was formed. Then, the space surrounded by the working electrode substrate (the upper substrate 30) and the silicone rubber was filled with the electrolytic solution obtained in Preparation example 1-2. The space filled with the electrolytic solution was sealed with the counter electrode substrate obtained in Preparation example 1-3 from the upper side of the working electrode substrate. Thus, the working electrode and the counter electrode are brought into contact with the electrolytic solution. Then, the detection chip including the working electrode substrate and the counter electrode was placed in an electrochemical measurement device. The working electrode lead and the counter electrode lead were connected to the ammeter.

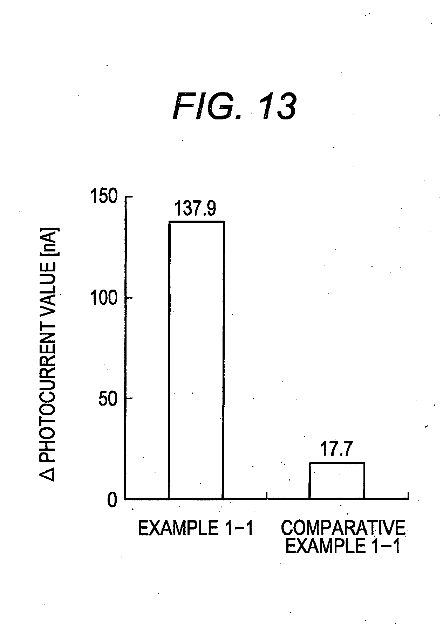

The light source (wavelength: 781 nm, laser light source with an output power of 13 mW) emits light from the working electrode substrate side toward the counter electrode substrate. The labeling substance is excited by photoirradiation, thereby generating electrons. When the generated electrons are transported to the working electrode, current flows between the working electrode and the counter electrode. Then, the electric current was measured. Fig. 13 shows examined results of a relationship between the kind of the detection method and photocurrent in Test example 1-1.

From the results shown in Fig. 13, it is found that the current detected in Example 1-1 in which ferritin (a support composed of polypeptide) is used as the support retaining the labeling substance is about 137 nA. On the other hand, it is found that the current detected in Comparative example 1-1 in which the support composed of polypeptide is not used as the support retaining the labeling substance like a conventional manner is about 17 nA. From these results, it is found that a very large current can be detected when the support composed of polypeptide is used as the support retaining the labeling substance.

### (Test example 1-2 (reference))

### (1-1) Production of DNA binding BSA

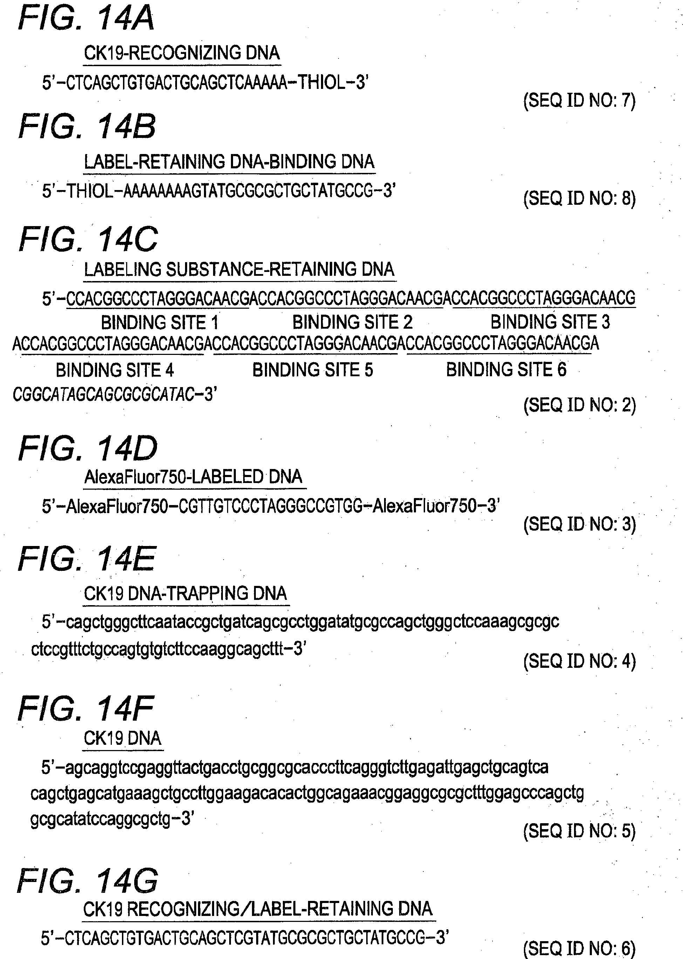

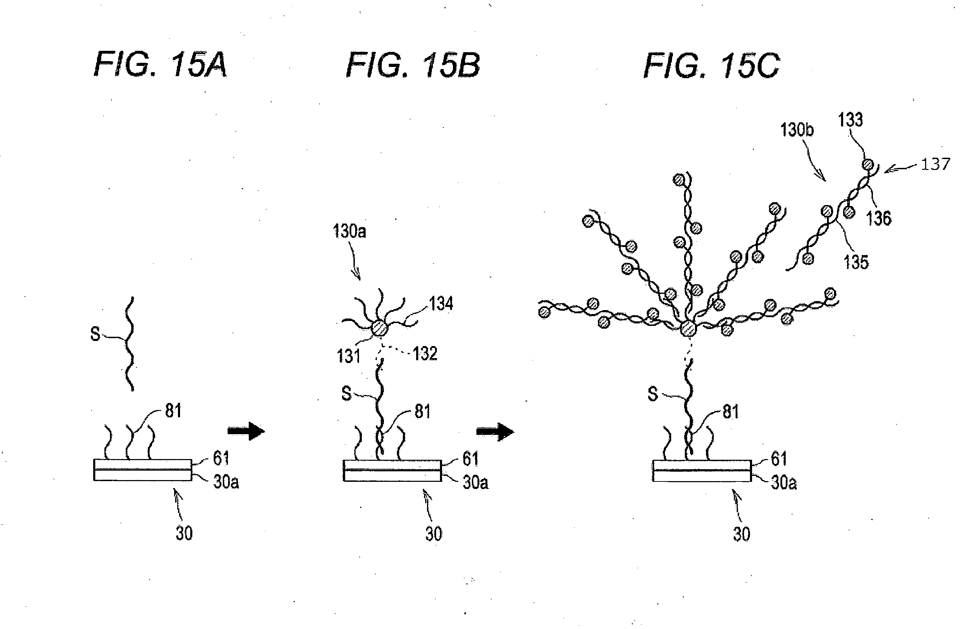

Bovine serum albumin (BSA) (manufactured by Sigma) was purified by gel filtration chromatography. The purified BSA [131 in Fig. 15B] was reacted with a cross-linker [trade name: GMBS, manufactured by Dojin Chemical Laboratory] and a maleimide group was bound to the surface of BSA. 2.6 nmol of the obtained maleimide-modified BSA was mixed with 2.7 nmol of CK19-recognizing DNA having a thiol group at the 3'terminal [SEQ ID NO: 7, 20 bases, see 132 in Fig. 14A and Fig. 15B] and 6.4 nmol of label-retaining DNA-binding DNA having a thiol group at the 3'terminal [SEQ ID NO: 8, see 134 in Fig. 14B and Fig. 15B]. The mixture was reacted in 100 µL of 0.15 M sodium chloride containing phosphoric acid buffer (pH 7) at 37°C for 2 hours. The obtained reaction product was ultrafiltered through a centrifugal filter [trade name: Amicon Ultra 0.5, 30 K cut-off, manufactured by Millipore] to remove unreacted DNA, and DNA binding BSA [see 130a in Fig. 15B] was obtained.

The CK19-recognizing DNA has a sequence [the sequence indicated by boldface in Fig. 14A] complementary to CK19 DNA (the analyte S) [SEQ ID NO: 5, Fig. 14F]. The label-retaining DNA-binding DNA has a sequence [the sequence indicated by boldface in Fig. 14B] complementary to a bonding site [the italicized sequence in Fig. 14C] of labeling substance-retaining DNA [SEQ ID NO: 2, see Fig. 14C].

### (1-2) Production of labeled form

100 nM of labeling substance-retaining DNA (the second binding substance) [manufactured by Hokkaido System Science Co.,Ltd., SEQ ID NO: 2, see 135 in Fig. 14C and Fig. 15C] and 1 µM of AlexaFluor750-labeled DNA [manufactured by Japan Bio Services Co.,LTD., SEQ ID NO: 3, see 137 in Fig. 14D and Fig. 15C] were incubated in a hybridization solution [trade name: PerfectHyb, manufactured by TOYOBO CO., LTD] at 60 °C for 1 hour to obtain a labeled form [see 130b in Fig. 15C]. The labeled form 130b is composed of an AlexaFluor750-labeled DNA 137 composed of a labeling substance 133 and a DNA 136 and a labeling substance-retaining DNA 135.

### (1-3) Trapping of analyte

Silicone rubber (0.1 mm in thickness) was placed around the working electrode 61 of the working electrode substrate (the upper substrate 30) obtained in a similar manner to (1-3) of Test example 1-1 so that a partition was formed. CK19 DNA-trapping DNA (trapping substance 81) [see Fig. 14E, SEQ ID NO: 4] was immobilized on the working electrode body 61 of the working electrode substrate (the upper substrate 30).

Thereafter, a hybridization buffer [trade name: Perfect Hyb, manufactured by TOYOBO CO., LTD] and 1 nM of CK19 DNA [150 bases, SEQ ID NO: 5, Manufactured by Hokkaido System Science Co., Ltd., see Fig. 14F] as the analyte S were placed in the space surrounded by the working electrode substrate (the upper substrate 30) and the silicone rubber. The working electrode substrate (the upper substrate 30) was incubated at 60°C for 2 hours. Thus, the analyte S was trapped by the trapping substance 81 on the working electrode body 61 [see Fig. 15A].

After discharge of the reaction solution, a hybridization buffer [trade name: Perfect Hyb, manufactured by TOYOBO CO., LTD.] and 50 nM DNA binding BSA (conjugate) [see 130a in Fig. 15B] were placed in the above space. The working electrode substrate (the upper substrate 30) was incubated at 60°C for 1 hour. Thus, the analyte S and the DNA binding BSA 130a (conjugate) were trapped on the working electrode body 61 [see Fig. 15B] by hybridizing the analyte S trapped by the trapping substance 81 on the working electrode body 61 with a CK19-recognizing DNA 132 (the first binding substance) included in a DNA binding BSA 130a (conjugate).

After discharge of the reaction solution, a hybridization buffer [trade name: Perfect Hyb, manufactured by TOYOBO CO., LTD.] and the labeled form 130b were placed in the above space. The labeling substance-retaining DNA 135 in the labeled form 130b (the second binding substance) was hybridized with the label-retaining DNA-binding DNA (the first linker) [see 134 in Fig. 15B] included in the DNA binding BSA 130a (conjugate) immobilized on the working electrode body 61 via the trapping substance 81 and the analyte S by incubating the working electrode substrate (the upper substrate 30) at 60°C for 1 hour. Thus, a complex containing the trapping substance 81, the analyte S, the DNA binding BSA 130a (conjugate), and the labeled form 130b was formed on the working electrode body 61 of the working electrode substrate (the upper substrate 30) [see Fig. 15C] (Example 1-2 (reference)). A label-retaining DNA-binding DNA 134 (the first linker) is bound to the 3' terminal region [the italicized sequence in Fig. 14C] of the labeling substance-retaining DNA 135 (the second binding substance).

### (2) Control experiment

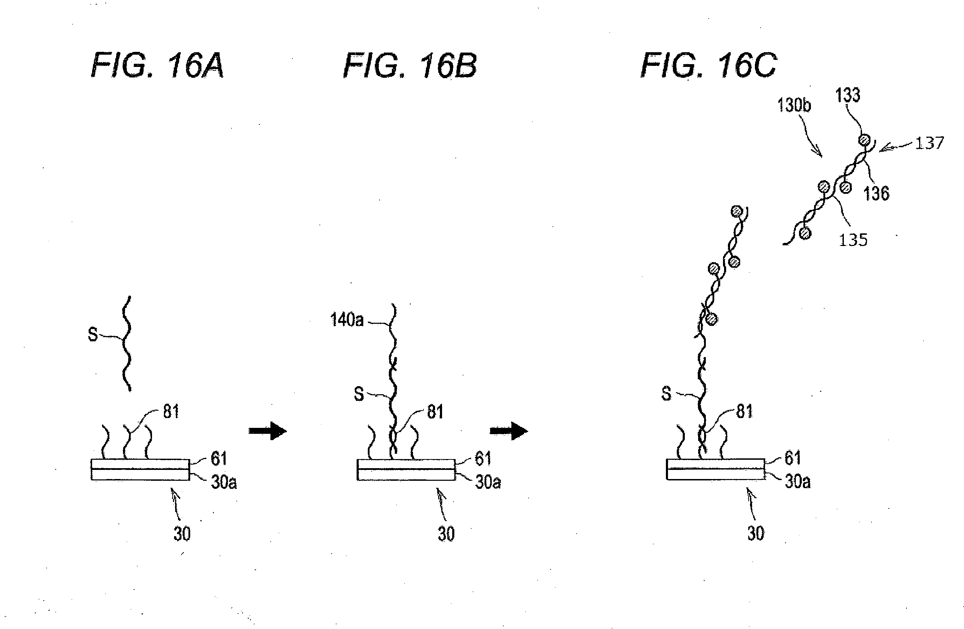

The same operation as Example 1-1 was performed except that 1 µM of CK19 recognizing/label-retaining DNA [SEQ ID NO: 6, see Fig. 14G and 140a in Fig. 16] was used in place of the DNA binding BSA in Example 1-2. Then, a complex containing the trapping substance 81, the analyte S, a CK19 recognizing/label-retaining DNA 140a, and the labeled form 130b was formed on the working electrode body 61 of the working electrode substrate (the upper substrate 30) [see Fig. 16C] (Comparative example 1-2(reference)).

### (3) Measurement of photocurrent

Silicone rubber was placed around the working electrode substrates of Example 1-2 and Comparative example 1-2 so that a 0.2-mm-thick side wall was formed. Then, the space surrounded by the working electrode substrate (the upper substrate 30) and the silicone rubber was filled with the electrolytic solution obtained in Preparation example 1-2. The space filled with the electrolytic solution was sealed with the counter electrode substrate obtained in Preparation example 1-3 from the upper side of the working electrode substrate. Thus, the working electrode and the counter electrode are brought into contact with the electrolytic solution. Then, the detection chip including the working electrode substrate and the counter electrode was placed in an electrochemical measurement device. The working electrode lead and the counter electrode lead were connected to the ammeter.

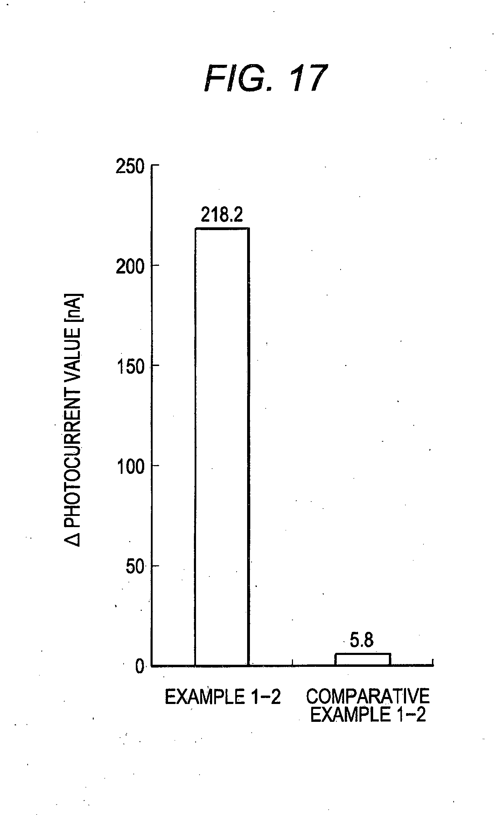

The light source (wavelength: 781 nm, laser light source with an output power of 13 mW) emits light from the working electrode substrate side toward the counter electrode substrate. The labeling substance is excited by photoirradiation, thereby generating electrons. When the generated electrons are transported to the working electrode, current flows between the working electrode and the counter electrode. Then, the electric current was measured. Fig. 17 shows examined results of a relationship between the kind of the detection method and photocurrent in Test example 1-2.

From the results shown in Fig. 17, it is found that the current detected in Example 1-2 in which BSA (a support composed of polypeptide) is used as the support retaining the labeling substance is about 218.2 nA. On the other hand, it is found that the current detected in Comparative example 1-2 in which the support composed of polypeptide is not used as the support retaining the labeling substance like a conventional manner is about 5.8 nA. From these results, it is found that a very large current can be detected when the support composed of polypeptide is used as the support retaining the labeling substance.

### (Test example 1-3 (reference))

### (1-1) Production of DNA binding BSA

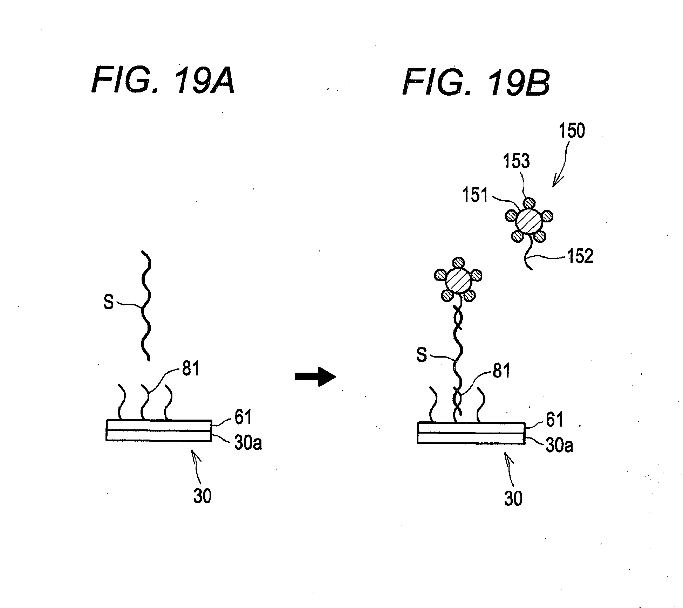

BSA (manufactured by Sigma) was purified by gel filtration chromatography. The purified BSA [see 151 in Fig. 19B] and AlexaFluor750 derivative (trade name: AlexaFluor750 carboxylic acid, succinimidyl ester, manufactured by Invitrogen) were reacted in 0.1 M sodium carbonate (pH 8.5) at room temperature for 1 hour. The obtained reaction product was filtered through a desalting column [trade name: Zeba Spin Micro desalting Column, manufactured by Pierce] to remove the unreacted AlexaFluor750 derivative, and AlexaFluor750-labeled BSA was obtained. The AlexaFluor750-labeled BSA was assumed to have a structure in which about ten AlexaFluor750 derivatives were bound to BSA based on the band shift and absorption spectrum by SDS-PAGE. The complex was reacted with a cross-linker [trade name: GMBS, manufactured by Dojin Chemical Laboratory], and a maleimide group was bound to the surface of the AlexaFluor750-labeled BSA.

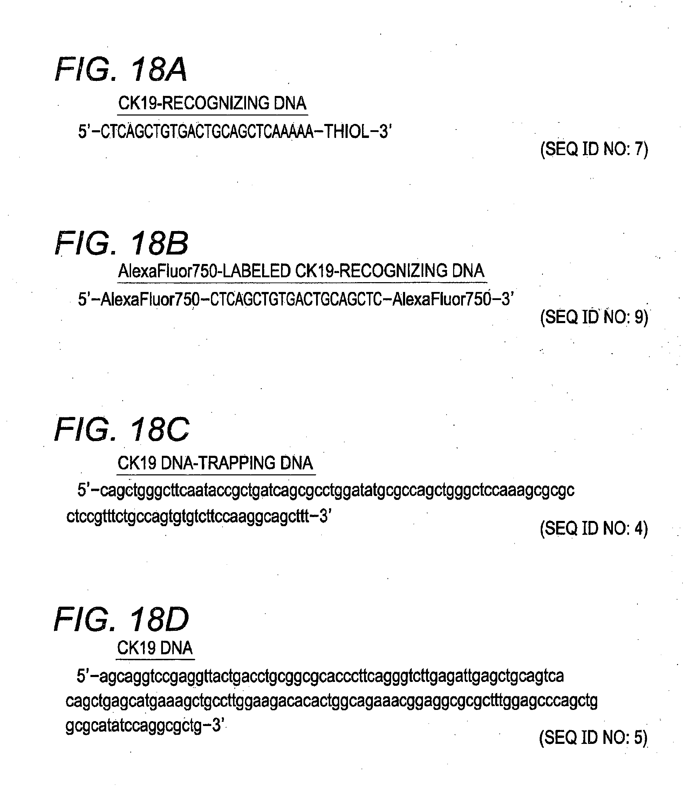

The obtained reaction product was purified through a desalting column to remove an unreacted cross-linker. Thereafter, 1 nmol of the maleimide-modified AlexaFluor750-labeled BSA thus obtained was mixed with 2 nmol of CK19-recognizing DNA having a thiol group at the 3' terminal [SEQ ID NO: 7, 20 bases, see Fig. 18A]. The mixture was reacted in 50 µL of 0.15 M sodium chloride containing phosphoric acid buffer (pH 7) at 37°C for 3 hours. The obtained reaction product was ultrafiltered through a centrifugal filter [trade name: Amicon Ultra 0.5, 30 K cut-off, manufactured by Millipore] to remove unreacted DNA, and DNA binding AlexaFluor750-labeled BSA was obtained.

### (1-2) Trapping of analyte

Silicone rubber (0.1 mm in thickness) was placed around the working electrode 60 of the working electrode substrate (the upper substrate 30) obtained in a similar manner to (1-3) of Test example 1-1 so that a partition was formed. CK19 DNA-trapping DNA (trapping substance 81) [see Fig. 18C, SEQ ID NO: 4] was immobilized on the working electrode body 61 of the working electrode substrate (the upper substrate 30).

Thereafter, a hybridization buffer [trade name: Perfect Hyb, manufactured by TOYOBO CO., LTD] and 1 nM CK19 DNA [150 bases, SEQ ID NO: 5, manufactured by Hokkaido System Science Co., Ltd., see Fig. 18D] as the analyte S were placed in the space surrounded by the working electrode substrate (the upper substrate 30) and the silicone rubber. The working electrode substrate (the upper substrate 30) was incubated at 60°C for 2 hours. Thus, the analyte S was trapped by the trapping substance 81 on the working electrode body 61 [see of Fig. 19A].

After discharge of the reaction solution, a hybridization buffer [trade name: Perfect Hyb, manufactured by TOYOBO CO., LTD.] and 100 nM of DNA binding AlexaFluor750-labeled BSA (label binding substance) [see 150 in Fig. 19B] were placed in the above space. The working electrode substrate (the upper substrate 30) was incubated at 60°C for 1 hour. Thus, the analyte S trapped by the trapping substance 81 on the working electrode body 61 was hybridized with DNA 152 (binding substance) included in a DNA binding AlexaFluor750-labeled BSA 150 [see of Fig. 19B]. Thereafter, the working electrode 60 was washed with 6 x SSPE containing 0.1% by mass of Tween-20 (Example 1-3 (reference)).

### (2) Control experiment

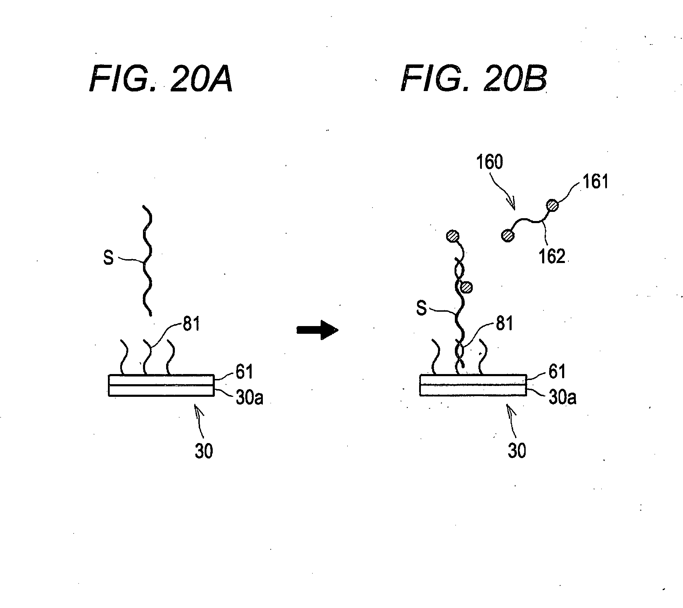

The same operation as Example 1-1 was performed except that 100 nM DNA recognizing Alexa Fluor750-labeled CK19 [manufactured by Japan Bio Services Co., LTD., SEQ ID NO: 9, see 160 in Fig. 18B and Fig. 20B] was used in place of the DNA binding AlexaFluor750-labeled BSA 150 in Example 1-3. A complex containing the trapping substance 81, the analyte S, and the Alexa Fluor750-labeled CK19-recognizing DNA 160 was formed on the working electrode 61 of the working electrode substrate (the upper substrate 30) [see Fig. 20B] (Comparative example 1-3 (reference)). The Alexa Fluor750-labeled CK19-recognizing DNA 160 has AlexaFluor750 [see 161 Fig. 20B] at both ends of CK19 recognizing DNA [see 162 in Fig. 20B].

### (3) Measurement of photocurrent

Silicone rubber was placed around the working electrode substrates of Example 1-3 and Comparative example 1-3 so that a 0.2-mm-thick side wall was formed. Then, the space surrounded by the working electrode substrate (the upper substrate 30) and the silicone rubber was filled with the electrolytic solution obtained in Preparation example 1-2. The space filled with the electrolytic solution was sealed with the counter electrode substrate obtained in Preparation example 1-3 from the upper side of the working electrode substrate. Thus, the working electrode and the counter electrode are brought into contact with the electrolytic solution. Then, the detection chip including the working electrode substrate and the counter electrode was placed in an electrochemical measurement device. The working electrode lead and the counter electrode lead were connected to the ammeter.

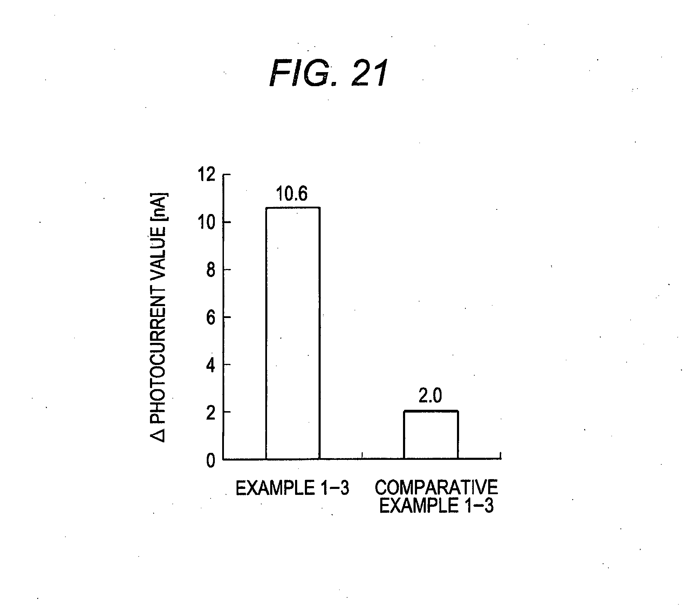

The light source (wavelength: 781 nm, laser light source with an output power of 13 mW) emits light from the working electrode substrate side toward the counter electrode substrate. The labeling substance is excited by photoirradiation, thereby generating electrons. When the generated electrons are transported to the working electrode, current flows between the working electrode and the counter electrode. Then, the electric current was measured. Fig. 21 shows examined results of a relationship between the kind of the detection method and photocurrent in Test example 1-3.

From the results shown in Fig. 21, it is found that the current detected in Example 1-3 in which BSA (a support composed of polypeptide) is used as the support retaining the labeling substance is about 10 nA. On the other hand, it is found that the current detected in Comparative example 1-3 in which the support composed of polypeptide is not used as the support retaining the labeling substance like a conventional manner is about 2.0 nA. From these results, it is found that according to the method for electrochemically detecting an analyte using the support composed of polypeptide as the support retaining the labeling substance, it is possible to detect a current about five times as large as the current detected by the method for electrochemically detecting an analyte not using the support composed of polypeptide.

As described above, in the DNA binding AlexaFluor750-labeled BSA 150, the number of AlexaFluor750 added to BSA is about 10. On the other hand, in the Alexa Fluor750-labeled CK19-recognizing DNA, the number of AlexaFluor750 added to DNA is 2. Therefore, the current value to be detected is proportional to the number of labeling. Thus, according to the method for electrochemically detecting an analyte using the support composed of polypeptide, it is suggested that the analyte can be quantified.

### - Second embodiment -

### [Method for electrochemically detecting analyte]

The method for electrochemically detecting an analyte according to the present invention is as defined in the claims.

A major characteristic of the method for electrochemically detecting an analyte according to the present invention is that a complex containing an analyte and a label binding substance in which a labeling substance is attached to a binding substance via a modulator is formed on the working electrode.

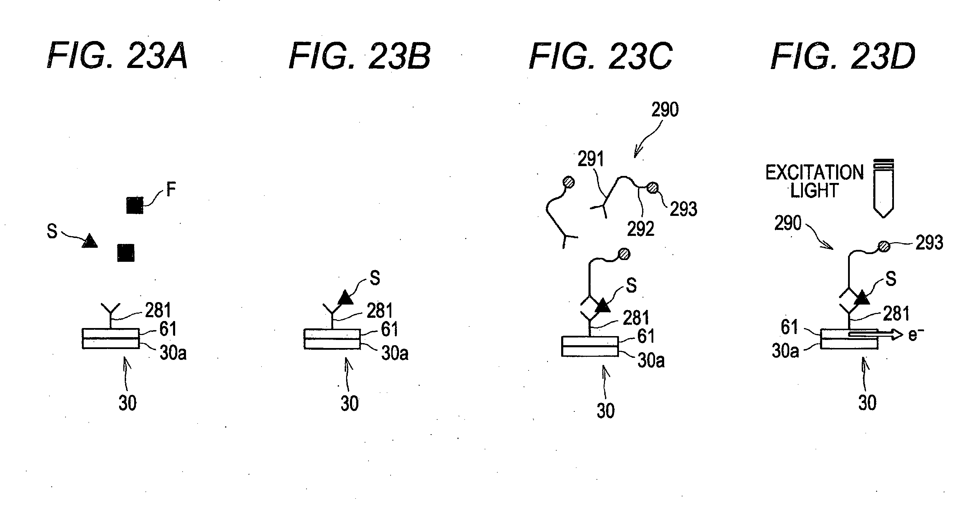

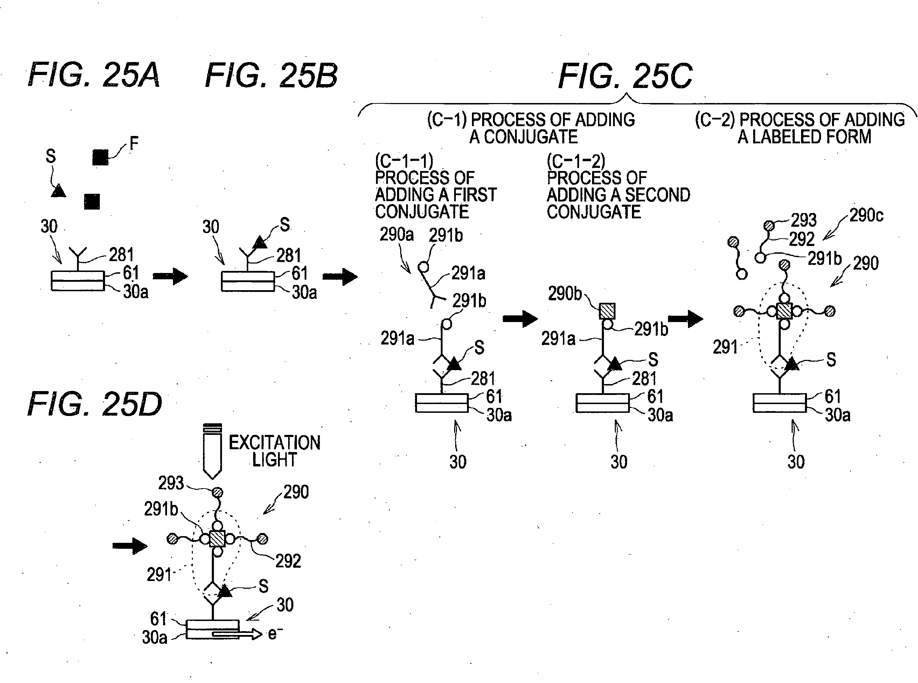

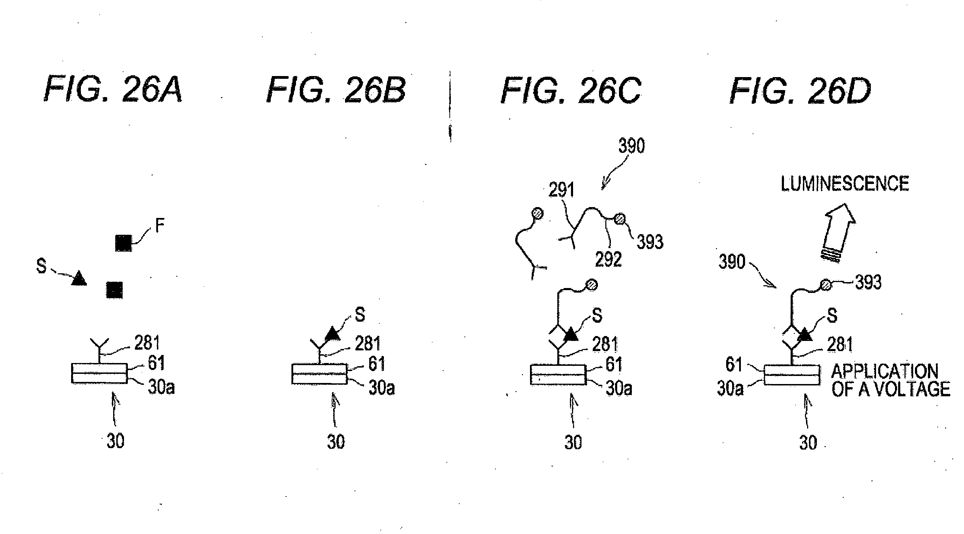

In the method according to the present invention, a photochemically or electrochemically active substance is used as the labeling substance. The photochemically active substance is detected using electrons released by excitation of the substance by light. On the other hand, the electrochemically active substance is detected using an oxidation reduction current and/or electrochemical luminescence based on the substance. Therefore, the method for the present invention can be divided broadly into the photoelectrochemical detection method (see Figs. 23 and 25) and the oxidation reduction current/electrochemiluminescence detection method (see Fig. 26) depending on the type of detection technique of the labeling substance.

### 1. Photoelectrochemical detection method

First, the photoelectrochemical detection method will be explained. In the photoelectrochemical detection method, the detector illustrated in Fig. 1 and the detection chip illustrated in Fig. 3 can be used, however, they are not limited thereto. Hereinafter, the method will be explained taking an example of the case of using the detector illustrated in Fig. 1 and the detection chip illustrated in Fig. 3.

Referring to Fig. 23, in the photoelectrochemical detection method, a user injects a sample containing the analyte S through the sample inlet 30b of the detection chip 20 [see the process of supplying a sample of Fig. 23A]. Thus, the analyte in the sample is trapped by the trapping substance 281 on the working electrode body 61 of the upper substrate 30 constituting the detection chip 20 [see the process of trapping an analyte of Fig. 6B]. In this case, substances (contaminants F) other than the analyte S in the sample are not trapped by the trapping substance 281.

As the trapping substance 281, the same substance as that of the trapping substance 81 described in the first embodiment can be used.

The process of trapping an analyte by the trapping substance 281 can be performed under the same conditions as those in the process of trapping an analyte by the trapping substance 81 described in the first embodiment.

Then, the user injects the label binding substance 290 into the detection chip 20 from the sample inlet 30b to allow the label binding substance 290 to be bound to the analyte S trapped by the trapping substance 281 on the working electrode body 61 [see the labeling process of Fig. 6C]. In the labeling process, a complex containing the trapping substance 281, the analyte S, and the label binding substance 290 is formed on the working electrode body 61.

The label binding substance 290 is formed of a binding substance 291 to be bound to the analyte S, a modulator 292, and a labeling substance 293. In the label binding substance 290, the labeling substance 293 is fixed to the binding substance 291 via the modulator 292.

The binding substance 291 may be a substance which binds to a position or site in the analyte S, which is different from that of the trapping substance 281. The binding substance 291 is suitably selected depending on the type of the analyte S. For example, when the analyte S is a nucleic acid, a nucleic acid probe hybridizing to the nucleic acid, an antibody to the nucleic acid, a protein binding to the nucleic acid or the like can be used as the binding substance 291. When the analyte S is a protein or peptide, an antibody to the protein or peptide can be used as the binding substance 291.

The modulator 292 lies between the binding substance 291 and the labeling substance 293. The modulator 292 is a substance which interacts with the electrolytic solution and the working electrode body 61.

The modulator 292 is preferably selected from modulators having hydrophilicity and modulators having hydrophobicity depending on the electrolytic solution to be used in the detection process to be described later and the polarity of the surface of the working electrode.

In the method for electrochemically detecting an analyte according to the present invention, the electrolytic solution contains an aprotic solvent and the surface of the working electrode and the modulator exhibit hydrophilicity, or the electrolytic solution contains a protic solvent and the surface of the working electrode and the modulator exhibit hydrophobicity.

Examples of the modulator having hydrophilicity include nucleic acids such as DNA and RNA; polyethylene glycol (a hydrophilic high molecular compound); and hydrophilic polypeptides mainly comprised of asparagine (a hydrophilic amino acid), serine, aspartic acid, glutamine, glutamic acid, threonine, arginine, histidine, ricin, tyrosine, and cysteine. Examples of the aprotic solvent include nitrils such as acetonitrile (CH₃CN); carbonates such as propylene carbonate and ethylene carbonate; heterocyclic compounds such as 1,3-dimethylimidazolinone, 3-methyloxazolinone, and dialkyl imidazolium salt; dimethylformamide, dimethyl sulfoxide, and sulfolane. Among the aprotic solvents, acetonitrile is preferred. As an example of the working electrode having hydrophilicity, an electrode having a hydrophilic functional group on the surface is listed. Examples of the hydrophilic functional group include a functional group containing a hydroxyl group (for example, a silanol group), an amino group, and a thiol group. The working electrode having hydrophilicity can be obtained by, for example, treating the working electrode body with silane coupling agents such as aminopropyltriethoxysilane which provides an amino group and mercaptopropyltriethoxysilane which provides a thiol group. In the present invention, the electrode having a hydrophilic functional group on the surface may be subjected to hydrophilic treatment by binding polyethylene glycol, nucleic acid or the like to the electrode.

On the other hand, examples of the modulator having hydrophobicity include hydrophobic peptides mainly comprised of glycine (hydrophobic amino acid), tryptophan, methionine, proline, phenylalanine, alanine, valine, leucine, and isoleucine; and polymeric compounds mainly comprised of hydrocarbon having hydrophobicity (e.g. styrene oligomer and acrylic oligomer). As the protic solvent, for example, water; a polar solvent containing a water-based buffer component or the like is listed. As the working electrode having hydrophobicity, for example, an electrode having a hydrophobic functional group on the surface is listed. The working electrode having hydrophobicity can be obtained by, for example, treating the surface of the working electrode body formed of a metal oxide in which a silanol group is introduced with compounds to which a methyl group or a phenyl group may be introduced, such as methyltrimetoxysilane and phenyltriethoxysilane.

The length of the modulator 292 can be suitably selected within a range that does not inhibit the formation reaction of a complex on the working electrode, for example, the ligation reaction of the binding substance to the trapped analyte. It is preferable that the length of the modulator 292 is usually 100 nm or less. The lower limit of the length of the modulator 292 varies depending on the type of the modulator 292. Thus, it is desirable to suitably set the lower limit according to the type of the modulator 292.

As the labeling substance 293, the same substance as the labeling substance 93 described in the first embodiment can be used.

Examples of the bond form of the modulator 292 and the binding substance 291 include covalent and non-covalent bonds.

The method for binding the modulator 292 to the binding substance 291 by a covalent bond is not particularly limited.

In the binding substance 291, a site to which the modulator 292 is covalently bound is not particularly limited. From the viewpoint that the operation of binding the binding substance 291 to the modulator 292 is simple, amino and sulfhydryl groups are preferred.

Examples of a reaction group capable of binding to an amino group include a succinimido group (NHS), an isothiocyano group (ITC), a chlorosulfonyl group, a chloroacyl group, an oxyethylene group, a chloroalkyl group, an aldehyde group, and a carboxyl group. Among them, NHS and ITC are preferred because when the modulator 292 as a target is covalently bound via an amino group, a reaction in an aqueous system is essential, and the conditions capable of using a reaction compound are limited such that the pH of the reaction solution is in a neutral to weak alkaline region and the reaction is progressed at a reaction temperature of ice-cooling to about 37°C for a short time. Therefore, a modulator having NHS and/or ITC can be used as the modulator 292.

Examples of the reaction group capable of binding to a sulfhydryl group include a maleimide group and a bromoacetamide group. The sulfhydryl group normally forms a disulfide (S-S) bond in a polypeptide. Thus, when the sulfhydryl group is used as a site for binding to the modulator 292, the disulfide structure in the polypeptide is reduced to be used as a sulfhydryl group. In the reduction of the disulfide bond, dithiothreitol (DTT), β-mercaptoethanol (β-ME), and mercaptoethylamine (MEA) can be used. Therefore, the modulator 292 has a functional group having high reactivity with an amino group and a sulfhydryl group (e.g. a succinimido group and a maleimide group), the modulator 292 can be directly bound to amino and sulfhydryl groups of the binding substance 291 by mixing the binding substance 291 and the modulator 292. As the modulator 292, for example, succinimide ester-modified DNA is listed.

When the modulator 292 has an amino group, a sulfhydryl group, an aldehyde group, a carboxyl group or the like, the binding substance 291 can be easily bound to the modulator 292 by binding the binding substance 291 to the modulator 292, for example, via a chemical cross-linker; forming a dithiol bond between the binding substance 291 and the modulator 292 when the binding substance 291 has a sulfhydryl group; and performing a general chemical reaction.

The cross-linker generally has a linear structure and is formed of a spacer having a succinimido group which reacts with amino and thiol groups as well as a maleimide group at the both ends. The use of the cross-linker allows the binding substance 291 to be bound to the modulator 292.

For example, when the modulator 292 has a thiol group, a cross-linker having a succinimido group at one end and having a maleimide group at the other end can be used in binding the modulator 292 to an amino group of the binding substance 291. In this case, the amino group in the binding substance 291 is first reacted with the succinimido group in the cross-linker to introduce the maleimide group of the cross-linker into the binding substance 291. The binding can be performed by reacting the maleimide group with the thiol group in the modulator 292. Here, the length of the spacer of the cross-linker is not particularly limited. Specific examples of the cross-linker to be used include the same cross-linkers described in the first embodiment. The cross-linker may be glutaraldehyde in which functional groups at both ends have reactivity with an amino group, a cross-linker which has two functional groups (an amine-reactive NHS ester group and a light-reactive diazirine group) at the end or the like.

When the binding substance 291 and the modulator 292 have thiol groups, the binding is possible by reacting the thiol group of the binding substance 291 with the thiol group of the modulator 292 to form a dithiol bond. When the modulator 292 has a carboxyl group and the binding substance 291 has an amino group, the carboxyl group can be bound to the amino group of the binding substance 291 by activating with NHS. When the modulator 292 has an aldehyde group and the binding substance 291 has an amino group, a stable bond can be formed by forming a Schiff base between the aldehyde group of the modulator 292 and the amino group of the binding substance 291 and reducing it.

The method for binding the modulator 292 to the binding substance 291 by a non-covalent bond is not particularly limited.

As the method for binding the modulator 292 to the binding substance 291 by a non-covalent bond, a method for directly binding the binding substance 292 to the modulator 291 by a non-covalent bond and a method for binding the modulator 292 to the binding substance 291 via a substance bound by a covalent bond by a non-covalent bond are contemplated.

Examples of the method for binding the modulator 292 to the binding substance 291 by a non-covalent bond include a method for using bonding of streptoavidin to biotin and the like. Examples of the method for binding the modulator 292 to the binding substance 291 via a substance bound by a covalent bond by a non-covalent bond include a method comprising covalent-bonding DNA having an amino group at the end to the binding substance 291 and non-covalently binding complementary DNA to which the modulator 292 is bound to the DNA by hybridization and the like.

The method for binding the labeling substance 293 to the modulator 292 is performed by the same method as the method for binding the modulator 292 to the binding substance 291.

Subsequently, the detection process is performed [see the detection process of Fig. 23D].

In the detection process, the user first injects an electrolytic solution through the sample inlet 30b of the detection chip 20. Thereafter, the user inserts the detection chip 20 into the chip insertion unit 11 of the detector 1 shown in Fig. 1. Then, the user gives an instruction to start measuring to the detector 1. Here, the electrode leads 71, 72, and 73 of the detection chip 20 inserted into the detector 1 are connected to the ammeter 14 and the power source 15. Then, an arbitrary potential based on the reference electrode 69 is applied to the working electrode body 61 by the power source 15 of the detector 1. As the potential to be applied to the electrode, a potential in which the current value (stationary current, dark current) is low when the analyte is not irradiated with excitation light and the photocurrent generated from the analyte becomes a maximum photocurrent is preferred. The potential may be applied to the counter electrode 66 or the working electrode body 61.

Thereafter, the light source 13 of the detector 1 emits excitation light to the labeling substance 293 on the working electrode 60. Thus, the labeling substance 293 is excited to generate electrons. The generated electrons move to the working electrode 60. As a result, current flows between the working electrode 60 and the counter electrode 66. Then, the current flowing between the working electrode 60 and the counter electrode 66 is measured by the ammeter 14 of the detector 1. The current value measured by the ammeter 14 correlates with the number of the labeling substance 293. Therefore, the analyte S can be quantified based on the measured current value. The excitation light may be only light in a specified wavelength region, which is obtained using a spectrometer or a bandpass filter, if necessary.

Thereafter, a current value digitally converted by the A/D converting unit 16 is input into the control unit 17. Then, the control unit 17 estimates the amount of the analyte in the sample from the digitally converted current value based on a calibration curve indicating a relationship between a current value created in advance and the amount of the analyte. The control unit 17 creates a detection result screen for displaying the information on the estimated amount of the analyte on the display 12. Thereafter, the detection result screen created by the control unit 17 is sent to the display 12 so as to be displayed on the display 12.

As the electrolytic solution, a solution containing an electrolyte composed of salts which may supply electrons to the labeling substance 293 in an oxidized state, an aprotic solvent or a protonic polar solvent can be used. The electrolytic solution may further contain other components, if desired. The electrolytic solution may be in gel or solid form.

As the electrolyte, the same electrolyte described in the first embodiment can be used.

The electrolyte concentration of the electrolytic solution is preferably from 0.001 to 15 M.

When the labeling substance 293 is irradiated with light, a light source which can emit light in a wavelength capable of photoexciting the labeling substance 293 can be used. The light source can be suitably selected depending on the type of the labeling substance 293. As the light source, the same light source described in the first embodiment can be used.

In the measurement of a photocurrent derived from the labeling substance 293, for example, a measurement device which includes an ammeter, a potentiostat, a recorder, and a computer can be used.

In the detection process, the amount of the analyte can be examined by quantifying the photocurrent.

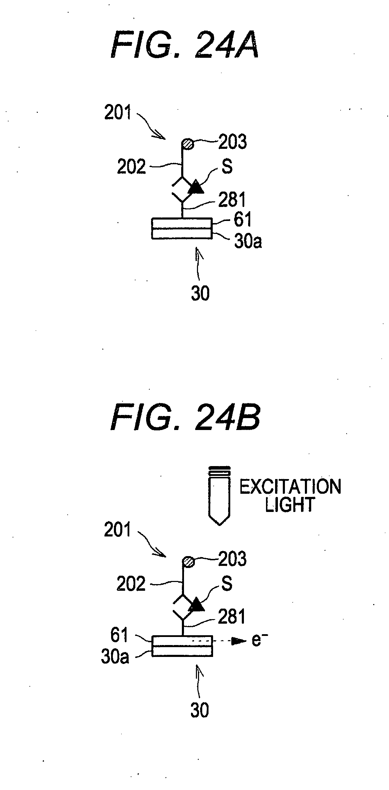

As described above, in the method for electrochemically detecting an analyte according to the present embodiment, when detecting the analyte S, the modulator 292 intervenes between the binding substance 291 and the labeling substance 293 [see Fig. 23D]. On the other hand, in the conventional method for electrochemically detecting an analyte, for example, as shown in Fig. 24A, a labeled antibody 201 obtained by directly labeling an antibody 202 as a binding substance with a labeling substance 203 is used in labeling of the analyte S. A complex containing the trapping substance 281 on the working electrode body 61, the analyte S, and the labeled antibody 201 is formed, and the analyte S is detected based on the current (photocurrent) generated from the labeling substance 203 in the complex formed on the electrode. Thus, in the conventional method shown in Fig. 24, the specific volume of the complex formed in detecting the analyte S becomes a large restriction in achieving high sensitivity. Thus, the photocurrent being detected tends to be small.

However, in the method for electrochemically detecting an analyte according to the present invention, the specific volume of the complex to be formed on the working electrode 60 is larger than that of the complex formed when labeling the analyte S by the conventional method shown in Fig. 24. Despite that, the analyte can be unexpectedly detected with high detection sensitivity as compared with the conventional method shown in Fig. 24.

In the method for electrochemically detecting an analyte according to the present embodiment, from the viewpoint of suppressing the generation of noises due to contaminants, the user may discharge a remaining liquid containing contaminants from the sample inlet 30b of the detection chip 20 after the process of trapping an analyte and wash an inside of the detection chip 20. In the washing of the inside of the detection chip 20, organic solvents such as a buffer (particularly a buffer containing a surfactant); purified water (particularly purified water containing a surfactant); and ethanol can be used.

In the method for electrochemically detecting an analyte according to the present embodiment, from the viewpoint of removing free label binding substance 290 which is not bound to the analyte S and improving the detection accuracy, the process of washing the inside of the detection chip 20 to remove the free label binding substance 290 may be further performed after the labeling process. For example, ethanol and purified water can be used for the washing.

In the present invention, the operation may be performed so as to form a label binding substance in the labeling process as shown in Fig. 25 in place of labeling the analyte S using the label binding substance to which the labeling substance is bound in advance in the labeling process. In the method for electrochemically detecting an analyte shown in Fig. 25, the process of supplying a sample (Fig. 25A), the process of trapping an analyte (Fig. 25B), and the detection process (Fig. 25D) are the same as the process of supplying a sample (Fig. 23A), the process of trapping an analyte (Fig. 23B), and the detection process (Fig. 23D) in the above method shown in Fig. 23. On the other hand, in the method for electrochemically detecting an analyte shown in Fig. 25, conjugates [a first conjugate 290a and a second conjugate 290b] are added to the analyte S in the labeling process [see the process of adding a conjugate (C-1) of Fig. 25C]. In the process of adding a conjugate (C-1), the first conjugate 290a formed of a first binding substance 291a which binds to the analyte S and a second binding substance 291b is bound to the analyte S trapped by the trapping substance on the working electrode body 61 [see the process of adding the first conjugate (C-1-1) of Fig. 25C]. Then, the second conjugate 290b which binds to the second binding substance 291b included in the first conjugate 290a is bound to the first conjugate 290a [see the process of adding the second conjugate (C-1-2) of Fig. 25C]. Thereafter, a labeled form 290c in which the labeling substance 293 is immobilized on the second binding substance 291b via the modulator 292 is bound to the second conjugate 290b [see the process of adding a labeled form (C-2) of Fig. 25C]. In the method shown in Fig. 25, the first conjugate 290a and the labeled form 290c are bound to the second conjugate 290b via the same binding substance (the second binding substance 291b). However, the binding substance which binds to the second conjugate 290b in the first conjugate 290a may be mutually different from the binding substance which binds to the second conjugate 290b in the labeled form 290c.

### 2. Oxidation reduction current/electrochemiluminescence detection method

Subsequently, the oxidation reduction current/electrochemiluminescence detection method will be explained.

Referring Fig. 26, the oxidation reduction current/electrochemiluminescence detection method according to the present embodiment is largely different from the photoelectrochemical detection method in that a labeling substance which generates oxidation reduction current when a voltage is applied or a labeling substance which emits light when a voltage is applied is used as the labeling substance 393 in the labeling process [see Fig. 26C], and a voltage is applied to the working electrode 60 and the light generated from the labeling substance 393 is detected in the detection process [see Fig. 26D]. Therefore, the process of supplying a sample [see Fig. 26A] and the process of trapping an analyte [see Fig. 26B] are the same as those in the photoelectrochemical detection method. The detector 1 which is used in the method for electrochemically detecting an analyte according to the present embodiment does not include the light source 13 and includes a sensor for detecting light generated from the labeling substance. In the detection chip 20 to be used in the method for electrochemically detecting an analyte according to the present embodiment, the working electrode body 61 is composed of a conductive material.

In the labeling process, the user injects the label binding substance 390 into the detection chip 20 from the sample inlet 30b to allow the label binding substance 390 to be bound to the analyte S trapped by the trapping substance 281 on the working electrode body 61 [see the labeling process of Fig. 26C]. In the labeling process, a complex containing the trapping substance 281, the analyte S, and the label binding substance 390 is formed on the working electrode body 61.

The label binding substance 390 is formed of the first binding substance 291 to be bound to the analyte S, the modulator 292, and the labeling substance 393. In the label binding substance 390, the first binding substance 291 is linked to the labeling substance 393 via the modulator 292.

The labeling substance 393 is a labeling substance which emits light when a voltage is applied.

Examples of the labeling substance which emits light when a voltage is applied include luminol, lucigenin, pyrene, diphenylanthracene, and rubrene.

The luminescence of the labeling substance can be enhanced, for example, by using luciferin derivatives such as firefly luciferin and dehydro luciferin, enhancers such as phenols such as phenylphenol and chlorophenol or naphthols.

In the method for electrochemically detecting an analyte according to the present embodiment, as the labeling substance 393, a labeling substance which generates oxidation reduction current when a voltage is applied may be used in place of the labeling substance which emits light when a voltage is applied.

Examples of the labeling substance which generates oxidation reduction current when a voltage is applied include metal complexes containing metal which causes an electrically reversible oxidation-reduction reaction as a central metal. As the metal complexes, the same metal complexes described in the first embodiment can be used.

The first binding substance 291 and the modulator 292 are the same as those in the photoelectrochemical detection method.

Subsequently, the detection process is performed [see the detection process of Fig. 26D].

In the detection process, the user first injects an electrolytic solution through the sample inlet 30b of the detection chip 20. Thereafter, the user inserts the detection chip 20 into the chip insertion unit 11 of the detector 1 shown in Fig. 1. Then, the user gives an instruction to start measuring to the detector 1. Here, the electrode leads 71, 72, and 73 of the detection chip 20 inserted into the detector 1 are connected to the ammeter 14 and the power source 15. Then, a voltage is applied to the working electrode 60 by the power source 15 of the detector 1. Thus, the labeling substance 393 is excited to generate light. In the measurement of light based on the labeling substance 393, a photon counter is used. In this case, the light can be indirectly detected by using an optical fiber electrode obtained by forming a transparent electrode at the distal end of an optical fiber in place of the electrode (see U.S. Patent No. 5776672 and U.S. Patent No. 5972692).

Thereafter, a light value digitally converted by the A/D converting unit 16 is input into the control unit 17. Then, the control unit 17 estimates the amount of the analyte in the sample from the digitally converted current value based on a calibration curve indicating a relationship between a light value created in advance and the amount of the analyte. The control unit 17 creates a detection result screen for displaying the information on the estimated amount of the analyte on the display 12. Thereafter, the detection result screen created by the control unit 17 is sent to the display 12 so as to be displayed on the display 12.

In the method for electrochemically detecting an analyte according to the present embodiment, from the viewpoint of suppressing the generation of noises due to contaminants, the user may discharge a remaining liquid containing contaminants from the sample inlet 30b of the detection chip 20 after the process of trapping an analyte and wash an inside of the detection chip 20. In the washing of the inside of the detection chip 20, organic solvents such as a buffer (particularly a buffer containing a surfactant); purified water (particularly purified water containing a surfactant); and ethanol can be used.

In the method for electrochemically detecting an analyte according to the present embodiment, from the viewpoint of removing free label binding substance 390 which is not bound to the analyte S and improving the detection accuracy, the process of washing the inside of the detection chip 20 to remove the free label binding substance 390 may be further performed after the labeling process. For example, ethanol and purified water can be used for the washing.

In the present invention, the operation may be performed so as to form a complex containing the trapping substance 281 on the working electrode body 61, the analyte S, and the label binding substance 390 in the labeling process, in place of labeling the analyte S using the label binding substance 390 to which the labeling substance 393 is bound in advance in the labeling process.

In Fig. 26D, taking the case where the light is measured as an example, the process is illustrated. When the labeling substance 393 is the labeling substance which generates oxidation reduction current when a voltage is applied, the labeling substance 393 is excited to generate electrons. The generated electrons move to the working electrode 60. As a result, current flows between the working electrode 60 and the counter electrode 66. Then, the current flowing between the working electrode 60 and the counter electrode 66 is measured by the ammeter 14 of the detector 1. The current value measured by the ammeter 14 correlates with the number of the labeling substance. Therefore, the analyte can be quantified based on the measured current value.

### [Second example]

Hereinafter, the present invention will be described in detail with reference to Examples, however, the present invention is not limited thereto.

### (Preparation example 2-1)

1% by volume of 3-mercaptopropyltriethoxysilane (MPTES), i.e., a silane coupling agent, was added to toluene to prepare a solution A.

### (Preparation example 2-2) Production of working electrode substrate

A working electrode body composed of a thin film (about 200 nm in thickness) of tin-doped indium oxide was formed on the surface of the substrate body composed of silicon dioxide (SiO₂) by the spattering method. The thin film serves both as the conductive layer and the electron accepting layer. Subsequently, a working electrode lead for connecting to the ammeter was connected to the working electrode body.

Then, the surface of the working electrode body was brought into contact with the solution A obtained in Preparation example 2-1 to provide a thiol group on the surface of the main body of the working electrode.

Anti-mouse IgG F(ab')2 antibody as a trapping substance (manufactured by Dako) was reduced by bringing into contact with tris(2-carboxyethyl)phosphine hydrochloride (TCEP) fixed gel as a reducing agent (trade name: Immobilized TCEP Disulfide Reducing Gel, manufactured by Pierce], and an anti-mouse IgG Fab antibody was produced. 10 µg/mL of the obtained anti-mouse IgG Fab antibody was added to a tris buffer solution [pH 7.2, hereinafter called "TBS"] to prepare an antibody solution.

Then, the obtained antibody solution was dropped onto the working electrode body. The working electrode body was incubated at 4°C overnight to react the anti-mouse IgG Fab antibody with the thiol group on the working electrode body, and a dithiol bond was formed. Thus, the anti-mouse IgG Fab antibody was immobilized on the working electrode body. Then, 1 mM triethylene glycol mono-11-mercaptoundecyl ether [manufactured by Sigma] was dropped onto the working electrode body. Blocking was performed by incubating the working electrode body at 4°C overnight. Thus, a working electrode substrate was obtained.

### (Preparation example 2-3)

Acetonitrile and ethylene carbonate were mixed at a volume ratio of 2:3 to prepare an aprotic solvent. As an electrolyte salt, tetrapropylammonium iodide was dissolved in the aprotic solvent at a concentration of 0.6 M. As an electrolyte, iodine was dissolved in the obtained solution at a concentration of 0.06 M to prepare an electrolytic solution.

### (Preparation example 2-4)

A counter electrode of a 200-nm thick platinum thin film (conductive layer) was formed on the substrate body of silicon dioxide (SiO₂) by the spattering method to obtain a counter electrode substrate. The counter electrode lead for connecting to the ammeter was connected to the counter electrode. Thus, the counter electrode substrate was obtained.

### (Example 2-1)

Anti-mouse IgG antibody (manufactured by Sigma) was added to 0.1M sodium phosphate buffer (pH 7) so that the concentration was 7.3 µM. Then, a dimethyl sulfoxide (DMSO) solution of a cross-linker [N-(4-maleimidebutyryloxy)succinimide (GMBS), manufactured by Dojin Chemical Laboratory] [concentration of the cross-linker: 25 mM] was added to the obtained mixture so that the concentration of the cross-linker was 2.5 mM. The anti-mouse IgG antibody was reacted with GMBS by incubating the obtained mixture at room temperature for 30 minutes. The unreacted GMBS was removed by subjecting the obtained product to a desalting column [trade name: Zeba Spin Micro desalting Column, manufactured by Pierce] and a cross-linker-binding antibody was obtained.

The cross-linker-binding antibody thus obtained was mixed with AlexaFluor750-labeled thiolated DNA so that a molar ratio (a succinimide group introduced antibody/AlexaFluor750-labeled thiolated DNA) was 1/14. The cross-linker-binding antibody was reacted with the AlexaFluor750-labeled thiolated DNA by incubating the obtained mixture at room temperature for 4 hours. The AlexaFluor750-labeled thiolated DNA is DNA in which the 5' terminal of DNA with a length of 24 nucleotides [5'-AACTACTGTCTTCACGCAGAAAGC-3' (SEQ ID NO: 10), manufactured by Invitrogen] is labeled with AlexaFluor750 and the 3' terminal is modified by a thiol group.

The unreacted AlexaFluor750-labeled thiolated DNA was completely removed by filtering the obtained product through an ultrafiltration column [trade name: Amicon Ultra-0.5 100K, manufactured by Amicon] 4 times, and a label binding substance was obtained. 0.1M sodium phosphate buffer (pH 7.0) was added to the obtained label binding substance so that the concentration was 1 mg/mL, and a solution containing the label binding substance was obtained.

As for the obtained label binding substance, the number of the labeling substance per molecule of antibody (AlexaFluor750) was examined by measuring the absorption at an absorption wavelength of 749 nm of AlexaFluor750. As a result, it is confirmed that the number of the labeling substance per molecule of antibody (AlexaFluor750) is 9.

### (Comparative example 2-1)

Anti-mouse IgG antibody (manufactured by Sigma) was added to 0.1M sodium carbonate buffer (pH 8.5) so that the concentration was 14.6 µM. Then, a DMSO solution of AlexaFluor750 derivative [trade name: AlexaFluor750 carboxylic acid, succinimidyl ester, manufactured by (Invitrogen)] [concentration of [AlexaFluor750 derivative: 15 mM] was added to the obtained mixture so that the concentration of the AlexaFluor750 derivative was 1.5 mM. The anti-mouse IgG antibody was reacted with the AlexaFluor750 derivative by incubating the obtained mixture at room temperature for 1 hour. The unreacted AlexaFluor750 was removed by subjecting the obtained product to a desalting column [trade name: Zeba Spin Micro desalting Column, manufactured by Pierce] and a labeled antibody was obtained. 0.1M sodium phosphate buffer (pH 7.0) was added to the obtained labeled antibody so that the concentration was 2 mg/mL, and a solution containing the labeled antibody was obtained.

As for the obtained labeled antibody, the number of the labeling substance per molecule of antibody (AlexaFluor750) was examined by measuring the absorption at an absorption wavelength of 749 nm of AlexaFluor750. As a result, it is confirmed that the number of the labeling substance per molecule of antibody (AlexaFluor750) is 10.

### (Test example 2-1)

### (1-1) Trapping of analyte

Silicone rubber (0.1 mm in thickness) was placed around the working electrode of the working electrode substrate obtained in Preparation example 2-2 so that a partition was formed. Thereafter, a tris buffer solution (TBS-T) containing 0.05% by mass of polyoxyethylene sorbitan mono laurate (Tween-20) including 1% by mass of bovine serum albumin (BSA) was poured into the space surrounded by the working electrode substrate and the silicone rubber. Then, after the discharge of the liquid in the space, 30 µL of TBS-T containing 1% by mass of BSA containing 100 ng/mL mouse IgG (analyte) was added to the above space. Thereafter, the working electrode substrate was incubated at 25°C for 1 hour to allow the analyte [mouse IgG] to be trapped by the trapping substance [anti-mouse IgG F(ab')2 antibody] [see the process of trapping an analyte of Fig. 23B].

### (1-2) Labeling

The working electrode substrate was washed with TBS-T. Then, the solution containing 1 mg/mL of the label binding substance obtained in Example 2-1 was added to TBS-T containing 1% by mass of BSA so that the concentration of the label binding substance was 20 µg/mL. 30 µL of the obtained mixture was poured into the above space. Thereafter, the analyte on the working electrode was labeled by incubating the working electrode substrate at 25°C for 1 hour [see Fig. 23C] (Test No. 1).

On the other hand, the control experiment when no analyte was present was performed as follows. First, the working electrode substrate by which the analyte was not trapped was washed with TBS-T. Then, the solution containing 1 mg/mL of the label binding substance obtained in Example 2-1 was added to TBS-T containing 1% by mass of BSA so that the concentration of the label binding substance was 20 µg/mL. 30 µL of the obtained mixture was poured into the above space. Thereafter, the working electrode substrate was incubated at 25°C for 1 hour (Test No. 2) .

### (2) Control experiment

An analyte [mouse IgG] was trapped by a trapping substance [anti-mouse IgG F(ab')2 antibody] by performing the same operation as (1-1).

Thereafter, the same operation as (1-2) was performed to label the analyte on the working electrode (Test No. 3) except that the solution containing the labeled antibody obtained in Comparative example 2-1 was used in place of the solution containing the label binding substance obtained in Example 2-1 in (1-2).

On the other hand, the control experiment when no analyte was present was performed as follows. The working electrode substrate by which the analyte was not trapped was washed with TBS-T. Then, the solution containing 2 mg/mL of the labeled antibody obtained in Comparative example 2-1 was added to TBS-T containing 1% by mass of BSA so that the concentration of the label binding substance was 20 µg/mL. 30 µL of the obtained mixture was poured into the above space. Thereafter, the working electrode substrate was incubated at 25°C for 1 hour (Test No. 4).

### (3) Measurement of photocurrent

Silicone rubber was placed around the working electrode substrates of Test Nos. 1-4 so that a 0.2-mm-thick side wall was formed. Then, the space surrounded by the working electrode substrate and the silicone rubber was filled with the electrolytic solution obtained in Preparation example 2-3. Thereafter, the space filled with the electrolytic solution was sealed with the counter electrode substrate obtained in Preparation example 2-4 from the upper side of the working electrode substrate. Thus, the working electrode and the counter electrode are brought into contact with the electrolytic solution. Then, the detection chip including the working electrode substrate and the counter electrode was placed in an electrochemical measurement device. The working electrode lead and the counter electrode lead were connected to the ammeter.

The light source (wavelength: 781 nm, laser light source with an output power of 13 mW) emits excitation light from the working electrode substrate side toward the counter electrode substrate. The labeling substance is excited by photoirradiation, thereby generating electrons. When the generated electrons are transported to the working electrode, current flows between the working electrode and the counter electrode. Then, the current was measured [see the detection process of Fig. 23D].

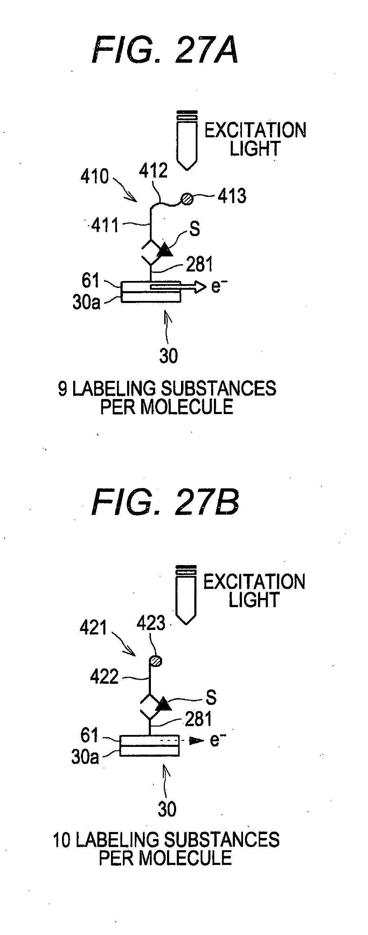

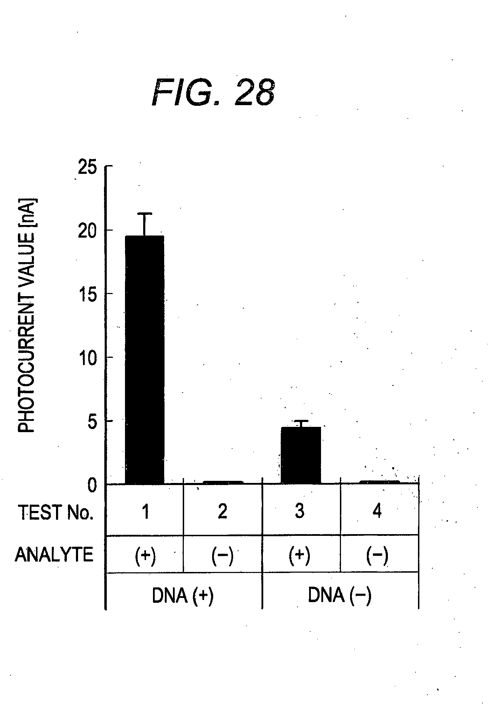

Fig. 27A shows an outline explanatory view showing the detection process when an analyte is detected using the label binding substance obtained in Example 2-1 in Test example 2-1 (Test No. 1). Fig. 27B shows an outline explanatory view showing the detection process when an analyte is detected using the labeled antibody obtained in Comparative example 2-1 in Test example 2-1 (Test No. 3). Fig. 28 shows examined results of a relationship between the kind of the detection method and photocurrent in Test example 2-1.

In the method for electrochemically detecting an analyte of Test No. 1, the label binding substance obtained in Example 2-1 [see 410 in Fig. 27A] is used. Therefore, as shown in Fig. 27A, photocurrents are generated from 9 labeling substances per molecule of antibody by the irradiation of the excitation light in the detection process. On the other hand, in the method for electrochemically detecting an analyte of Test No. 3, the labeled antibody obtained in Comparative example 2-1 [see 421 in Fig. 27B] is used. Thus, as shown in Fig. 27B, photocurrents are generated from 10 labeling substances per molecule of antibody by the irradiation of the excitation light in the detection process. Therefore, it may be predicted that the photocurrent detected by the method for electrochemically detecting an analyte of Test No. 1 is almost the same as or slightly smaller than that detected by the method for electrochemically detecting an analyte of Test No. 3.

However, from the results shown in Fig. 28, it is found that the photocurrent detected by the method for electrochemically detecting an analyte of Test No. 1 is 20 nA, while the photocurrent detected by the method for electrochemically detecting an analyte of Test No. 3 is 4.5 nA. From these results, it is found that, unexpectedly, the photocurrent detected by the method for electrochemically detecting an analyte of Test No. 1 is far larger than that detected by the method for electrochemically detecting an analyte of Test No. 3.

As for the label binding substance obtained in Example 2-1 and the labeled antibody obtained in Comparative example 2-1, the same antibody is used as the binding substance. Thus, it is considered that the binding efficiency of the label binding substance obtained in Example 2-1 to the analyte is almost equal to that of the labeled antibody obtained in Comparative example 2-1. In the case where there is no analyte, the photocurrent is about 0 nA (see Test Nos. 2 and 4 in Fig. 28). Thus, it is considered that a difference between the photocurrent detected by the method for electrochemically detecting an analyte of Test No. 1 and the photocurrent detected by the method for electrochemically detecting an analyte of Test No. 3 is not caused by the influence of noise. Therefore, it is considered that a difference between the photocurrent detected by the method for electrochemically detecting an analyte of Test No. 1 and the photocurrent detected by the method for electrochemically detecting an analyte of Test No. 3 is dependent on the presence of DNA between the labeling substance and the binding substance.

### (Test example 2-2)

### (Example 2-2)

### (1-1) Trapping of analyte

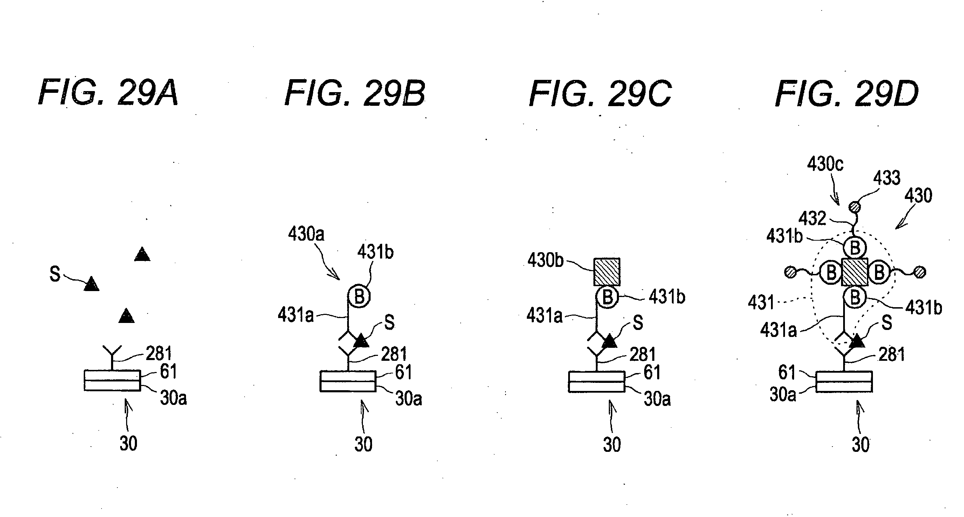

Silicone rubber (0.1 mm in thickness) was placed around the working electrode of the working electrode substrate obtained in Preparation example 2-2 so that a partition was formed. Thereafter, TBS-T containing 1% by mass of BSA was poured into the space surrounded by the working electrode substrate and the silicone rubber. Then, after the discharge of the liquid in the space, 30 µL of TBS-T containing 1% by mass of BSA containing 100 ng/mL mouse IgG (analyte) was added to the above space [see Fig. 29A]. Thereafter, the working electrode substrate was incubated at 25°C for 1 hour to allow the analyte [mouse IgG] to be trapped by the trapping substance [anti-mouse IgG F(ab')2 antibody] [see Fig. 29B].

### (1-2) Labeling

The working electrode substrate was washed with TBS-T. Then, 4 µg/mL of a solution containing 2.1 mg/mL biotin-labeled anti-mouse IgG antibody [manufactured by Sigma] was added to TBS-T containing 1% by mass of BSA. 30 µL of the obtained mixture was poured into the above space. Thereafter, the working electrode substrate was incubated at 25°C for 1 hour. Thus, the biotin-labeled anti-mouse IgG antibody (the first conjugate) [see 430a in Fig. 29B] was bound to the analyte [see S in Fig. 29B] trapped by the trapping substance [see 281 in Fig. 29B]. The biotin-labeled anti-mouse IgG antibody is obtained by labeling an anti-mouse IgG antibody [see 431a in Fig. 29B] with biotin [see 431b in Fig. 29B].

The working electrode substrate was washed with TBS-T. A solution containing streptoavidin [manufactured by Vector] as the second conjugate [concentration of the second conjugate: 2 mg/mL] was added to TBS-T so that the concentration of the second conjugate was 4 µg/mL. 30 µL of the obtained mixture was poured into the above space. Thereafter, the working electrode substrate was incubated at 25°C for 30 minutes. Thus, streptoavidin [see 430b in Fig. 29C] was bound to biotin-labeled anti-mouse IgG antibody on the working electrode.

The working electrode substrate was washed with TBS-T. Then, a solution containing biotinylated AlexaFluor750-labeled DNA [concentration of the labeled form: 100 µM] as the labeled form was added to TBS-T so that the concentration of the labeled form was 1 µM. 30 µL of the obtained mixture was poured into the above space. Thereafter, the working electrode substrate was incubated at 25°C for 30 minutes. Thus, a complex containing the analyte, the first conjugate, the second conjugate, and the labeled form was formed on the working electrode (Test No. 5). The complex formed of the first conjugate and the second conjugate [see 431 in Fig. 29D] corresponds to the binding substance in the label binding substance obtained in Example 2-1. That is, on the working electrode, AlexaFluor750 as the labeling substance [see 433 in Fig. 29D] is bound to the binding substance bound to the analyte [see 431 in Fig. 29D] via DNA as the modulator [see 432 in Fig. 29D].

The biotinylated AlexaFluor750-labeled DNA is DNA in which the 5' terminal of DNA with a length of 24 nucleotides [5'-AACTACTGTCTTCACGCAGAAAGC-3' (SEQ ID NO: 10), manufactured by Invitrogen] is modified by biotin and the 3' terminal is labeled with AlexaFluor750.

On the other hand, the operation was performed in the same manner as described above except that the analyte was not used. The control experiment when the analyte was not present in Example 2-2 was performed (Test No. 6).

### (2) Control experiment

### (Comparative example 2-2)

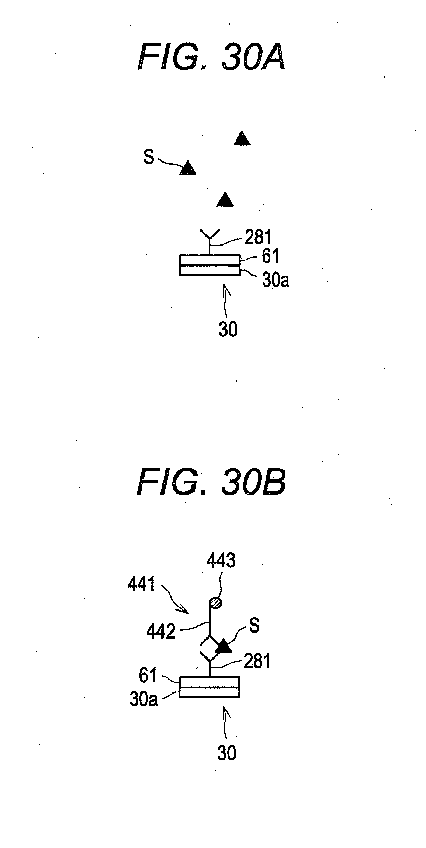

The labeled antibody to which the labeling substance was directly bound to the antibody [the labeled antibody obtained in Comparative example 2-1] was used in place of the first conjugate, the second conjugate, and the labeled form. The control experiment was performed in the following manner. The same operation as (1-1) was performed to allow the analyte [mouse IgG] to be trapped by the trapping substance [anti-mouse IgG F(ab')2 antibody] [see Fig. 30B].

After washing the working electrode substrate with TBS-T, the solution containing 1 mg/mL of the labeled antibody obtained in Comparative example 2-1 was added to TBS-T containing 1% by mass of BSA so that the concentration of the labeled antibody was 20 µg/mL. 30 µL of the obtained mixture was poured into the above space. Thereafter, the analyte on the working electrode was labeled by incubating the working electrode substrate at 25°C for 1 hour [see Fig. 30B] (Test No. 7).

On the other hand, the control experiment when no analyte was present in Comparative example 2-2 was performed as follows. First, the working electrode substrate by which the analyte was not trapped was washed with TBS-T. Then, the solution containing 2 mg/mL of the labeled antibody obtained in Comparative example 2-1 was added to TBS-T containing 1% by mass of BSA so that the concentration of the labeled antibody was 20 µg/mL. 30 µL of the obtained mixture was poured into the above space. Thereafter, the working electrode substrate was incubated at 25°C for 1 hour (Test No. 8).

### (3) Measurement of photocurrent

Silicone rubber was placed around the working electrode substrates of Test Nos. 5 to 8 so that a 0.2-mm-thick side wall was formed. Then, the space surrounded by the working electrode substrate and the silicone rubber was filled with the electrolytic solution obtained in Preparation example 2-3. Thereafter, the space filled with the electrolytic solution was sealed with the counter electrode substrate obtained in Preparation example 2-4 from the upper side of the working electrode substrate. Thus, the working electrode and the counter electrode are brought into contact with the electrolytic solution. Then, the detection chip including the working electrode substrate and the counter electrode was placed in an electrochemical measurement device. The working electrode lead and the counter electrode lead were connected to the ammeter.

The light source (wavelength: 781 nm, laser light source with an output power of 13 mW) emits excitation light from the working electrode substrate side toward the counter electrode substrate. Alexa Fluor750, i.e., a labeling substance [see 433 in Fig. 29D] is excited by light radiation and electrons are generated. When the generated electrons are transported to the working electrode, current flows between the working electrode and the counter electrode. Then, the electric current was measured.

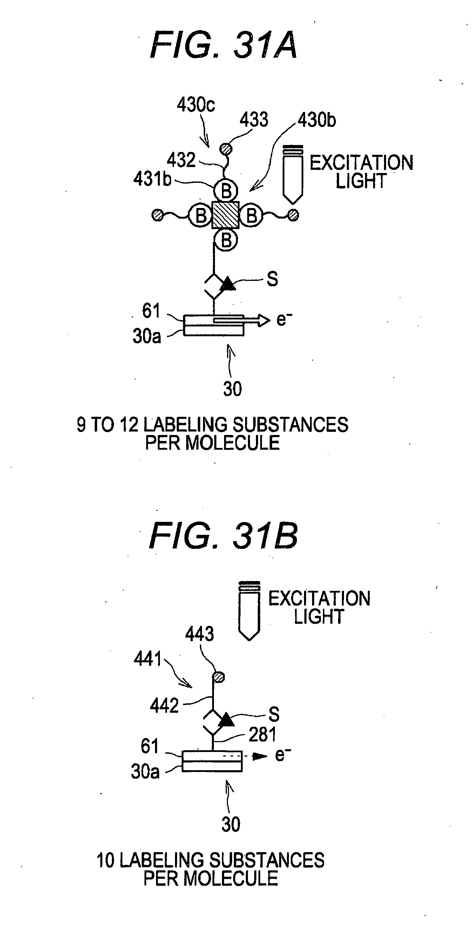

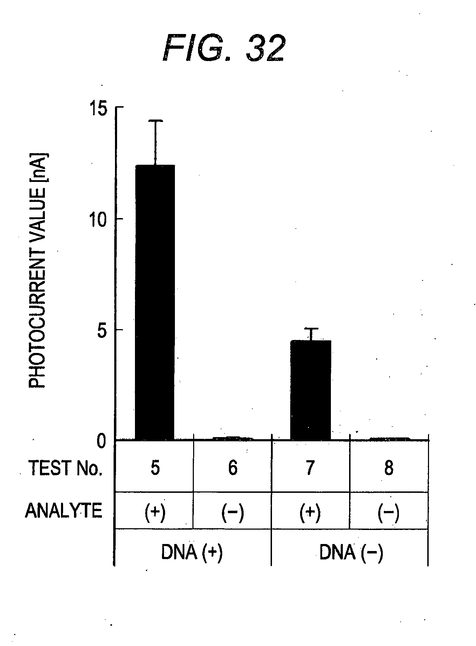

Fig. 31A shows an outline explanatory view showing the detection process of the method for electrochemically detecting an analyte of Test No. 5 in Example 2-2. Fig. 31B shows an outline explanatory view showing the detection process of the method for electrochemically detecting an analyte of Test No. 7 in Example 2. Fig. 32 shows examined results of a relationship between the kind of the detection method and photocurrent in Test example 2-2.

In the method for electrochemically detecting an analyte of Test No. 5, as shown in Fig. 31A, the biotin labeled anti-mouse IgG antibody as the first conjugate [see 431a in the drawing], streptoavidin as the second conjugate [see 430b in the drawing], and the biotinylated AlexaFluor750-labeled DNA as the labeled form [see 430c in the drawing] are used. Here, the molecular weight of streptoavidin is about 53 kDa. Taking into consideration a steric hindrance when biotin of the biotin labeled anti-mouse IgG antibody is bound to streptoavidin, the number of AlexaFluor750 which can be bound to the biotin labeled anti-mouse IgG antibody of a molecule (molecular weight: about 150 kDa) is considered to be up to about four molecules. Further, the biotinylated AlexaFluor750-labeled DNA can be bound to three sites of four biotin-binding sites in the streptoavidin. Therefore, as shown in Fig. 31A, photocurrents are generated from 9 to 12 labeling substances per molecule of antibody by the irradiation of the excitation light in the detection process. On the other hand, in the method for electrochemically detecting an analyte of Test No. 7, the labeled antibody [see 441 in Fig. 31B] is used. Thus, as shown in Fig. 31B, photocurrents are generated from 10 labeling substances [AlexaFluor750, see 443 in Fig. 31B] per molecule of antibody [see 442 in Fig. 31B] by the irradiation of the excitation light in the detection process. Therefore, unless taking into consideration DNA, it may be predicted that the photocurrent detected by the method for electrochemically detecting an analyte of Test No. 5 is the same as that detected by the method for electrochemically detecting an analyte of Test No. 7.

However, from the results shown in Fig. 32, it is found that the photocurrent detected by the method for electrochemically detecting an analyte of Test No. 5 is 12 nA, while the photocurrent detected by the method for electrochemically detecting an analyte of Test No. 7 is 4.5 nA. From these results, it is found that, unexpectedly, the photocurrent detected by the method for electrochemically detecting an analyte of Test No. 5 is far larger than that detected by the method for electrochemically detecting an analyte of Test No. 7. Therefore, it is considered that a difference between the photocurrent detected by the method for electrochemically detecting an analyte of Test No. 5 and the photocurrent detected by the method for electrochemically detecting an analyte of Test No. 7 is dependent on the presence of DNA between the labeling substance and the binding substance.

A DNA exhibits hydrophilicity. Therefore, it is considered that the DNA has an interaction (hydrophilic interaction) with the working electrode body into which an electrolytic solution containing an aprotic solvent and a hydrophilic triethylene glycol (TEG) chain are introduced. On the other hand, when the electrolytic solution contains a protic solvent and a hydrophobic functional group is introduced into the working electrode body, it is considered that the interaction (hydrophobic interaction) is generated by using a substance having hydrophobicity in place of DNA.

The above results suggest that the analyte can be detected with high sensitivity by using the label binding substance in which the labeling substance is at least immobilized on the binding substance via the modulator which generates an interaction with the electrolytic solution and the working electrode site except the site bound to the trapping substance in order to label the analyte.

### (Preparation example 2-5)

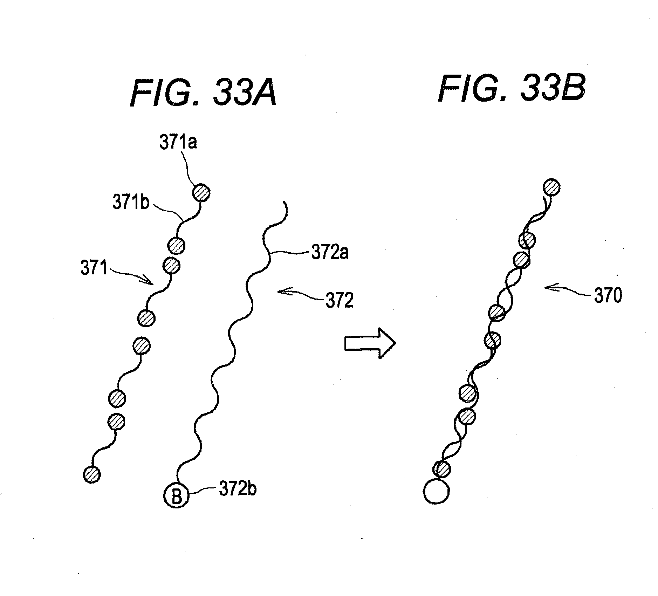

Biotinylated-DNA and Alexa Fluor 750-labeled DNA were added to 1M sodium chloride-containing phosphate buffer so that their concentrations were 1 µM and 10 µM, respectively. The obtained mixture was heated at 80°C for 1 minute. Thereafter, the mixture was cooled to 4°C while decreasing the temperature of the mixture concerned to 1°C/min. Thus, the biotinylated-DNA was hybridized with the Alexa Fluor 750-labeled DNA to produce a biotinylated-DNA/Alexa Fluor 750-labeled DNA complex (see 370 in Fig. 33). The biotinylated-DNA is DNA (DNA whose 5' terminal of phosphate group is a biotinylated adenine base through the (CH₂)₃ linker) in which the 5' terminal of DNA with a length of 84 nucleotides [5'-biotin-AAACCACGGCCCTAGGGACAACGACCACGGCCCTAGGGACAACGACCACGGCCCTAGGG ACAACGACCACGGCCCTAGGGACAACGA-3', manufactured by Invitrogen, (SEQ ID NO: 11), see 372a in Fig. 33] is labeled with biotin (see 372b in Fig. 33) (see 372 in Fig. 33). The AlexaFluor 750-labeled DNA is DNA in which the 3' and 5' terminals of DNA with a length of 20 nucleotides [5'-CGTTGTCCCTAGGGCCGTGGGTATGCGCGCTGCTATGCCG-3', manufactured by Invitrogen, (SEQ ID NO: 12), see 371b in Fig. 33] are labeled with AlexaFluor 750 (see 371a in Fig. 33) (see 371 in Fig. 33).

### (Example 2-3)

### Detection of mouse IgG with multivalent-labeled DNA (1-1) Trapping of analyte

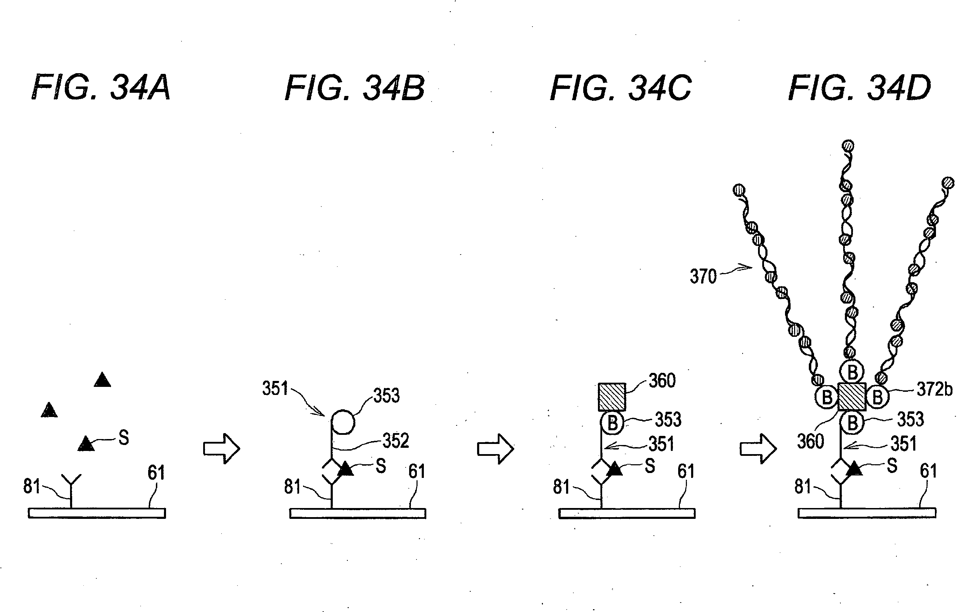

Silicone rubber (0.1 mm in thickness) was placed around the working electrode of the working electrode substrate obtained in Preparation example 2-2 so that a partition was formed. Thereafter, TBS-T containing 0.4% by mass of Block Ace [manufactured by DS Pharma Biomedical Co., Ltd.] was poured into the space surrounded by the working electrode substrate and the silicone rubber. Then, the working electrode substrate was incubated at 25°C for 30 minutes. After the washing the working electrode substrate with TBS-T, 30 µL of TBS-T containing 1% by mass of BSA containing 10 ng/mL mouse IgG (analyte) was added to the above space. Thereafter, the working electrode substrate was incubated at 25°C for 1 hour to allow the analyte to be trapped by the trapping substance [anti-mouse IgG F(ab')₂ antibody, see 81 in Fig. 34] immobilized on the working electrode body of the working electrode substrate (see 61 in Fig. 34) [see Fig. 34A].

### (1-2) Labeling

The working electrode substrate subjected to the process (1-1) was washed with TBS-T. Then, biotin-labeled anti-mouse IgG F(ab') ₂ antibody [manufactured by Dako] was added to TBS-T containing 1% by mass of BSA so that the concentration of the antibody was 4 µg/mL. Thereafter, 30 µL of the obtained mixture was poured into the above space. Thereafter, the working electrode substrate was incubated at 25°C for 1 hour. Thus, the biotin-labeled anti-mouse IgG F(ab') ₂ antibody (the first conjugate) (see 351 in Fig. 34) was bound to the analyte (see S in Fig. 34) trapped by the trapping substance (see 81 in Fig. 34) [see Fig. 34B]. The biotin-labeled anti-mouse IgG F(ab') ₂ antibody was obtained by labeling anti-mouse IgG F(ab') ₂ antibody (see 352 in Fig. 34) with biotin (see 353 in Fig. 34).

Then, the working electrode substrate was washed with TBS-T. Then, streptoavidin [manufactured by Vector Laboratories] (the second conjugate) was added to TBS-T so that its concentration was 4 µg/mL. 30 µL of the obtained mixture was poured into the above space. Thereafter, the working electrode substrate was incubated at 25°C for 30 minutes. Thus, streptoavidin (see 360 in Fig. 34) was bound to biotin (353 in Fig. 34) in the biotin-labeled anti-mouse IgG F(ab') ₂ antibody (see 351 in Fig. 34) on the working electrode body (see 61 in Fig. 34) [see Fig. 34C].

### (1-3) Dye-labeling

The working electrode substrate subjected to the process (1-2) was washed with TBS-T. Then, TBS-T was added to 30 µL of a solution containing the biotinylated-DNA/Alexa Fluor 750-labeled DNA complex obtained in Preparation example 2-5 (concentration of the complex: 93 µg/mL) in an amount 10 times the amount of the solution. Thereafter, 30 µL of the obtained mixture was poured into the above space. Thereafter, the working electrode substrate was incubated at 25°C for 30 minutes. Thus, a complex containing the analyte (see S in Fig. 34), the biotin-labeled anti-mouse IgG F(ab') ₂ antibody (the first conjugate) (see 351 in Fig. 34), streptoavidin (the second conjugate) (see 360 in Fig. 34), and the biotinylated-DNA/Alexa Fluor 750 labeled DNA complex (see 370 in Fig. 34) as a labeled form was formed on the working electrode body (see 61 in Fig. 34) [see Fig. 34D]. The complex formed of the first conjugate and the second conjugate corresponds to the binding substance in the label binding substance obtained in Example 2-1. That is, on the working electrode, Alexa Fluor 750 as the labeling substance is bound to the binding substance bound to the analyte via DNA as the modulator.

### (2) Measurement of photocurrent

Silicone rubber was placed around the working electrode substrate so that a 0.2-mm-thick side wall was formed. Then, the space surrounded by the working electrode substrate and the silicone rubber was filled with the electrolytic solution obtained in Preparation example 2-3. The space filled with the electrolytic solution was sealed with the counter electrode substrate obtained in Preparation example 2-4 from the upper side of the working electrode substrate. Thus, the working electrode and the counter electrode are brought into contact with the electrolytic solution. Then, the detection chip including the working electrode substrate and the counter electrode was placed in an electrochemical measurement device. The working electrode lead and the counter electrode lead were connected to the ammeter.

The light source (wavelength: 781 nm, laser light source with an output power of 13 mW) emits excitation light from the working electrode substrate side toward the counter electrode substrate. The labeling substance Alexa Fluor 750 is excited by photoirradiation, thereby generating electrons. When the generated electrons are transported to the working electrode, current flows between the working electrode and the counter electrode. Then, the electric current was measured (Test No. 9).

The same operation as above-described was performed by using the complex used in Test No. 5 (biotinylated Alexa Fluor 750-labeled DNA: SEQ ID NO 10) in place of the biotinylated-DNA/Alexa Fluor 750 labeled DNA complex obtained in Preparation example 2-5, and the resulting product was used for the control experiment (Test No. 10).

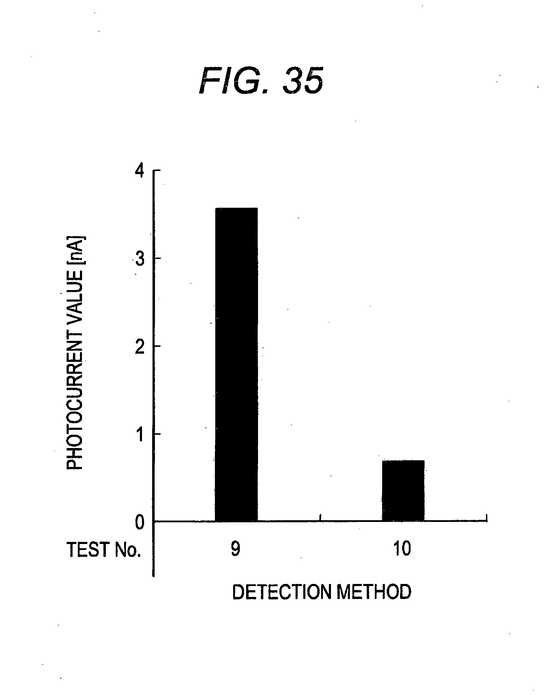

Fig. 35 shows examined results of a relationship between the kind of the detection method and photocurrent in Example 2-3.

From the results shown in Fig. 35, it is found that the photocurrent detected by the method for electrochemically detecting an analyte of Test No. 9 is 3.6 nA, while the photocurrent detected by the method for electrochemically detecting an analyte of Test No. 10 is 0.68 nA. From these results, it is found that the photocurrent detected by the method for electrochemically detecting an analyte of Test No. 9 is larger than the photocurrent detected by the method for electrochemically detecting an analyte of Test No. 10.

The above results suggest that the analyte can be detected with higher sensitivity by using a multivalent-labeled binding substance in which more labeling substances are immobilized to the binding substance through the interaction between modulators in order to label the analyte.

### (Example 2-4)

### Quantitative detection of mouse IgG with multivalent-labeled DNA

### (1-1) Trapping of analyte

Silicone rubber (0.1 mm in thickness) was placed around the working electrode of the working electrode substrate obtained in Preparation example 2-2 so that a partition was formed. Thereafter, TBS-T containing 0.4% by mass of Block Ace [manufactured by DS Pharma Biomedical Co., Ltd.] was poured into the space surrounded by the working electrode substrate and the silicone rubber. Thereafter, the working electrode substrate was incubated at 25°C for 30 minutes. After the washing of the working electrode substrate with TBS-T, 30 µL of TBS-T containing 1% by mass of BSA containing 10 ng/mL mouse IgG (analyte) was added to the above space. Thereafter, the working electrode substrate was incubated at 25°C for 1 hour to allow the analyte [mouse IgG] to be trapped by the trapping substance [anti-mouse IgG F(ab')₂ antibody].

### (1-2) Labeling

The working electrode substrate subjected to the process (1-1) was washed with TBS-T. Then, biotin-labeled anti-mouse IgG F(ab') ₂ antibody [manufactured by Dako] was added to TBS-T containing 1% by mass of BSA so that the concentration of the antibody was 4 µg/mL. Thereafter, 30 µL of the obtained mixture was poured into the above space. Thereafter, the working electrode substrate was incubated at 25°C for 1 hour. Thus, the biotin-labeled anti-mouse IgG F(ab') ₂ antibody (the first conjugate) was bound to the analyte trapped by the trapping substance. The biotin-labeled anti-mouse IgG F(ab') ₂ antibody was obtained by labeling the anti-mouse IgG F(ab') ₂ antibody with biotin.

Then, the working electrode substrate was washed with TBS-T. Thereafter, 2 mg/mL of streptoavidin[manufactured by Vector Laboratories] (the second conjugate) was added to TBS-T so that its concentration was 4 µg/mL. 30 µL of the obtained mixture was poured into the above space. Thereafter, the working electrode substrate was incubated at 25°C for 30 minutes. Thus, streptoavidin was bound to the biotin-labeled anti-mouse IgG F(ab') ₂ antibody on the working electrode.

### (1-3) Dye-labeling

The working electrode substrate subjected to the process (1-2) was washed with TBS-T. Then, TBS-T was added to 30 µL of a solution containing the biotinylated-DNA/Alexa Fluor 750-labeled DNA complex obtained in Preparation example 2-5 (concentration of the complex: 93 µg/mL) in an amount 10 times the amount of the solution. Thereafter, 30 µL of the obtained mixture was poured into the above space. Thereafter, the working electrode substrate was incubated at 25°C for 30 minutes. Thus, a complex containing the analyte, the first conjugate, the second conjugate, and the labeled form was formed on the working electrode. The complex formed of the first conjugate and the second conjugate corresponds to the binding substance in the label binding substance obtained in Example 2-1. That is, on the working electrode, Alexa Fluor 750 as the labeling substance is bound to the binding substance bound to the analyte via DNA as the modulator.

### (2) Measurement of photocurrent

Silicone rubber was placed around the working electrode substrate so that a 0.2-mm-thick side wall was formed. Then, the space surrounded by the working electrode substrate and the silicone rubber was filled with the electrolytic solution obtained in Preparation example 2-3. The space filled with the electrolytic solution was sealed with the counter electrode substrate obtained in Preparation example 2-4 from the upper side of the working electrode substrate. Thus, the working electrode and the counter electrode are brought into contact with the electrolytic solution. Then, the detection chip including the working electrode substrate and the counter electrode was placed in an electrochemical measurement device. The working electrode lead and the counter electrode lead were connected to the ammeter.

The light source (wavelength: 781 nm, laser light source with an output power of 13 mW) emits excitation light from the working electrode substrate side toward the counter electrode substrate. The labeling substance Alexa Fluor 750 is excited by photoirradiation, thereby generating electrons. When the generated electrons are transported to the working electrode, current flows between the working electrode and the counter electrode. Then, the electric current was measured.

The current was measured by performing the same operation as described above except that 100 pg/mL of mouse IgG and 1 ng/mL of mouse IgG were used as the analyte in place of 10 ng/mL of mouse IgG in (1-1). The operation was performed in the same manner as described above except that the analyte was not used. The control experiment when the analyte was not present was performed.

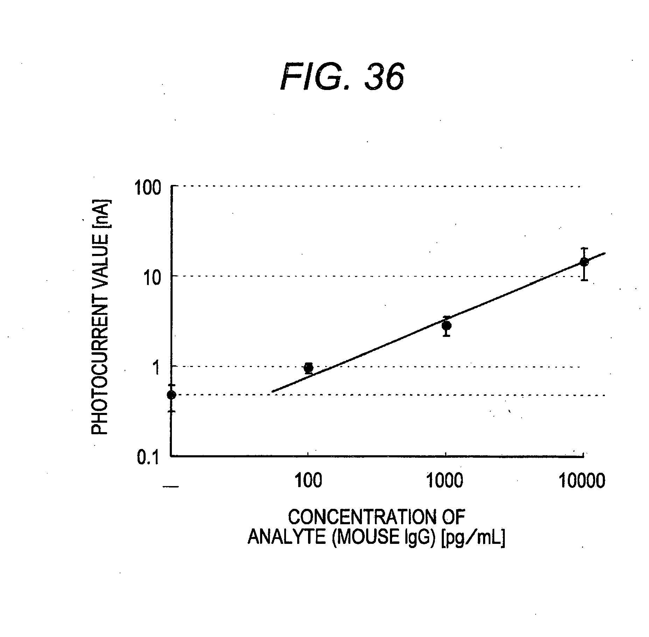

Fig. 36 is a graph showing examined results of a relationship between the concentration of the analyte (mouse IgG) and photocurrent in Example 2-4.

From the results shown in Fig. 36, it is found that the photocurrents detected when the concentrations of the analyte is 100 pg/mL, 1 ng/mL, and 10 ng/mL are 0.95 nA, 2.85 nA, and 14.9 nA, respectively. It is found that the photocurrent detected when the analyte is not present is 0.47 nA. From these results, it is found that the photocurrent detected by the method for electrochemically detecting an analyte is increased according to the concentration of the analyte. From these results, it is found that when the concentration of the analyte is in the range of 100 pg/mL to 10 ng/mL, the analyte can be quantitatively detected.

The above results suggest that the analyte can be quantitatively detected with high sensitivity by using a multivalent-labeled binding substance in which more labeling substances are immobilized to the binding substance through the interaction between modulators in order to label the analyte.

### (Preparation example 2-6) Production of working electrode substrate

A working electrode body composed of a thin film (about 200 nm in thickness) of tin-doped indium oxide was formed on the surface of the substrate body composed of silicon dioxide (SiO₂) by the spattering method. The thin film serves both as the conductive layer and the electron accepting layer. Subsequently, a working electrode lead for connecting to the ammeter was connected to the working electrode body.

Then, the surface of the working electrode body was brought into contact with the solution A obtained in Preparation example 2-1 to provide a thiol group on the surface of the main body of the working electrode.

Anti-human interleukin-6 antibody [manufactured by BioLegend] as a trapping substance was reduced by bringing into contact with tris(2-carboxyethyl)phosphine hydrochloride (TCEP) fixed gel as a reducing agent (trade name: Immobilized TCEP Disulfide Reducing Gel, manufactured by Pierce], and a reduced heavy chain of anti-human interleukin-6 antibody was produced. 10 µg/mL of the obtained anti-human interleukin-6 antibody was added to TBS to prepare an antibody solution.

Then, the obtained antibody solution was dropped onto the working electrode body. The working electrode body was incubated at 4°C overnight to react the anti-human interleukin-6 antibody with the thiol group on the working electrode body, and a dithiol bond was formed. Thus, the anti-human interleukin-6 antibody was immobilized on the working electrode body. Then, 1 mM triethylene glycol mono-11-mercaptoundecyl ether [manufactured by Sigma] was dropped onto the working electrode body. Blocking was performed by incubating the working electrode body at 4°C overnight. Thus, a working electrode substrate was obtained.

### (Example 2-5)

### Quantitative detection of interleukin-6 (IL-6) with multivalent-labeled DNA

### (1-1) Trapping of analyte

Silicone rubber (0.1 mm in thickness) was placed around the working electrode of the working electrode substrate obtained in Preparation example 2-6 so as to form a partition. Thereafter, TBS-T containing 0.4% by mass of Block Ace [manufactured by DS Pharma Biomedical Co., Ltd.] was poured into the space surrounded by the working electrode substrate and the silicone rubber. The liquid in the above space was discharged. Thereafter, 30 µL of the analyte solution (concentration of the analyte: 500 pg/mL) obtained by diluting human-interleukin-6 as an analyte with TBS-T containing 25% by mass of bovine serum [manufactured by Thermo Scientific] and 0.75% by mass of BSA was added to the above space. Thereafter, the working electrode substrate was incubated at 25°C for 1 hour to allow the analyte [interleukin-6] to be trapped by the trapping substance [anti-human interleukin-6 antibody].

### (1-2) Labeling

The working electrode substrate subjected to the process (1-1) was washed with TBS-T. Then, biotin-labeled anti-human interleukin-6 antibody [manufactured by BioLegend] was added to TBS-T containing 1% by mass of BSA so that the concentration was 1 µg/mL. Thereafter, 30 µL of the obtained mixture was poured into the above space. Thereafter, the working electrode substrate was incubated at 25°C for 1 hour. Thus, the biotin-labeled anti-human interleukin-6 antibody (the first conjugate) was bound to the analyte trapped by the trapping substance. The biotin-labeled anti-human interleukin-6 antibody was obtained by labeling the anti-human interleukin-6 antibody with biotin.

Then, the working electrode substrate was washed with TBS-T. Then, 2 mg/mL of streptoavidin [manufactured by Vector Laboratories] (the second conjugate) was added to TBS-T so that its concentration was 4 µg/mL. 30 µL of the obtained mixture was poured into the above space. Thereafter, the working electrode substrate was incubated at 25°C for 30 minutes. Thus, streptoavidin was bound to the biotin-labeled human interleukin-6 antibody on the working electrode.

### (1-3) Dye-labeling

The working electrode substrate subjected to the process (1-2) was washed with TBS-T. Then, TBS-T was added to 100 µL of a solution containing the biotinylated-DNA/Alexa Fluor 750-labeled DNA complex obtained in Preparation example 2-5 (concentration of the complex: 93 µg/mL) in an amount 10 times the amount of the solution. Thereafter, 30 µL of the obtained mixture was poured into the above space. Thereafter, the working electrode substrate was incubated at 25°C for 30 minutes. Thus, a complex containing the analyte, the first conjugate, the second conjugate, and the labeled form was formed on the working electrode. The complex formed of the first conjugate and the second conjugate corresponds to the binding substance in the label binding substance obtained in Example 2-1. That is, on the working electrode, Alexa Fluor 750 as the labeling substance is bound to the binding substance bound to the analyte via DNA as the modulator.

### (2) Measurement of photocurrent

Silicone rubber was placed around the working electrode substrate so that a 0.2-mm-thick side wall was formed. Then, the space surrounded by the working electrode substrate and the silicone rubber was filled with the electrolytic solution obtained in Preparation example 2-3. The space filled with the electrolytic solution was sealed with the counter electrode substrate obtained in Preparation example 2-4 from the upper side of the working electrode substrate. Thus, the working electrode and the counter electrode are brought into contact with the electrolytic solution. Then, the detection chip including the working electrode substrate and the counter electrode was placed in an electrochemical measurement device. The working electrode lead and the counter electrode lead were connected to the ammeter.

The light source (wavelength: 781 nm, laser light source with an output power of 13 mW) emits excitation light from the working electrode substrate side toward the counter electrode substrate. The labeling substance Alexa Fluor 750 is excited by photoirradiation, thereby generating electrons. When the generated electrons are transported to the working electrode, current flows between the working electrode and the counter electrode. Then, the electric current was measured.

The current was measured by performing the same operation as described above except that an analyte solution having an analyte concentration of 7. 8 pg/mL, 15.6 pg/mL, 31. 2 pg/mL, 62.5 pg/mL, 125 pg/mL or 250 pg/mL was used in place of an analyte solution having a concentration of 500 pg/mL in (1-1). The operation was performed in the same manner as described above except that the analyte was not used. The control experiment when the analyte was not present was performed.

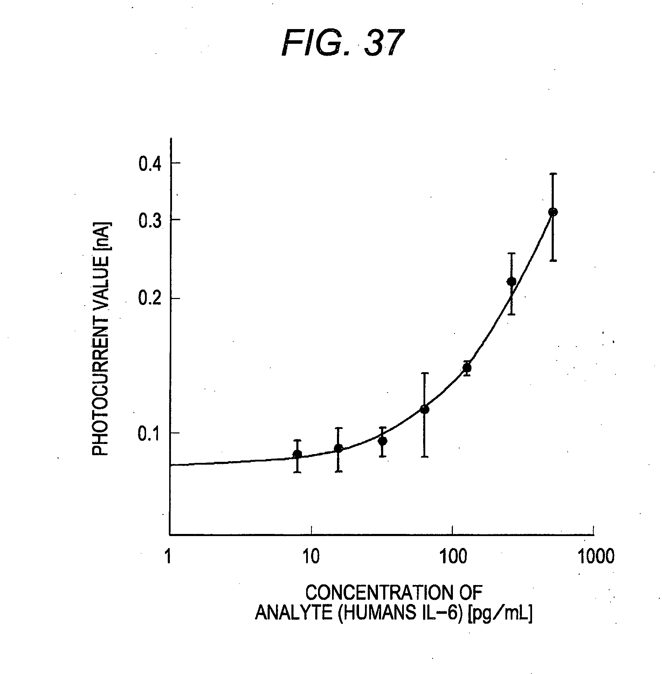

Fig. 37 is a graph showing examined results of a relationship between the concentration of the analyte (human IL-6) and photocurrent in Example 2-5.

From the results shown in Fig. 37, it is found that when the concentrations of the analyte are 7. 8 pg/mL, 15.6 pg/mL, 31.2 pg/mL, 62.5 pg/mL, 125 pg/mL, 250 pg/mL, and 500 pg/mL, the photocurrents detected are 0.089 nA, 0.092 nA, 0.095 nA, 0.11 nA, 0.14 nA, 0.21 nA, and 0.3 nA, respectively. It is found that the photocurrent detected when the analyte is not present is 0.083 nA. From these results, it is found that the photocurrent detected by the method for electrochemically detecting an analyte is increased according to the concentration of the analyte and the analyte can be quantitatively detected.

The above results suggest that analytes other than mouse IgG can be quantitatively detected with high sensitivity by using a multivalent-labeled binding substance in which more labeling substances are immobilized to the binding substance through the interaction between modulators.

### (Preparation example 2-7) Production of working electrode substrate

A working electrode body composed of a thin film (about 200 nm in thickness) of tin-doped indium oxide was formed on the surface of the substrate body composed of silicon dioxide (SiO₂) by the spattering method. The thin film serves both as the conductive layer and the electron accepting layer. Subsequently, a working electrode lead for connecting to the ammeter was connected to the working electrode body.

Then, the surface of the working electrode body was brought into contact with the solution A obtained in Preparation example 2-1 to provide a thiol group on the surface of the main body of the working electrode.

Anti-human interleukin-6 antibody [manufactured by BioLegend] as a trapping substance or anti-human interferon-γ antibody [manufactured by BioLegend] was reduced by bringing into contact with tris(2-carboxyethyl)phosphine hydrochloride (TCEP) fixed gel as a reducing agent (trade name: Immobilized TCEP Disulfide Reducing Gel, manufactured by Pierce], and a reduced heavy chain of anti-human interleukin-6 antibody or an anti-human interferon-γ antibody was produced. The obtained anti-human interleukin-6 antibody or anti-human interferon-γ antibody was added to TBS so that the concentration was 10 µg/mL, and an anti-human interleukin-6 antibody solution or an anti-human interferon-γ antibody solution was obtained.

Silicone rubber (0.1 mm in thickness) having two openings was placed on the working electrode so as to form a partition. Thereafter, the anti-human interleukin-6 antibody solution was poured into one of the spaces surrounded by the working electrode body and the silicone rubber. The anti-human interferon-γ antibody was placed in the other space. The working electrode body was incubated at 4°C overnight to react the anti-human interleukin-6 antibody or anti-human interferon-γ antibody with the thiol group on the working electrode body, and a dithiol bond was formed. Thus, the anti-human interleukin-6 antibody and anti-human interferon-γ antibody were immobilized on the working electrode body. The silicone rubber was removed from the working electrode body. Thereafter, silicone rubber (0.1 mm in thickness) having one opening with the size of the range corresponding to the two openings was placed on the working electrode so as to form a partition. Then, 1 mM triethylene glycol mono-11-mercaptoundecyl ether [manufactured by Sigma] was dropped onto the working electrode body. Blocking was performed by incubating the working electrode body at 4°C overnight. Thus, a working electrode substrate was obtained.

### (Example 2-6)

### Simultaneous detection of interleukin-6 (IL-6) and interferon-γ with multivalent-labeled DNA

### (1-1) Trapping of analyte

Silicone rubber was placed around the working electrode substrate obtained in Preparation example 2-7 so that a 0.2-mm-thick side wall was formed. Then, TBS-T containing 0.4% by mass of Block Ace [manufactured by DS Pharma Biomedical Co., Ltd.] was poured into the space surrounded by the working electrode substrate and the silicone rubber. The liquid in the above space was discharged. Thereafter, 30 µL of the detecting object solution (concentration of the detecting object: 250 pg/mL) obtained by diluting a single human-interleukin-6 [manufactured by BioLegend] as an analyte, a single human-interferon-γ [manufactured by BioLegend] as an analyte, or a mixture of human-interleukin-6 and human-interferon-γ as analytes with TBS-T containing 25% mass of bovine serum [manufactured by Thermo Scientific] and 0.75% by mass of BSA was added to the above space. Thereafter, the working electrode substrate was incubated at 25°C for 1 hour to allow analytes [interleukin-6 and human-interferon-γ] to be trapped by trapping substances [anti-human interleukin-6 antibody and anti-human interferon-γ antibody].

### (1-2) Labeling

The working electrode substrate subjected to the process (1-1) was washed with TBS-T. Then, biotin-labeled anti-human interleukin-6 antibody [manufactured by BioLegend] and anti-human interferon-γ antibody [manufactured by BioLegend] were added to TBS-T containing 1% by mass of BSA so that the concentrations thereof were 1 µg/mL, respectively. 30 µL of the obtained mixture was poured into the above space. Thereafter, the working electrode substrate was incubated at 25°C for 1 hour. Thus, the biotin-labeled anti-human interleukin-6 antibody (the first conjugate) and the biotin-labeled anti-human interferon-γ antibody were bound to the analyte trapped by the trapping substance. The biotin-labeled anti-human interleukin-6 antibody and the biotin-labeled anti-human interferon-γ antibody were obtained by respectively labeling the anti-human interleukin-6 antibody and the anti-human interferon-γ antibody with biotin.

Then, the working electrode substrate was washed with TBS-T. Then, 2 mg/mL of streptoavidin [manufactured by Vector Laboratories] (the second conjugate) was added to TBS-T so that its concentration was 4 µg/mL. 30 µL of the obtained mixture was poured into the above space. Thereafter, the working electrode substrate was incubated at 25°C for 30 minutes. Thus, streptoavidin was bound to the biotin-labeled anti-human interleukin-6 antibody or the anti-human interferon-γ antibody on the working electrode.

### (1-3) Dye-labeling

The working electrode substrate subjected to the process (1-1) was washed with TBS-T. Then, TBS-T was added to 100 µL of a solution containing the biotinylated-DNA/Alexa Fluor 750-labeled DNA complex obtained in Preparation example 2-5 (concentration of the complex: 93 µg/mL) in an amount 10 times the amount of the solution. Thereafter, 30 µL of the obtained mixture was poured into the above space. Thereafter, the working electrode substrate was incubated at 25°C for 30 minutes. Thus, a complex containing the analyte (a detecting object), the first conjugate, the second conjugate, and the labeled form was formed on the working electrode. The complex formed of the first conjugate and the second conjugate corresponds to the binding substance in the label binding substance obtained in Example 2-1. That is, on the working electrode, Alexa Fluor 750 as the labeling substance is bound to the binding substance bound to the analyte via DNA as the modulator.

### (2) Measurement of photocurrent

Silicone rubber was placed around the working electrode substrate so that a 0.2-mm-thick side wall was formed. Then, the space surrounded by the working electrode substrate and the silicone rubber was filled with the electrolytic solution obtained in Preparation example 2-3. The space filled with the electrolytic solution was sealed with the counter electrode substrate obtained in Preparation example 2-4 from the upper side of the working electrode substrate. Thus, the working electrode and the counter electrode are brought into contact with the electrolytic solution. Then, the detection chip including the working electrode substrate and the counter electrode was placed in an electrochemical measurement device. The working electrode lead and the counter electrode lead were connected to the ammeter.

The light source (wavelength: 781 nm, laser light source with an output power of 13 mW) emits excitation light from the working electrode substrate side toward the counter electrode substrate. The labeling substance Alexa Fluor 750 is excited by photoirradiation, thereby generating electrons. When the generated electrons are transported to the working electrode, current flows between the working electrode and the counter electrode. Then, the electric current was measured. Current measurement was performed on an anti-interleukin-6 antibody-immobilized portion and an anti-interferon-γ-antibody-immobilized portion. The operation was performed in the same manner as described above except that the analyte was not used. The control experiment when the analyte was not present was performed.

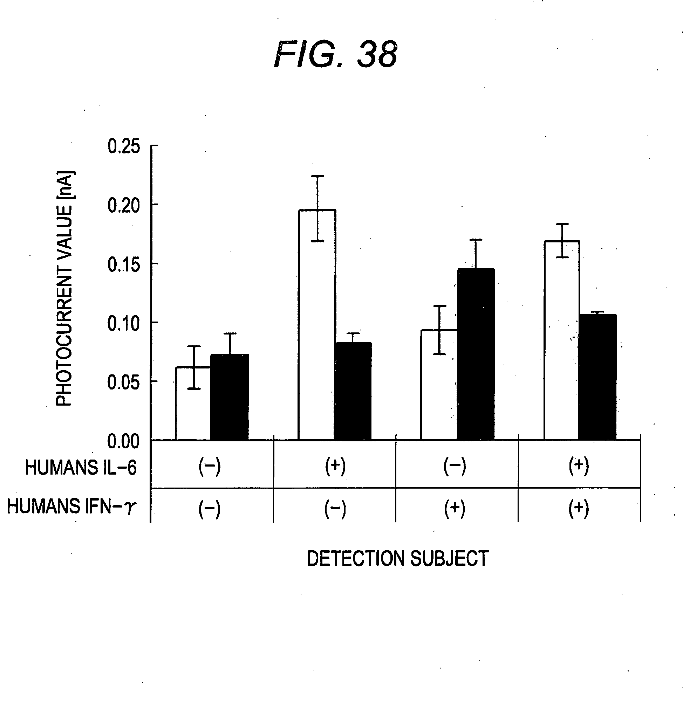

Fig. 38 shows the examined results of a relationship between the kind of the detection subject and photocurrent in Example 2-6.

From the results shown in Fig. 38, it is found that when the analyte is interleukin-6, the photocurrent detected in the anti-interleukin-6 antibody-immobilized portion is 0.20 nA and the photocurrent detected in the anti-interferon-γ-antibody-immobilized portion is 0.08 nA, while when the photocurrent is detected in the absence of the analyte, the photocurrent detected in the anti-interleukin-6 antibody-immobilized portion is 0.06 nA and the photocurrent detected in the anti-interferon-γ-antibody-immobilized portion is 0.07 nA. It is found that when the analyte is interferon-γ, the photocurrent detected in the anti-interleukin-6 antibody-immobilized portion is 0.09 nA and the photocurrent detected in the anti-interferon-γ-antibody-immobilized portion is 0.14 nA. These results suggest that it is possible to detect an analyte specific to the antibody which is the trapping substance immobilized on the same electrode.

On the other hand, it is found that when the analyte is a mixture of interleukin-6 and interferon-γ, the photocurrent detected in the anti-interleukin-6 antibody-immobilized portion is 0.17 nA and the photocurrent detected in the anti-interferon-γ-antibody-immobilized portion is 0.10 nA. These photocurrents are larger than those in the absence of the analyte, however, they are smaller than the photocurrent detected when the analyte is a single interleukin-6 or a single interferon-γ. Therefore, it is found that when the detecting object is a mixture of a plurality of types of analytes, it is possible to specifically detect each analyte, however, a slight decrease in detection sensitivity is observed as compared with the case where a single analyte is detected.

The above results suggest that it is possible to simultaneously detect various kinds of analytes on the same electrode by using a multivalent-labeled binding substance in which more labeling substances are immobilized to the binding substance through the interaction between modulators in order to label the analyte.

### [Sequence listing free text]

SEQ ID NO: 1 is a sequence of maleimidized DNA.
SEQ ID NO: 2 is a sequence of labeling substance-retaining DNA.
SEQ ID NO: 3 is a sequence of Alexa Fluor750-labeled DNA.
SEQ ID NO: 4 is a sequence of CK19 DNA-trapping DNA.
SEQ ID NO: 5 is a sequence of CK19 DNA.
SEQ ID NO: 6 is a sequence of CK19 recognizing/label-retaining DNA.
SEQ ID NO: 7 is a sequence of CK19-recognizing DNA.
SEQ ID NO: 8 is a sequence of label-retaining DNA-binding DNA.
SEQ ID NO: 9 is a sequence of Alexa Fluor750-labeled CK19-recognizing DNA.
SEQ ID NO: 10 is a sequence of DNA used as a modulator in Examples 2-1 and 2-2.
SEQ ID NO: 11 is a sequence of biotinylated-DNA. The 5' terminal of phosphate group is a biotinylated adenine base through the (CH₂)₃ linker
SEQ ID NO: 12 is a sequence of Alexa Fluor 750-labeled DNA.

## Claims

1. A method for electrochemically detecting an analyte in an electrolytic solution comprising:
bringing a sample containing an analyte into contact with a working electrode on which trapping substance (281) for trapping the analyte is immobilized to allow the analyte to be trapped on the working electrode by the trapping substance;
forming a complex containing the analyte (S) trapped on the working electrode in the trapping process and a label binding substance (290, 390), wherein the label binding substance (290, 390) is formed of a binding substance (291), a modulator (292), and a labeling substance (293, 393), wherein the labeling substance (293, 393) is retained via the modulator (292) on a binding substance (291) which binds to the analyte (S) on the working electrode, wherein the modulator generates an interaction with an electrolytic solution and a working electrode site except a site where the trapping substance are bound; and
electrochemically detecting the labeling substance present on the working electrode obtained in the complex formation process, wherein
the labeling substance is retained via the modulator on the binding substance, wherein
the electrolytic solution contains an aprotic solvent, and the surface of the working electrode and the modulator exhibit hydrophilicity, or
the electrolytic solution contains a protic solvent, and the surface of the working electrode and the modulator exhibit hydrophobicity.

2. The method according to claim 1, wherein the modulator is DNA.

3. The method according to claim 1 or 2, wherein in the process of forming a complex, the analyte trapped on the working electrode in the trapping process is brought into contact with the label binding substance to form the complex on the working electrode.

4. A method for electrochemically detecting an analyte in an electrolytic solution comprising:
bringing a sample containing an analyte into contact with a working electrode on which trapping substance (281) for trapping the analyte is immobilized to allow the analyte to be trapped on the working electrode by the trapping substance;
forming a complex, on the working electrode, by bringing a conjugate containing a first binding substance which binds to the analyte into contact with the analyte trapped on the working electrode in the trapping process and bringing the conjugate bound to the analyte into contact with a labeled form in which the labeling substance is bound, via the modulator, to a second binding substance, which binds to the conjugate in the complex formation process; and
electrochemically detecting the labeling substance present on the working electrode obtained in the complex formation process, wherein
the labeling substance is retained via the modulator on the binding substance, wherein
the electrolytic solution contains an aprotic solvent, and the surface of the working electrode and the modulator exhibit hydrophilicity, or
the electrolytic solution contains a protic solvent, and the surface of the working electrode and the modulator exhibit hydrophobicity.

5. The method according to any one of claims 1 to 4, wherein the working electrode is washed to remove the label binding substance which is not bound to the analyte after the process of forming a complex.

6. The method according to any one of claims 1 to 5, wherein the labeling substance is an electrochemically or photochemically active substance.

7. The method according to any one of claims 1 to 6, wherein the label binding substance is a multivalent-labeled binding substance in which a plurality of labeling substances are attached to the binding substance via modulators.

8. The method according to any one of claims 1 to 7, wherein the analyte is quantified based on the detection results obtained in the detecting process.

9. The method according to any one of claims 1 to 8, wherein the analyte is a plurality of analytes.

## Patentansprüche

1. Verfahren zum elektrochemischen Nachweisen eines Analyten in einer Elektrolytlösung, das umfasst:
in Kontaktbringen einer Probe, enthaltend einen Analyten mit einer Arbeitselektrode, auf dem ein Haftstoff (281) zum Anhaften des Analyten immobilisiert ist, um zu ermöglichen, dass der Analyt durch den Haftstoff an der Arbeitselektrode anhaftet;
Bilden eines Komplexes enthaltend den Analyten (S), der im Haftvorgang an der Arbeitselektrode angehaftet wurde, und eines Markierung-Bindestoffes (290, 390), wobei der Markierung-Bindestoff (290, 390) aus einem Bindestoff (291), einem Modulator (292) und einem Markierungsstoff (293, 393) gebildet wird, wobei der Markierungsstoff (293, 393) über den Modulator (292) auf einem Bindestoff (291) gehalten wird, der an den Analyten (S) auf der Arbeitselektrode bindet, wobei der Modulator eine Wechselwirkung mit einer Elektrolytlösung und einer Arbeitselektrodenstelle erzeugt, außer an der Stelle, an der der Haftstoff gebunden ist; sowie
elektrochemisches Nachweisen des Markierungsstoffs, der auf der Arbeitselektrode vorhanden ist, der in dem Komplexbildungsverfahren erhalten wurde, wobei
der Markierungsstoff über den Modulator auf dem Bindestoff zurückgehalten wird, wobei
die Elektrolytlösung ein aprotisches Lösungsmittel enthält und die Oberfläche der Arbeitselektrode und der Modulator Hydrophilizität zeigen, oder
die Elektrolytlösung ein protisches Lösungsmittel enthält, und die Oberfläche der Arbeitselektrode und der Modulator Hydrophobizität zeigen.

2. Verfahren gemäß Anspruch 1, wobei der Modulator DNA ist.

3. Verfahren gemäß Anspruch 1 oder 2, wobei im Komplexbildungsverfahren der Analyt, der im Haftverfahren auf der Arbeitselektrode angehaftet wird, in Kontakt mit dem Markierung-Bindestoff gebracht wird, um den Komplex auf der Arbeitselektrode zu bilden.

4. Verfahren zum elektrochemischen Nachweisen eines Analyten in einer Elektrolytlösung, das umfasst:
in Kontaktbringen einer Probe, enthaltend einen Analyten mit einer Arbeitselektrode, auf dem ein Haftstoff (281) zum Anhaften des Analyten immobilisiert ist, um zu ermöglichen, dass der Analyt durch den Haftstoff an der Arbeitselektrode anhaftet;
Bilden eines Komplexes auf der Arbeitselektrode, in dem ein Konjugat, enthaltend einen ersten Bindestoff, der an den Analyten bindet, mit dem Analyten in Kontakt gebracht wird, der im Haftverfahren auf der Arbeitselektrode angehaftet wird, und in Kontaktbringen des Konjugats, das an den Analyten gebunden ist, über den Modulator mit der markierten Form, an die der Markierungsstoff gebunden ist, mit einem zweiten Bindestoff, der das Konjugat im Komplexbildungsverfahren bindet; sowie
elektrochemisches Nachweisen des Markierungsstoffs, der auf der Arbeitselektrode vorhanden ist, der in dem Komplexbildungsverfahren erhalten wurde, wobei
der Markierungsstoff über den Modulator auf dem Bindestoff zurückgehalten wird, wobei
die Elektrolytlösung ein aprotisches Lösungsmittel enthält und die Oberfläche der Arbeitselektrode und der Modulator Hydrophilizität zeigen, oder
die Elektrolytlösung ein protisches Lösungsmittel enthält, und die Oberfläche der Arbeitselektrode und der Modulator Hydrophobizität zeigen.

5. Verfahren gemäß einem der Ansprüche 1 bis 4, wobei die Arbeitselektrode gewaschen wird, um den Markierung-Bindestoff zu entfernen, der nicht an den Analyten gebunden ist, im Anschluss an das Komplexbildungsverfahren.

6. Verfahren gemäß einem der Ansprüche 1 bis 5, wobei der Markierungsstoff elektrochemisch oder fotochemisch aktiver Stoff ist.

7. Verfahren gemäß einem der Ansprüche 1 bis 6, wobei der Markierung-Bindestoff ein multivalent markierter Bindestoff ist, bei dem eine Vielzahl von Markierungsstoffen über Modulatoren an den Bindestoff gebunden sind.

8. Verfahren gemäß einem der Ansprüche 1 bis 7, wobei der Analyt basierend auf den Nachweisergebnissen quantifiziert wird, die im Nachweisverfahren erhalten werden.

9. Verfahren gemäß einem der Ansprüche 1 bis 8, wobei der Analyt eine Vielzahl von Analyten ist.

## Revendications

1. Procédé permettant de détecter électrochimiquement un analyte dans une solution électrolytique comprenant le fait :
de mettre un échantillon contenant un analyte en contact avec une électrode de travail sur laquelle une substance de piégeage (281) permettant de piéger l'analyte est immobilisée pour permettre à l'analyte d'être piégé sur l'électrode de travail par la substance de piégeage ;
de former un complexe contenant l'analyte (S) piégé sur l'électrode de travail dans le processus de piégeage et une substance de liaison de marqueur (290, 390), où la substance de liaison de marqueur (290, 390) est formée d'une substance de liaison (291), d'un modulateur (292), et d'une substance de marquage (293, 393), où la substance de marquage (293, 393) est retenue par l'intermédiaire du modulateur (292) sur une substance de liaison (291) qui se lie à l'analyte (S) sur l'électrode de travail, où le modulateur génère une interaction avec une solution électrolytique et un site d'électrode de travail à l'exception d'un site où la substance de piégeage est liée ; et
de détecter électrochimiquement la substance de marquage présente sur l'électrode de travail obtenue dans le processus de formation de complexe, où
la substance de marquage est retenue par l'intermédiaire du modulateur sur la substance de liaison, où
la solution électrolytique contient un solvant aprotique, et la surface de l'électrode de travail et le modulateur présentent un caractère hydrophile, ou
la solution électrolytique contient un solvant protique, et la surface de l'électrode de travail et le modulateur présentent un caractère hydrophobe.

2. Procédé selon la revendication 1, dans lequel le modulateur est de l'ADN.

3. Procédé selon la revendication 1 ou 2, dans lequel, dans le processus qui consiste à former un complexe, l'analyte piégé sur l'électrode de travail dans le processus de piégeage est amené en contact avec la substance de liaison de marqueur pour former le complexe sur l'électrode de travail.

4. Procédé permettant de détecter électrochimiquement un analyte dans une solution électrolytique comprenant le fait :
d'amener un échantillon contenant un analyte en contact avec une électrode de travail sur laquelle une substance de piégeage (281) permettant de piéger l'analyte est immobilisée pour permettre à l'analyte d'être piégé sur l'électrode de travail par la substance de piégeage ;
de former un complexe, sur l'électrode de travail, en amenant un conjugué contenant une première substance de liaison qui se lie à l'analyte en contact avec l'analyte piégé sur l'électrode de travail dans le processus de piégeage et en amenant le conjugué lié à l'analyte en contact avec une forme marquée dans laquelle la substance de marquage est liée, par l'intermédiaire du modulateur, à une deuxième substance de liaison, qui se lie au conjugué dans le processus de formation de complexe ; et
de détecter électrochimiquement la substance de marquage présente sur l'électrode de travail obtenue dans le processus de formation de complexe, où
la substance de marquage est retenue par l'intermédiaire du modulateur sur la substance de liaison, où
la solution électrolytique contient un solvant aprotique, et la surface de l'électrode de travail et le modulateur présentent un caractère hydrophile, ou
la solution électrolytique contient un solvant protique, et la surface de l'électrode de travail et le modulateur présentent un caractère hydrophobe.

5. Procédé selon l'une quelconque des revendications 1 à 4, dans lequel l'électrode de travail est lavée pour éliminer la substance de liaison de marqueur qui n'est pas liée à l'analyte après le processus qui consiste à former un complexe.

6. Procédé selon l'une quelconque des revendications 1 à 5, dans lequel la substance de marquage est une substance photochimiquement ou électrochimiquement active.

7. Procédé selon l'une quelconque des revendications 1 à 6, dans lequel la substance de liaison de marqueur est une substance de liaison multivalente marquée dans laquelle une pluralité de substances de marquage sont attachées à la substance de liaison par l'intermédiaire de modulateurs.

8. Procédé selon l'une quelconque des revendications 1 à 7, dans lequel l'analyte est quantifié sur la base des résultats de détection obtenus dans le processus de détection.

9. Procédé selon l'une quelconque des revendications 1 à 8, dans lequel l'analyte est une pluralité d'analytes.


### SEQUENCE LISTING

<110> Sysmex Corporation
<120> METHOD FOR ELECTROCHEMICALLY DETECTING ANALYTE
<130> 10-079EP
<150> JP2011-096341
   <151> 2011-04-22
<150> JP2011-096319
   <151> 2011-04-22
<150> JP2012-075914
   <151> 2012-03-29
<160> 12
<170> PatentIn version 3.1
<210> 1
   <211> 40
   <212> DNA
   <213> Artificial
<220>
   <223> a sequence of maleimide-modified DNA
<400> 1
   ctcagctgtg actgcagctd gtatgcgcgc tgctatgccg 40
<210> 2
   <211> 146
   <212> DNA
   <213> Artificial
<220>
   <223> a sequence of DNA for holding a labeling substance
<400> 2
   ccacggccct agggacaacg accacggccc tagggacaac gaccacggcc ctagggacaa 60
   cgaccacggc cctagggaca acgaccacgg ccctagggac aacgaccacg gccctaggga 120
   caacgacggc atagcagcgc gcatac 146
<210> 3
   <211> 20
   <212> DNA
   <213> Artificial
<220>
   <223> a sequence of AlexaFluor750-labeled DNA
<400> 3
   cgttgtccct agggccgtgg 20
<210> 4
   <211> 99
   <212> DNA
   <213> Artificial
<220>
   <223> a sequence of DNA for capturing CK19 DNA
<400> 4
   cagctgggct tcaataccgc tgatcagcgc ctggatatgc gccagctggg ctccaaagcg 60
   cgcctccgtt tctgccagtg tgtcttccaa ggcagcttt 99
<210> 5
   <211> 150
   <212> DNA
   <213> Artificial
<220>
   <223> a sequence of CK19 DNA
<400> 5
   agcaggtccg aggttactga cctgcggcgc acccttcagg gtcttgagat tgagctgcag 60
   tcacagctga gcatgaaagc tgccttggaa gacacactgg cagaaacgga ggcgcgcttt 120
   ggagcccagc tggcgcatat ccaggcgctg 150
<210> 6
   <211> 40
   <212> DNA
   <213> Artificial
<220>
   <223> a sequence of DNA for holdingCK19 recognition sequence-labeling
<400> 6
   ctcagctgtg actgcagctc gtatgcgcgc tgctatgccg 40
<210> 7
   <211> 25
   <212> DNA
   <213> Artificial
<220>
   <223> a sequence of DNA for recognizing CK19
<400> 7
   ctcagctgtg actgcagctc aaaaa 25
<210> 8
   <211> 28
   <212> DNA
   <213> Artificial
<220>
   <223> a sequence of DNA for binding to the DNA for holding a labeling
<400> 8
   aaaaaaaagt atgcgcgctg ctatgccg 28
<210> 9
   <211> 20
   <212> DNA
   <213> Artificial
<220>
   <223> a sequence of AlexaFluor750-labeled CK19 recognition DNA
<400> 9
   ctcagctgtg actgcagctc 20
<210> 10
   <211> 24
   <212> DNA
   <213> Artificial
<220>
   <223> a sequence of DNA used as modified substance in Example 2-1 and E xample 2-2
<400> 10
   aactactgtc ttcacgcaga aagc 24
<210> 11
   <211> 87
   <212> DNA
   <213> Artificial
<220>
   <223> a sequence of biotinylated DNA
<400> 11
   aaaccacggc cctagggaca acgaccacgg ccctagggac aacgaccacg gccctaggga 60
   caacgaccac ggccctaggg acaacga 87
<210> 12
   <211> 40
   <212> DNA
   <213> Artificial
<220>
   <223> a sequence of AlexaFluor750 labeled DNA
<400> 12
   cgttgtccct agggccgtgg gtatgcgcgc tgctatgccg 40